# EUROPÄISCHE PATENTSCHRIFT

(11) **EP 1 765 842 B1**
(45) Veröffentlichungstag und Bekanntmachung des Hinweises auf die Patenterteilung: **09.01.2008**
(21) Anmeldenummer: 05755962.7
(22) Anmeldetag: 30.06.2005
(51) Int. Cl.: C07H 7/04, A61K 31/70

(54) **D-XYLOPYRANOSYL-SUBSTITUIERTE PHENYLE, DIESE VERBINDUNGEN ENTHALTENDE ARZNEIMITTEL, DEREN VERWENDUNG UND VERFAHREN ZU IHRER HERSTELLUNG**
D-XYLOPYRANOSYL-SUBSTITUTED PHENYLS, MEDICAMENTS CONTAINING SAID COMPOUNDS, THE USE THEREOF, AND METHODS FOR PRODUCING THE SAME
PHENYLES SUBSTITUES PAR D-XYLOPYRANOSYLE, MEDICAMENTS CONTENANT CES COMPOSES, UTILISATION ET PROCEDE DE PRODUCTION DE CES COMPOSES

(30) Priorität: 06.07.2004 DE 102004032823; 11.11.2004 DE 102004054603
(43) Veröffentlichungstag der Anmeldung: 28.03.2007
(73) Patentinhaber: BOEHRINGER INGELHEIM INTERNATIONAL GMBH, D-55216 Ingelheim (DE); Boehringer Ingelheim Pharma GmbH & Co. KG, 55216 Ingelheim (DE)
(72) Erfinder: ECKHARDT, Matthias, 88400 Biberach (DE); HIMMELSBACH, Frank, 88441 Mittelbiberach (DE); EICKELMANN, Peter, 88441 Mittelbiberach (DE); THOMAS, Leo, 88400 Biberach (DE); BARSOUMIAN, Edward, Leon, Saratoge, CA 95070 (US)
(74) Vertreter: Hammann, Heinz
(86) Internationale Anmeldenummer: PCT/EP2005/007042
(87) Internationale Veröffentlichungsnummer: WO 2006/002912

(56) Entgegenhaltungen:
- WO-A-02/083066
- WO-A-03/099836

## Beschreibung

Gegenstand der vorliegenden Erfindung sind D-Xylopyranosyl-substituierte Phenyle der allgemeinen Formel I 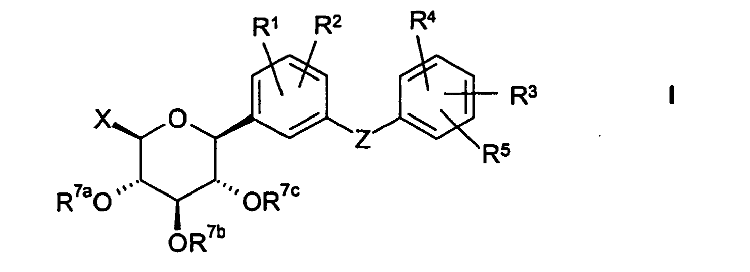 wobei die Reste R¹ bis R⁵, X, Z sowie R^{7a}, R^{7b}, R^{7c} nachfolgend definiert sind, einschließlich deren Tautomere, deren Stereoisomere, deren Gemische und deren Salze. Ein weiterer Gegenstand dieser Erfindung betrifft Arzneimittel enthaltend eine erfindungsgemäße Verbindung der Formel I sowie die Verwendung einer erfindungsgemäßen Verbindung zur Herstellung eines Arzneimittels zur Behandlung von Stoffwechselerkrankungen. Darüber hinaus sind Verfahren zur Herstellung eines Arzneimittels sowie einer erfindungsgemäßen Verbindung Gegenstand dieser Erfindung.

In der Literatur werden Verbindungen, die eine Hemmwirkung auf natriumabhängige Glucose-Cotransporter SGLT besitzen, zur Behandlung von Krankheiten, insbesondere von Diabetes vorgeschlagen.

Aus den internationalen Offenlegungsschriften WO 98/31697, WO 01/27128, WO 02/083066, WO 03/099836, WO 04/13118, WO 04/80990, WO 04/52902, WO 04/52903 und WO 05/12326 sind Glucopyranosyl-substituierte Aromaten sowie deren Herstellung und deren mögliche Aktivität als SGLT2-Inhibitoren bekannt.

### Aufgabe der Erfindung

Der vorliegenden Erfindung liegt die Aufgabe zugrunde, neue Pyranosyl-substituierte Phenyle aufzuzeigen, insbesondere solche, die eine Aktivität bezüglich natriumabhängiger Glucose-Cotransporter SGLT, insbesondere SGLT2 besitzen. Eine weitere Aufgabe der vorliegenden Erfindung besteht im Aufzeigen von Pyranosyl-substituierten Phenylen, die *in vitro* und/oder *in vivo* im Vergleich mit bekannten, strukturähnlichen Verbindungen eine erhöhte Hemmwirkung bezüglich des natriumabhängigen Glucose-Cotransporters SGLT2 besitzen und/oder verbesserte pharmakologische oder pharmakokinetische Eigenschaften aufweisen.

Ferner ist es eine Aufgabe der vorliegenden Erfindung, neue Arzneimittel bereit zu stellen, welche zur Prophylaxe und/oder Behandlung von Stoffwechselerkrankungen, insbesondere von Diabetes geeignet sind.

Ebenfalls eine Aufgabe dieser Erfindung ist es, ein Verfahren zur Herstellung der erfindungsgemäßen Verbindungen bereit zu stellen.

Weitere Aufgaben der vorliegenden Erfindung ergeben sich für den Fachmann unmittelbar aus den vorhergehenden und nachfolgenden Ausführungen.

### Gegenstand der Erfindung

Ein erster Gegenstand der vorliegenden Erfindung sind D-Xylopyranosyl-substituierte Phenyle der allgemeinen Formel I 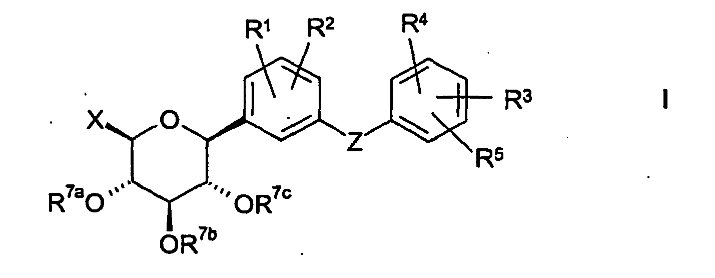 in der
- R¹: Wasserstoff, Fluor, Chlor, Brom, C₁₋₆-Alkyl, C₂₋₆-Alkinyl, C₂₋₆-Alkenyl, C₃₋₇-Cycloalkyl, C₃₋₇-Cycloalkyl-C₁₋₃-alkyl, C₅₋₇-Cycloalkenyl, C₅₋₇-Cycloalkenyl-C₁₋₃-alkyl, C₁₋₄-Alkylcarbonyl, Arylcarbonyl, Heteroarylcarbonyl, Aminocarbonyl, C₁₋₄-Alkylaminocarbonyl, Di-(C₁₋₃-Alkyl)aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)piperazin-1-ylcarbonyl, C₁₋₄-Alkoxycarbonyl, Amino, C₁₋₄-Alkylamino,-Di-(C₁₋₃-alkyl)amino, Pyrrolidin-1-yl, Piperidin-1-yl, Morpholin-4-yl, Piperazin-1-yl, 4-(C₁₋₄-Alkyl)piperazin-1-yl, C₁₋₄-Alkylcarbonylamino, C₁₋₆-Alkyloxy, C₃₋₇-Cycloalkyloxy, C₅₋₇-Cycloalkenyloxy, Aryloxy, C₁₋₄-Alkylsulfanyl, C₁₋₄-Alkylsulfinyl, C₁₋₄-Alkylsulfonyl, C₃₋₇-Cycloalkylsulfanyl, C₃₋₇-Cycloalkylsulfinyl, C₃₋₇-Cycloalkylsulfonyl, C₅₋₇-Cycloalkenylsulfanyl, C₅₋₇-Cycloalkenylsulfinyl, C₅₋₇-Cycloalkenylsulfonyl, Arylsulfanyl, Arylsulfinyl, Arylsulfonyl, Hydroxy, Cyan oder Nitro bedeutet,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und
wobei in N-Heterocycloalkyl-Resten eine Methylengruppe durch CO oder SO₂ ersetzt sein kann, und
- R²: Wasserstoff, Fluor, Chlor, Brom, Hydroxy, C₁₋₄-Alkyl, C₁₋₄-Alkoxy, Cyan oder Nitro bedeutet, wobei Alkyl-Reste ein- oder mehrfach mit Fluor substituiert sein können, oder
für den Fall, dass R¹ und R² an zwei miteinander benachbarte C-Atome des Phenylrings gebunden sind, können R¹ und R² derart miteinander verbunden sein, dass R¹ und R² zusammen eine C₃₋₅-Alkylen- oder C₃₋₅-Alkenylen-Brücke bilden, die teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert kann und in der eine oder zwei Methylengruppen unabhängig voneinander durch O, S, CO, SO, SO₂ oder NR^{N} ersetzt sein können,
- R³: Wasserstoff, Fluor, Chlor, Brom, C₁₋₆-Alkyl, C₂₋₆-Alkinyl, C₂₋₈-Alkenyl, C₃₋₇-Cyclo-alkyl, C₃₋₇Cycloalkyl-C₁₋₃-alkyl, C₅₋₇-Cycloalkenyl, C₅₋₇-Cycloalkenyl-C₁₋₃-alkyl, Aryl, Heteroaryl, C₁₋₄-Alkylcarbonyl, Arylcarbonyl, Heteroarylcarbonyl, Aminocarbonyl, C₁₋₄-Alkylaminocarbonyl, Di-(C₁₋₃-Alkyl)aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)piperazin-1-ylcarbonyl, Hydroxycarbonyl, C₁₋₄-Alkoxycarbonyl, C₁₋₄-Alkylamino, Di-(C₁₋₃-alkyl)amino, Pyrrolidin-1-yl, Piperidin-1-yl⁻; Morpholin-4-yl, Piperazin-1-yl, 4-(C₁₋₄-Alkyl)piperazin-1-yl, C₁₋₄-Alkylcarbonylamino, Arylcarbonylamino, Heteroarylcarbonylamino, C₁₋₄-Alkylsulfonylamino, Arylsulfonylamino, C₁₋₆-Alkoxy, C₃₋₇-Cycloalkyloxy, C₅₋₇-Cycloalkenyloxy, Aryloxy, Heteroaryloxy, C₁₋₄-Alkylsulfanyl, C₁₋₄-Alkylsulfinyl, C₁₋₄-Alkylsulfonyl, C₃₋₇-Cycloalkylsulfanyl, C₃₋₇-Cycloalkylsulfinyl, C₃₋₇-Cycloalkylsulfonyl, C₅₋₇-Cycloalkenylsulfanyl, C₅₋₇-Cycloalkenylsulfinyl, C₅₋₇-Cycloalkenylsulfonyl, Arylsulfanyl, Arylsulfinyl, Arylsulfonyl, Amino, Hydroxy, Cyan oder Nitro bedeutet,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und
wobei in N-Heterocycloalkyl-Resten eine Methylengruppe durch CO oder SO₂ ersetzt sein kann, und
- R⁴: Wasserstoff, Fluor, Chlor, Brom, lod, Cyan, Nitro, C₁₋₃-Alkyl, C₁₋₃-Alkoxy oder durch 1 bis 3 Fluoratome substituiertes Methyl- oder Methoxy bedeutet, oder
für den Fall, dass R³ und R⁴ an zwei miteinander benachbarte C-Atome des Phenylrings gebunden sind, können R³ und R⁴ derart miteinander verbunden sein, dass R³ und R⁴ zusammen eine C₃₋₅-Alkylen- oder C₃₋₅-Alkenylen-Brücke bilden, die teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert kann und in der eine oder zwei Methylengruppen unabhängig voneinander durch O, S, CO, SO, SO₂ oder NR^{N} ersetzt sein können,
- R⁵: Wasserstoff, Fluor, Chlor, Brom, lod, Cyan, Nitro, C₁₋₃-Alkyl, C₁₋₃-Alkoxy oder durch 1 bis 3 Fluoratome substituiertes Methyl- oder Methoxy bedeutet, und
- R^{N}: unabhängig voneinander H oder C₁₋₄-Alkyl bedeuten,

- L: unabhängig voneinander ausgewählt aus der Gruppe bestehend aus Fluor, Chlor, Brom, Iod, C₁₋₃-Alkyl, Difluormethyl, Trifluormethyl, C₁₋₃-Alkoxy, Difluormethoxy, Trifluormethoxy und Cyan,
- R^{7a}, R^{7b}, R^{7c}: unabhängig voneinander eine Bedeutung ausgewählt aus der Gruppe Wasserstoff, (C₁₋₁₈-Alkyl)carbonyl, (C₁₋₁₈-Alkyl)oxycarbonyl, Arylcarbonyl und Aryl-(C₁₋₃-alkyl)carbonyl besitzen,
- X: Wasserstoff, C₁₋₆-Alkyl, C₂₋₆-Alkinyl, C₂₋₆-Alkenyl, C₃₋₇-Cycloalkyl, C₃₋₇-Cycloalkyl-C₁₋₃-alkyl, C₅₋₇-Cycloalkenyl, C₅₋₇₋Cycloalkenyl-C₁₋₃-alkyl, Aryl, Aryl-C₁₋₃-alkyl, Heteroaryl, Heteroaryl-C₁₋₃-alkyl, C₁₋₄-Alkylcarbonyl, Arylcarbonyl, Aminocarbonyl, C₁₋₄-Alkylaminocarbonyl, Di-(C₁₋₃-Alkyl)aminocarbonyl, (Aryl-C₁₋₃-alkyl)aminocarbonyl, Pyrrolidin-1-ylcarbonyl, Piperidin-1-ylcarbonyl, Morpholin-4-ylcarbonyl, Hydroxycarbonyl, C₁₋₄-Alkoxycarbonyl, C₁₋₄-Alkylcarbonylamino-C₁₋₃-alkyl, N-(C₁₋₄-Alkylcarbonyl)-N-(C₁₋₃-alkyl)-amino-C₁₋₃-alkyl, Arylcarbonylamino-C₁₋₃-alkyl, C₁₋₄-Alkylsulfonylamino-C₁₋₃-alkyl, Arylsulfonylamino-C₁₋₃-alkyl, C₁₋₆-Alkoxy-C₁₋₃-alkyl, C₃₋₇-Cycloalkyloxy-C₁₋₃-alkyl, C₅₋₇-Cycloalkenyloxy-C₁₋₃-alkyl, Aryloxy-C₁₋₃-alkyl, Heteroaryloxy-C₁₋₃-alkyl, C₁₋₄-Alkylsulfanyl-C₁₋₃-alkyl, C₁₋₄-Alkylsulfinyl-C₁₋₃-alkyl, C₁-₄-Alkylsulfinyl, C₁₋₄-Alkylsulfonyl, C₁₋₄-Alkylsulfonyl-C₁₋₃-alkyl, C₁₋₄-Arylsulfanyl-C₁-₃-alkyl, Arylsulfonyl-C₁₋₃-alkyl, Aryl-C₁₋₃-alkyl-sulfonyl-C₁₋₃-alkyl, C₁₋₄-Alkylsulfonyloxy-C₁₋₃-alkyl, Arylsulfonyloxy-C₁₋₃-alkyl, Aryl-C₁₋₃-alkyl-sulfonyloxy-C₁₋₃-alkyl, C₃-₇-Cycloalkylsulfanyl-C₁₋₃-alkyl, C₃₋₇-Cycloalkylsulfinyl, C₃₋₇Cycloalkylsulfonyl, C₅-₇-Cycloalkenylsulfanyl-C₁₋₃-alkyl, C₅₋₇-Cycloalkenylsulfinyl, C₅₋₇-Cycloalkenylsulfonyl, C₁₋₄-Alkylcarbonylsulfanyl-C₁₋₃-alkyl oder Cyan bedeuten,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Cyan, Hydroxy, Mercapto, C₁₋₃-Alkoxy und C₁-₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und
wobei in N-Heterocycloalkyl-Resten eine Methylengruppe durch CO oder SO₂ ersetzt sein kann, und
wobei X in der Bedeutung Hydroxymethyl ausgeschlossen ist,
- Z: Sauerstoff, Methylen, Dimethylmethylen, Difluormethylen oder Carbonyl;
wobei unter den bei der Definition der vorstehend genannten Reste erwähnten Arylgruppen Phenyl- oder Naphthylgruppen zu verstehen sind, welche unabhängig voneinander ein- oder zweifach mit gleichen oder verschiedenen Resten L substituiert sein können; und
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl-, Pyridyl- oder Imidazolylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
wobei die vorstehend erwähnten Heteroarylgruppen unabhängig voneinander ein- oder zweifach mit gleichen oder verschiedenen Resten L substituiert sein können;
wobei unter den bei der Definition der vorstehend erwähnten Reste erwähnten N-Heterocycloalkyl-Rest ein gesättigter carbocyclischer Ring, der eine Imino-Gruppe im Ring aufweist, zu verstehen ist, der eine weitere gegebenenfalls substituierte Imino-Gruppe oder ein O- oder S-Atom im Ring aufweisen kann, und
wobei, soweit nichts anderes erwähnt wurde, die vorstehend erwähnten Alkylgruppen geradkettig oder verzweigt sein können,
deren Tautomere, deren Stereoisomere, deren Gemische und deren Salze, insbesondere deren physiologisch verträglichen Salze.

Die erfindungsgemäßen Verbindungen der allgemeinen Formel I und ihre physiologisch verträglichen Salze weisen wertvolle pharmakologische Eigenschaften auf, insbesondere eine Hemmwirkung auf natriumabhängige Glucose-Cotransporter SGLT, insbesondere SGLT2. Ferner können erfindunsgemäße Verbindungen eine Hemmwirkung auf den natriumabhängigen Glucose-Cotransporter SGLT1 aufweisen. Verglichen mit einer möglichen Hemmwirkung auf SGLT1 hemmen die erfindungsgemäßen Verbindungen vorzugsweise selektiv SGLT2.

Gegenstand der vorliegenden Erfindung sind auch die physiologisch verträglichen Salze der erfindungsgemäßen Verbindungen mit anorganischen oder organischen Säuren.

Daher ist die Verwendung der erfindungsgemäßen Verbindungen, einschließlich der physiologisch verträglichen Salze, als Arzneimittel ebenfalls ein Gegenstand dieser Erfindung.

Ein weiterer Gegenstand dieser Erfindung sind Arzneimittel, enthaltend mindestens eine erfindungsgemäße Verbindung oder ein erfindungsgemäßes physiologisch verträgliches Salz neben gegebenenfalls einem oder mehreren inerten Trägerstoffen und/oder Verdünnungsmitteln.

Ebenfalls ein Gegenstand dieser Erfindung ist die Verwendung mindestens einer erfindungsgemäßen Verbindung oder eines physiologisch verträglichen Salzes solch einer Verbindung zur Herstellung eines Arzneimittels, das zur Behandlung oder Prophylaxe von Erkrankungen oder Zuständen geeignet ist, die durch Inhibierung des natriumabhängigen Glucose-Cotransporters SGLT, insbesondere SGLT2 beeinflussbar sind.

Ein weiterer Gegenstand dieser Erfindung ist die Verwendung mindestens einer erfindungsgemäßen Verbindung zur Herstellung eines Arzneimittels, das zur Behandlung von Stoffwechselerkrankungen geeignet ist.

Ein weiterer Gegenstand dieser Erfindung ist die Verwendung mindestens einer erfindungsgemäßen Verbindung zur Herstellung eines Arzneimittels zur Inhibition des natriumabhängigen Glucose-Cotransporters SGLT, insbesondere SGLT2.

Ferner ist ein Verfahren zur Herstellung eines erfindungsgemäßen Arzneimittels Gegenstand dieser Erfindung, dadurch gekennzeichnet, dass auf nicht-chemischem Wege eine erfindungsgemäße Verbindung in einen oder mehrere inerte Trägerstoffe und/oder Verdünnungsmittel eingearbeitet wird.

Gegenstand der vorliegenden Erfindung ist auch ein Verfahren zur Herstellung der erfindungsgemäßen Verbindungen der allgemeinen Formel I, dadurch gekennzeichnet, dass
a) zur Herstellung von Verbindungen der allgemeinen Formel I, die wie vor- und nachstehend definiert ist,
   eine Verbindung der allgemeinen Formel II 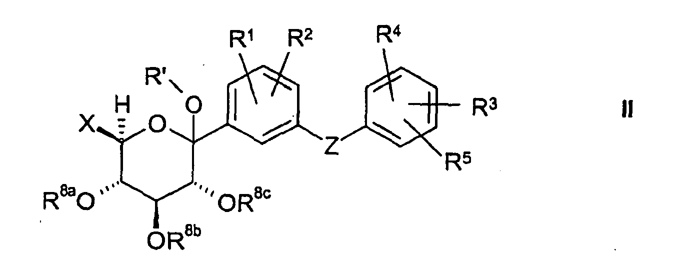 in der
   - R': H, C₁₋₄-Alkyl, (C₁₋₁₈-Alkyl)carbonyl, (C₁₋₁₈-Alkyl)oxycarbonyl, Arylcarbonyl oder Aryl-(C₁₋₃-alkyl)-carbonyl bedeutet, worin die Alkyl- oder Arylgruppen ein- oder mehrfach mit Halogen substituiert sein können;
   - R^{8a}, R^{8b}, R^{8c}: unabhängig voneinander eine zuvor und nachstehend für die Reste R^{7a}, R^{7b}, R^{7c} angegebenen Bedeutungen aufweisen, eine Benzyl-Gruppe oder eine R^{a}R^{b}R^{c}Si-Gruppe oder eine Ketal- oder Acetalgruppe, insbesondere eine Alkyliden- oder Arylalkyliden- Ketal- oder Acetalgruppe bedeuten, wobei jeweils zwei benachbarte Reste R^{6a}, R^{6b}, R^{6c} eine cyclische Ketal- oder Acetalgruppe oder eine 1,2-Di(C₁₋₃-alkoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring, insbesondere einen 2,3-Dimethyl-2,3-di(C₁₋₃-alkoxy)-1,4-dioxan-Ring, bildet, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁₋₃-alkyl)amino-Gruppe substituiert sein können; und
   - R^{a}, R^{b}, R^{c}: unabhängig voneinander C₁₋₄-Alkyl, Aryl oder Aryl-C₁₋₃-alkyl bedeuten, worin die Aryl- oder Alkylgruppen ein- oder mehrfach mit Halogen substituiert sein können;
   wobei unter den bei der Definition der vorstehend genannten Reste erwähnten Arylgruppen Phenyl- oder Naphthylgruppen, vorzugsweise Phenylgruppen zu verstehen sind;
   und in der die Reste X und R¹ bis R⁶ und die Brücke Z wie vor- und nachstehend definiert sind;
   mit einem Reduktionsmittel in Gegenwart einer Säure umgesetzt wird, wobei die eventuell vorhandenen Schutzgruppen gleichzeitig oder nachträglich abgespalten werden; oder
b) zur Herstellung von Verbindungen der allgemeinen Formel I, in der R^{7a}, R^{7b} und R^{7c} Wasserstoff bedeuten,
in einer Verbindung der allgemeinen Formel III 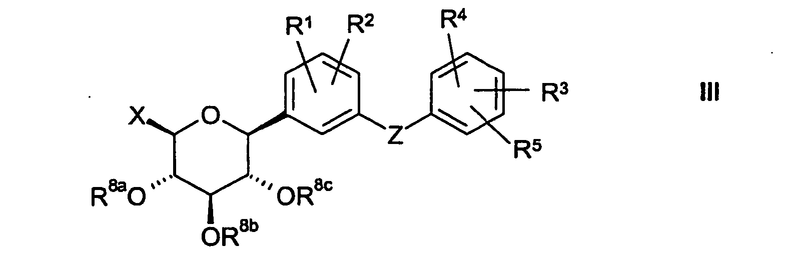 in der X, Z, R^{8a}, R^{8b}, R^{8c} sowie R¹ bis R⁵ wie zuvor und nachstehend definiert sind, wobei mindestens einer der Reste R^{8a}, R^{8b} und R^{8c} nicht Wasserstoff bedeutet,
die nicht Wasserstoff bedeutenden Reste R^{8a}, R^{8b} bzw. R^{8c} entfernt werden, insbesondere hydrolysiert werden; und
erforderlichenfalls ein bei den vorstehend beschriebenen Umsetzungen gemäß Verfahren a) oder b) verwendeter Schutzrest wieder abgespalten wird und/oder
gewünschtenfalls eine so erhaltene Verbindung der allgemeinen Formel I selektiv an einer Hydroxygruppe derivatisiert oder diese substituiert wird und/oder
gewünschtenfalls eine so erhaltene Verbindung der allgemeinen Formel I in ihre Stereoisomere aufgetrennt wird und/oder
gewünschtenfalls eine so erhaltene Verbindung der allgemeinen Formel I in ihre Salze, insbesondere für die pharmazeutische Anwendung in ihre physiologisch verträglichen Salze, überführt wird.

### Detailierte Beschreibung der Erfindung

Sofern nicht anders angegeben besitzen die Gruppen, Reste und Substituenten, insbesondere R¹ bis R⁵, X, Z, L, R^{N}, R^{7a}, R^{7b}, R^{7c} die zuvor und nachfolgend angegebenen Bedeutungen.

Kommen Reste, Substituenten oder Gruppen in einer Verbindung mehrfach vor, so können diese eine gleiche oder verschiedene Bedeutungen aufweisen.

Der Rest R³ steht vorzugsweise in meta- oder para Position zur -Z-Brücke, so dass Verbindungen gemäß der folgenden Formeln 1.1 und 1.2, insbesondere der Formel 1.2, bevorzugt sind:

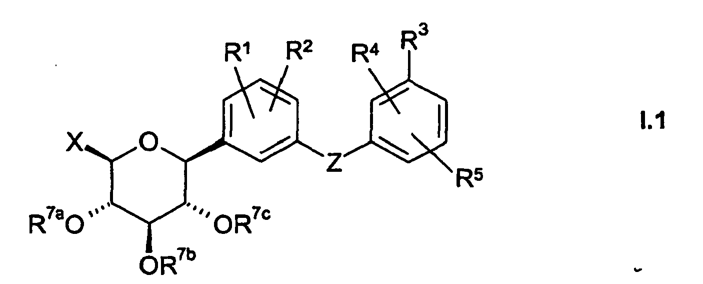

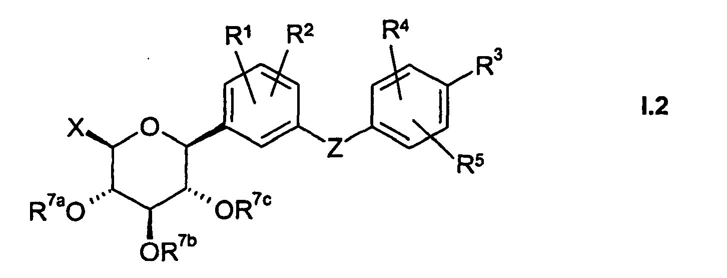

Die vorstehend und nachfolgend verwendete, beispielsweise in den Gruppen X, R¹ und R³ vorkommende Bezeichnung Aryl bedeutet vorzugsweise Phenyl. Gemäß der allgemeinen Definition und sofern nichts anderes angegeben ist, kann die Aryl-Gruppe, insbesondere die Phenylgruppe, ein- oder zweifach mit gleichen oder verschiedenen Resten L substituiert sein.

Die vorstehend und nachfolgend verwendete, beispielsweise in den Gruppen X, R¹ und R³ vorkommende Bezeichnung Heteroaryl bedeutet vorzugsweise Pyridinyl, Pyrimidinyl, Pyridazinyl, Pyrazinyl, Triazinyl, Imidazolyl, Pyrazolyl, Triazolyl, Tetrazolyl, Oxazolyl, Oxadiazolyl, Thiazolyl oder Thiadiazolyl. Gemäß der allgemeinen Definition und sofern nichts anderes angegeben ist, kann die Heteroaryl-Gruppe ein- oder zweifach mit gleichen oder verschiedenen Resten L substituiert sein.

Bevorzugt bedeutet R¹ Wasserstoff, Fluor, Chlor, Brom, C₁₋₆-Alkyl, C₂₋₆-Akinyl, C₂₋₆-Alkenyl, C₃₋₇-Cycloalkyl, C₅₋₇-Cycloalkenyl, C₁₋₄-Alkylcarbonyl, Aminocarbonyl, C₁₋₄-Alkylaminocarbonyl, Di-(C₁₋₃-Alkyl)aminocarbonyl, C₁₋₄-Alkoxycarbonyl, C₁₋₄-Alkylamino, Di-(C₁₋₃-alkyl)amino, Pyrrolidin-1-yl, Piperidin-1-yl, Morpholin-4-yl, C₁₋₄-Alkylcarbonylamino, C₁₋₆-Alkyloxy, C₃₋₇Cycloalkyloxy, C₅₋₇Cycloalkenyloxy, C₁₋₄-Alkylsulfanyl, C₁₋₄-Alkylsulfonyl, C₃₋₇-Cycloalkylsulfanyl, C₃₋₇-Cycloalkylsulfonyl, C₅₋₇-Cycloalkenylsulfanyl, C₅₋₇-Cycloalkenylsulfonyl, Hydroxy und Cyan,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und
wobei in N-Heterocycloalkyl-Resten eine Methylengruppe durch CO oder SO₂ ersetzt sein kann.

Bedeutet die Gruppe R¹ einen Cycloalkyl- oder Cycloalkenyl-Rest, in dem eine oder zwei Methylengruppen unabhängig voneinander durch O, S, CO, SO oder SO₂ ersetzt sind, so sind bevorzugte Bedeutungen des Rests R¹ ausgewählt aus der Gruppe bestehend aus Tetrahydrofuranyl, Tetrahydrofuranonyl, Tetrahydrothienyl, Tetrahydropyranyl, Tetrahydropyranonyl, Dioxanyl und Trioxanyl.

Bedeutet die Gruppe R¹ einen N-Heterocycloalkyl-Rest, in dem eine Methylengruppe durch CO oder SO₂ ersetzt ist, so sind bevorzugte Bedeutungen des Rests R¹ ausgewählt aus der Gruppe bestehend aus Pyrrolidinon, Piperidinon, Piperazinon und Morpholinon.

Besonders bevorzugt bedeutet R¹ Wasserstoff, Fluor, Chlor, Brom, C₁₋₈-Alkyl, C₂₋₆-Alkinyl, C₂₋₆-Alkenyl, C₃₋₇-Cycloalkyl, C₅₋₇-Cycloalkenyl, C₁₋₆-Alkyloxy, C₃₋₇-Cycloalkyloxy oder Cyan,
wobei in Cycloalkyl- und Cycloalkenylgruppen eine oder zwei Methyleneinheiten unabhängig voneinander durch O oder CO ersetzt und Alkyl-, Alkenyl- und Alkinyl-Reste teilweise oder vollständig fluoriert sein können.

Beispiele der ganz besonders bevorzugten Reste R¹ sind Wasserstoff, Fluor, Chlor, Brom, Methyl, Ethyl, Isopropyl, Trifluormethyl, Ethinyl, Methoxy, Cyclopentyloxy und Cyan, insbesondere Chlor und Methyl.

Der Rest R³ bedeutet vorzugsweise Fluor, Chlor, Brom, C₁₋₆-Alkyl, C₂₋₆-Alkinyl, C₂₋₆-Alkenyl, C₃₋₇-Cycloalkyl, C₃₋₇-Cycloalkyl-methyl, C₅₋₇-Cycloalkenyl, C₃₋₇-Cycloalkenyl-methyl, Aryl, Heteroaryl, C₁₋₄-Alkylcarbonyl, Aminocarbonyl, C₁₋₄-Alkylaminocarbonyl, Di-(C₁₋₃-Alkyl)-aminocarbonyl, C₁₋₄-Alkoxycarbonyl, Di-(C₁₋₃-alkyl)amino, Pyrrolidin-1-yl, Piperidin-1-yl, Morpholin-4-yl, C₁₋₄-Alkylcarbonylamino, C₁₋₆-Alkoxy, C₃₋₇-Cycloalkyloxy, C₅₋₇-Cycloalkenyloxy, Aryloxy, Heteroaryloxy, C₁₋₄-Alkylsulfanyl, C₁₋₄-Alkylsulfonyl, C₃₋₇-Cycloalkylsulfanyl, C₃-₇-Cycloalkylsulfonyl, C₅₋₇-Cycloalkenylsulfanyl, C₅₋₇-Cycloalkenylsulfonyl, Hydroxy und Cyan,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und
wobei in N-Heterocycloalkyl-Resten eine Methylengruppe durch CO oder SO₂ ersetzt sein kann,
wobei die Begriffe Aryl und Heteroaryl wie zuvor definiert sind und Aryl- und HeteroarylGruppen unabhängig voneinander ein- oder zweifach mit gleichen oder verschiedenen Resten L substituiert sein können.

Bedeutet die Gruppe R³ einen Cycloalkyl- oder Cycloalkenyl-Rest, in dem eine oder zwei Methylengruppen unabhängig voneinander durch O, S, CO, SO oder SO₂ ersetzt sind, so sind bevorzugte Bedeutungen der Gruppe R³ ausgewählt aus der Gruppe bestehend aus Tetrahydrofuranyl, Tetrahydrofuranonyl, Tetrahydrothienyl, Tetrahydropyranyl, Tetrahydropyranonyl, Dioxanyl und Trioxanyl.

Bedeutet die Gruppe R³ einen N-Heterocycloalkyl-Rest, in dem eine Methylengruppe durch CO oder SO₂ ersetzt ist, so sind bevorzugte Bedeutungen des Rests R³ ausgewählt aus der Gruppe bestehend aus Pyrrolidinon, Piperidinon, Piperazinon und Morpholinon.

Besonders bevorzugte R³ sind C₁₋₆-Alkyl, C₂₋₆-Alkinyl, C₁₋₄-Alkyloxy, C₃₋₇Cycloalkyl, C₃₋₇-Cycloalkyloxy und Hydroxy, wobei in den Cycloalkylgruppen eine oder zwei Methyleneinheiten unabhängig voneinander durch O oder CO ersetzt und Alkylreste teilweise oder vollständig fluoriert sein können.

Ganz besonders bevorzugte Reste R³ sind Methyl, Ethyl, Ethinyl, Isopropyl, Methoxy, Ethoxy, Isopropyloxy, Difluormethoxy, Cyclopentyloxy, Tetrahydro-furan-3-yloxy und Hydroxy.

Eine Auswahl der ganz besonders bevorzugten Vertreter von R³ ist Methyl, Ethyl, Isopropyl, Ethinyl, Methoxy, Ethoxy, Cyclopentyloxy und Hydroxy.

Gemäß einer bevorzugte Ausführungsvariante der vorliegenden Erfindung bedeutet R³ C₂₋₆-Alkinyl, insbesondere Ethinyl.

Die Gruppe X bedeutet vorzugsweise Wasserstoff, C₁₋₆-Alkyl, C₂₋₆-Alkinyl, C₂₋₆-Alkenyl, C₃-₇-Cycloalkyl-C₁₋₃-alkyl, C₅₋₇-Cycloalkenyl-C₁₋₃-alkyl, Aryl, Aryl-C₁₋₃-alkyl, Heteroaryl, Heteroaryl-C₁₋₃-alkyl, Aminocarbonyl, C₁₋₄-Alkylaminocarbonyl, Di-(C₁₋₃-Alkyl)aminocarbonyl, Pyrrolidin-1-ylcarbonyl, Piperidin-1-ylcarbonyl, Morpholin-4-ylcarbonyl, C₁₋₄-Alkoxycarbonyl, C₁₋₄-Alkylcarbonylamino-C₁₋₃-alkyl, N-(C₁₋₄-Alkylcarbonyl)-N-(C₁₋₃-alkyl)-amino-C₁₋₃-alkyl, Arylcarbonylamino-C₁₋₃-alkyl, C₁₋₈-Alkoxy-C₁₋₃-alkyl, C₃₋₇-Cycloalkyloxy-C₁₋₃-alkyl, C₅₋₇-Cycloalkenyloxy-C₁₋₃-alkyl, Aryloxy-C₁₋₃-alkyl, Heteroaryloxy-C₁₋₃-alkyl, C₁₋₄-Alkylcarbonylsulfanyl-C₁₋₃-alkyl oder C₁₋₄-Alkylsulfanyl-C₁₋₃-alkyl,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Cyan, Hydroxy, Mercapto, C₁₋₃-Alkoxy und C₁₋₃-Alkyl substituiert sein können, und
wobei in den vorstehend genannten Cycloalkyl- und Cycloalkenyl-Resten eine oder zwei Methylengruppen unabhängig voneinander durch O, S, CO, SO oder SO₂ ersetzt sein können,und
wobei in N-Heterocycloalkyl-Resten eine Methylengruppe durch CO oder SO₂ ersetzt sein kann, und
wobei die Begriffe Aryl und Heteroaryl wie zuvor definiert sind und Aryl- und HeteroarylGruppen unabhängig voneinander ein- oder zweifach mit gleichen oder verschiedenen Resten L substituiert sein können, und
wobei X in der Bedeutung Hydroxymethyl ausgeschlossen ist.

Gemäß einer ersten Ausführungsform bedeutet X vorzugsweise Wasserstoff, Cyan, C₁₋₆-Alkyl, C₂₋₆-Alkinyl, C₂₋₆-Alkenyl, C₃₋₇-Cycloalkyl-C₁₋₃-alkyl, C₅₋₇-Cycloalkenyl-C₁₋₃-alkyl, Aryl-C₁₋₃-alkyl, Heteroaryl-C₁₋₃-alkyl, Aminocarbonyl, C₁₋₄-Alkylaminocarbonyl, Di-(C₁₋₃-Alkyl)-aminocarbonyl, Pyrrolidin-1-ylcarbonyl, Piperidin-1-ylcarbonyl, Morpholin-4-ylcarbonyl, C₁₋₄-Alkylcarbonyl, C₁₋₄-Alkoxycarbonyl, C₁₋₄-Alkylcarbonylamino-C₁₋₃-alkyl, N-(C₁₋₄-Alkylcarbonyl)-N-(C₁₋₃-alkyl)-amino-C₁₋₃-alkyl, Arylcarbonylamino-C₁₋₃-alkyl, C₁₋₆-Alkoxy-C₂₋₃-alkyl, C₃₋₇Cycloalkyloxy-C₂₋₃-alkyl, C₅₋₇-Cycloalkenyloxy-C₂₋₃-alkyl, Aryloxy-C₂₋₃-alkyl, Heteroaryloxy-C₂₋₃-alkyl und C₁₋₄-Alkylsulfanyl-C₂₋₃-alkyl,
wobei Alkoxy-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Cyan, Hydroxy, Mercapto, C₁₋₃-Alkoxy und C₁₋₃-Alkyl substituiert sein können, und wobei Methyl-Reste teilweise oder vollständig fluoriert oder einfach mit Chlor substituiert sein können, und wobei Alkylreste mit 2 oder mehr C-Atomen teilweise oder vollständig fluoriert oder ein- oder zweifach mit gleichen oder verschiedenen Substituenten ausgewählt aus Chlor, Cyan, Hydroxy, Mercapto und C₁₋₃-Alkoxy substituiert sein können,
wobei in den vorstehend genannten Cycloalkyl- und Cycloalkenyl-Resten eine oder zwei Methylengruppen unabhängig voneinander durch O, S, CO, SO oder SO₂ ersetzt sein können,und
wobei in N-Heterocycloalkyl-Resten eine Methylengruppe durch CO oder SO₂ ersetzt sein kann,und
wobei die Begriffe Aryl und Heteroaryl wie zuvor definiert sind und Aryl- und HeteroarylGruppen unabhängig voneinander ein- oder zweifach mit gleichen oder verschiedenen Resten L substituiert sein können.

Bedeutet die Gruppe X einen Cycloalkyl- oder Cycloalkenyl-Rest, in dem eine oder zwei Methylengruppen unabhängig voneinander durch O, S, CO, SO oder SO₂ ersetzt sind, so sind bevorzugte Bedeutungen der Gruppe X ausgewählt aus der Gruppe bestehend aus Tetrahydrofuranyl, Tetrahydrofuranonyl, Tetrahydrothienyl, Tetrahydropyranyl, Tetrahydropyranonyl, Dioxanyl und Trioxanyl.

Bedeutet die Gruppe X einen N-Heterocycloalkyl-Rest, in dem eine Methylengruppe durch CO oder SO₂ ersetzt ist, so sind bevorzugte Bedeutungen des Rests X ausgewählt aus der Gruppe bestehend aus Pyrrolidinon, Piperidinon, Piperazinon und Morpholinon.

Besonders bevorzugte Reste der Gruppe X sind Wasserstoff, Cyan, C₁₋₆-Alkyl, C₂₋₆-Alklnyl, C₂₋₆-Alkenyl, C₁₋₄-Alkylcarbonyl, C₁₋₄-Alkoxycarbonyl, Aminocarbonyl, C₁₋₄-Alkylaminocarbonyl, Di-(C₁₋₄-alkyl)aminocarbonyl, C₁₋₄-Alkylcarbonylamino-C₁₋₃-alkyl,
wobei Alkyl-Reste ein- oder mehrfach fluoriert oder einfach mit Chlor oder Cyan substituiert sein können und X in der Bedeutung Alkyl mit 2 oder mehr C-Atomen einen Hydroxy-Substituenten aufweisen kann.

Ganz besonders bevorzugten Reste X sind Wasserstoff, Cyan, Methyl, Ethyl, Propyl, Fluormethyl, Trifluormethyl, Chlormethyl, Cyanmethyl, 1-Hydroxyethyl, 1-Hydroxy-1-methylethyl, 2-Hydroxyethyl, Prop-2-enyl, Prop-2-inyl, Methylcarbonyl, Aminocarbonyl, Methylaminocarbonyl, Methylaminocarbonylmethyl, Methoxycarbonyl und Acetylaminomethyl.

Eine Auswahl der ganz besonders bevorzugten Gruppen X ist Methyl, Ethyl, Fluormethyl, Chlormethyl, Cyanmethyl und Acetylaminomethyl, insbesondere Methyl, Fluormethyl und Cyanmethyl.

Gemäß einer zweiten Ausführungsform bedeutet X vorzugsweise C₁₋₆-Alkoxy-methyl, C₃₋₇-Cycloalkyloxy-methyl, C₅₋₇-Cycloalkenyloxy-methyl, Aryloxy-methyl oder Heteroaryloxymethyl,
wobei die vorstehend genannten Alkoxy-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können, und
wobei in den vorstehend genannten Cycloalkyl- und Cycloalkenyl-Resten eine oder zwei Methylengruppen unabhängig voneinander durch O, S, CO, SO oder SO₂ ersetzt sein können, und
wobei die Begriffe Aryl und Heteroaryl wie zuvor definiert sind und Aryl- und HeteroarylGruppen unabhängig voneinander ein- oder zweifach mit gleichen oder verschiedenen Resten L substituiert sein können.

Gemäß dieser Ausführungsform bevorzugte Bedeutungen des Rests X sind C₁₋₄-Alkyloxymethyl, C₃₋₇-Cycloalkyloxymethyl und Aryloxymethyl, wobei unter Aryl eine Phenyl- oder Naphthylgruppe, insbesondere Phenyl zu verstehen ist, welche ein- oder zweifach mit gleichen oder verschiedenen Substituenten L substituiert sein kann.

Besonders bevorzugte Bedeutungen des Rests X sind hierbei Phenoxymethyl und Methoxymethyl, wobei der Phenylring unsubstituiert oder mit gleichen oder verschiedenen Substituenten L mono- oder disubstituiert ist, insbesondere Methoxymethyl.

Gemäß einer dritten Ausführungsform bedeutet X vorzugsweise Mercaptomethyl, C₁₋₆-Alkylsulfanylmethyl oder C₁₋₆-Alkylcarbonylsulfanylmethyl,
wobei die vorstehend genannten Alkyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können.

Gemäß dieser Ausführungsform bevorzugte Bedeutungen des Rests X sind Mercaptomethyl und C₁₋₄-Alkylsulfanylmethyl.

Besonders bevorzugte Bedeutungen des Rests X sind hierbei Mercaptomethyl und Methylsulfanylmethyl.

Gemäß einer vierten Ausführungsform bedeutet X vorzugsweise Chlormethyl, Brommethyl, lodmethyl, C₁₋₆-Alkylsulfonyloxymethyl, Arylsulfonyloxymethyl oder Aryl-C₁₋₃-alkylsulfonyloxymethyl,
wobei die vorstehend genannten Alkyl-Reste teilweise oder vollständig fluoriert oder ein- oder zweifach chloriert sein können und wobei die vorstehend genannten Aryl-Gruppen ein- oder zweifach mit gleichen oder verschiedenen Resten L substituiert sein können, wobei L vorzugsweise ausgewählt ist aus der Gruppe Fluor, Chlor, Brom, lod, C₁₋₃-Alkyl, Difluormethyl, Trifluormethyl und Cyan.

Die Verbindungen gemäß dieser vierten Ausführungsform eignen sich über ihre beschriebene pharmazeutische Wirkung hinaus insbesondere als Zwischenprodukte in der Synthese von Verbindungen mit SGLT, vorzugsweise SGLT2 inhibierender Wirkung, insbesondere in der Synthese weiterer erfindungsgemäßer Verbindungen.

Besonders bevorzugte Reste X gemäß dieser Ausführungsform sind Brommethyl, lodmethyl, C₁₋₄-Alkylsulfonyloxymethyl, Phenylsulfonyloxymethyl oder Phenylmethylsulfonyloxymethyl, wobei die vorstehend genannten Alkyl-Reste teilweise oder vollständig fluoriert sein können und wobei die vorstehend genannten Phenyl-Gruppen ein- oder zweifach mit gleichen oder verschiedenen Resten L substituiert sein können, wobei L vorzugsweise ausgewählt ist aus der Gruppe Fluor, Chlor, Brom und Methyl.

Ganz besonders bevorzugt ist hierbei X in der Bedeutung Brommethyl oder lodmethyl.

Sind in den Resten oder Gruppen X, R¹ oder R³ Cycloalkyl- oder Cycloalkenyl-Ringe vorhanden, in denen zwei Methylengruppen durch O oder S ersetzt sind oder durch CO, SO oder SO₂ ersetzt sind, so sind diese Methylengruppen vorzugsweise nicht unmittelbar miteinander verbunden. Sind jedoch zwei Methylengruppen durch O und CO ersetzt, so können diese unmittelbar miteinander verbunden sein, so dass eine -O-CO- oder -CO-O-Gruppe gebildet wird. Für den Fall, dass X, R¹ oder R³ eine Cycloalkyl- oder Cycloalkenyl-Gruppe mit einer oder zwei erfindungsgemäß ersetzten Methylengruppen ist, so bedeutet die betreffende Gruppe X, R¹ bzw. R³ vorzugsweise eine Cycloalkyl- oder Cycloalkenyl-Gruppe, in der eine Methylengruppe durch O, S, CO, SO oder SO₂ ersetzt oder eine Ethylengruppe durch -O-CO- oder -CO-O- ersetzt ist.

Nachfolgend werden Bedeutungen weiterer Reste und Substituenten angegeben, die gemäß der allgemeinen Formel I, der Formeln I.1 und I.2 als auch gemäß der zuvor beschriebenen Ausführungsformen als bevorzugt anzusehen sind:

Bevorzugte Bedeutungen des Rests R² sind Wasserstoff, Fluor, Chlor, Brom, Methyl, Hydroxy, Methoxy, Ethoxy, Trifluormethoxy, Cyan, Nitro und durch 1 bis 3 Fluoratome substituiertes Methyl.

- Besonders bevorzugte Bedeutungen des Rests R² sind Wasserstoff, Fluor, Hydroxy, Methoxy, Ethoxy und Methyl, insbesondere Wasserstoff und Methyl.

Für den Fall, dass R¹ und R² an zwei miteinander benachbarte C-Atome des Phenylrings gebunden sind, können R¹ und R² derart miteinander verbunden sein, dass R¹ und R² zusammen vorzugsweise eine C₃₋₄-Brücke bilden, in der eine oder zwei Methyleneinheiten unabhängig voneinander durch O, NR^{N} oder CO ersetzt sein können. Bevorzugt bilden hierbei die miteinander verbundenen Reste R¹ und R² zusammen mit dem Phenylring, mit dem diese verbunden sind, ein bicyclisches Ringsystem ausgewählt aus Dihydroindan, Dihydroindol, Dihydrobenzofuran, Tetrahydrochinolin, Tetrahydrochinolinon, Tetrahydroisochinolin, Tetrahydroisochinolinon und Tetrahydronaphthalin.

Bevorzugte Bedeutungen des Rests R⁴ sind Wasserstoff und Fluor, insbesondere Wasserstoff.

Für den Fall, dass R³ und R⁴ an zwei unmittelbar miteinander benachbarte C-Atome des Phenylrings gebunden sind, können R³ und R⁴ derart miteinander verbunden sein, dass R¹ und R² zusammen vorzugsweise eine C₃₋₄-Brücke bilden, in der eine oder zwei Methyleneinheiten unabhängig voneinander durch O, NR^{N} oder CO ersetzt sein können. Bevorzugt bilden hierbei die miteinander verbundenen Reste R³ und R⁴ zusammen mit dem Phenylring, mit dem diese verbunden sind, ein bicyclisches Ringsystem ausgewählt aus Dihydroindan, Dihydroindol, Dihydrobenzofuran, Tetrahydrochinolin, Tetrahydrochinolinon, Tetrahydroisochinolin, Tetrahydroisochinolinon und Tetrahydronaphthalin.

Bevorzugte Bedeutungen des Rests R⁵ sind Wasserstoff und Fluor, insbesondere Wasserstoff.

Bevorzugte Bedeutungen des Rests Z sind Sauerstoff und Methylen, insbesondere Methylen.

Die Substituenten R^{7a}, R^{7b}, R^{7c} bedeuten unabhängig voneinander vorzugsweise Wasserstoff, (C₁₋₈-Alkyl)oxycarbonyl-, (C₁₋₁₈-Alkyl)carbonyl, Benzoyl, insbesondere Wasserstoff oder (C₁₋₆-Alkyl)oxycarbonyl-, (C₁₋₆-Alkyl)carbonyl, besonders bevorzugt Wasserstoff, Methoxycarbonyl, Ethoxycarbonyl, Methylcarbonyl oder Ethylcarbonyl. Ganz besonders bevorzugt bedeuten R^{7a}, R^{7b} und R^{7c} Wasserstoff.

Die Verbindungen der Formel I, in denen R^{7a}, R^{7b} und R^{7c} eine erfindungsgemäße, von Wasserstoff verschiedene Bedeutung aufweisen, beispielsweise C₁₋₈-Alkylcarbonyl, eignen sich bevorzugt als Zwischenprodukte bei der Synthese von Verbindungen der Formel I in denen R^{7a}, R^{7b} und R^{7c} Wasserstoff bedeuten.

Die Substituenten L sind unabhängig voneinander vorzugsweise ausgewählt aus der Gruppe bestehend aus Fluor, Chlor, Brom, C₁₋₃-Alkyl, Difluormethyl, Trifluormethyl, C₁₋₃-Alkoxy, Difluormethoxy, Trifluormethoxy und Cyan, besonders bevorzugt aus der Gruppe bestehend aus Fluor, Chlor, Methyl, Trifluormethyl, Methoxy und Difluormethoxy.

Besonders bevorzugte Verbindungen der allgemeinen Formel I sind ausgewählt aus der Gruppe der Formeln I.2a bis I.2d, insbesondere der Formel I.2c: 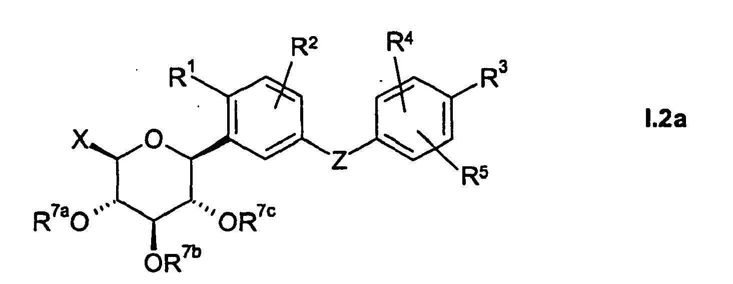 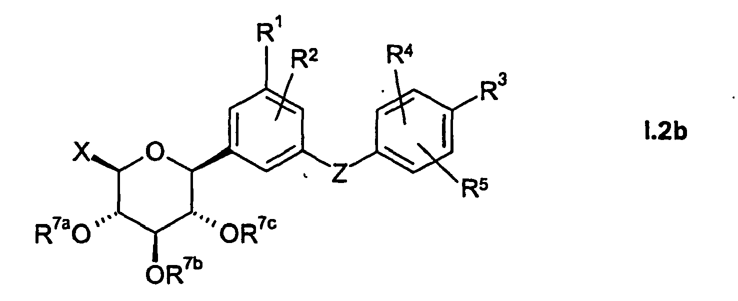 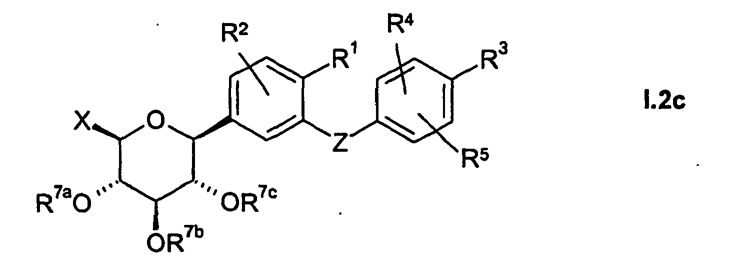 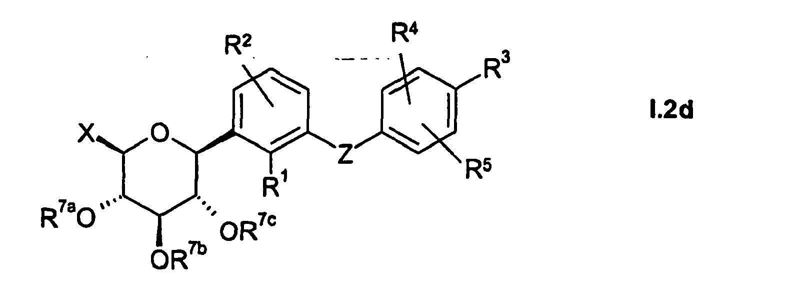 in denen R¹ bis R⁵, X, Z, R^{7a}, R^{7b}, R^{7c} wie zuvor definiert sind.

Ganz besonders bevorzugt sind diejenigen Verbindungen der Formeln I.2a, I2.b, I.2c und I.2d, insbesondere der Formel I.2c, in denen dir Reste R¹ bis R⁵, X, Z, R^{7a}, R^{7b}, R^{7c} die zuvor als bevorzugt angegebenen Bedeutungen aufweisen, insbesondere in denen
- R¹: Wasserstoff, Fluor, Chlor, Brom, C₁₋₆-Alkyl, C₂₋₆-Alkinyl, C₂₋₆-Alkenyl, C₃₋₇-Cycloalkyl, C₅₋₇-Cycloalkenyl, C₁₋₆-Alkyloxy, C₃₋₇-Cycloalkyloxy oder Cyan bedeutet, wobei in Cycloalkyl- und Cycloalkenylgruppen eine oder zwei Methyleneinheiten unabhängig voneinander durch O oder CO ersetzt und Alkyl-, Alkenyl- und Alkinyl-Reste teilweise oder vollständig fluoriert sein können, besonders bevorzugt Wasserstoff, Fluor, Chlor, Brom, Methyl, Ethyl, Isopropyl, Trifluormethyl, Ethinyl, Methoxy, Cyclopentyloxy oder Cyan bedeutet, insbesondere Chlor, Methyl oder Ethinyl, und
- R³: C₁₋₆-Alkyl, C₂₋₆-Alkinyl, C₁₋₄-Alkyloxy, C₃₋₇-Cycloalkyl, C₃₋₇-Cycloalkyloxy oder Hydroxy, insbesondere C₂₋₆-Alkinyl, bedeutet, wobei in den Cycloalkylgruppen eine oder zwei Methyleneinheiten unabhängig voneinander durch O oder CO ersetzt und Alkylreste teilweise oder vollständig fluoriert sein können, besonders bevorzugt Methyl, Ethyl, Ethinyl, Isopropyl, Methoxy, Ethoxy, Isopropyloxy, Difluormethoxy, Cyclopentyloxy, Tetrahydro-furan-3-yloxy oder Hydroxy, insbesondere Ethinyl, bedeutet, und
- X: gemäß einer ersten Ausführungsform Wasserstoff, Cyan, C₁₋₆-Alkyl, C₂₋₆-Alkinyl, C₂₋₆-Alkenyl, C₁₋₄-Alkylcarbonyl, C₁₋₄-Alkoxycarbonyl, Aminocarbonyl, C₁₋₄-Alkylaminocarbonyl, Di-(C₁-₄-alkyl)aminocarbonyl, C₁₋₄-Alkylcarbonylamino-C₁₋₃-alkyl bedeutet, wobei Alkyl-Reste ein- oder mehrfach fluoriert oder einfach mit Chlor oder Cyan substituiert sein können und X in der Bedeutung Alkyl mit 2 oder mehr C-Atomen einen Hydroxy-Substituenten aufweisen kann; besonders bevorzugt Wasserstoff, Cyan, Methyl, Ethyl, Propyl, Fluormethyl, Trifluormethyl, Chlormethyl, Cyanmethyl, 1-Hydroxyethyl, 1-Hydroxy-1-methylethyl, 2-Hydroxyethyl, Prop-2-enyl, Prop-2-inyl, Methylcarbonyl, Aminocarbonyl, Methylaminocarbonyl, Methylaminocarbonylmethyl, Methoxycarbonyl oder Acetylaminomethyl, insbesondere Methyl, Fluormethyl oder Cyanmethyl, bedeutet; oder
gemäß einer zweiten Ausführungsform C₁₋₄-Alkyloxymethyl, C₃₋₇-Cycloalkyloxymethyl oder Aryloxymethyl bedeutet, wobei unter Aryl eine Phenyl- oder Naphthylgruppe, insbesondere Phenyl zu verstehen ist, welche ein- oder zweifach mit gleichen oder verschiedenen Substituenten L substituiert sein kann, besonders bevorzugt Phenoxymethyl oder Methoxymethyl bedeutet, oder
gemäß einer dritten Ausführungsform Mercaptomethyl oder C₁₋₄-Alkylsulfanylmethyl bedeutet, besonders bevorzugt Mercaptomethyl oder Methylsulfanylmethyl bedeutet, oder
gemäß einer vierten Ausführungsform Chlormethyl, Brommethyl, lodmethyl, C₁₋₆-Alkylsulfonyloxymethyl, Arylsulfonyloxymethyl oder Aryl-C₁₋₃-alkyl-sulfonyloxymethyl bedeutet, wobei Alkyl-Reste teilweise oder vollständig fluoriert oder ein-oder zweifach chloriert sein können und wobei Aryl-Gruppen ein- oder zweifach mit gleichen oder verschiedenen Resten L substituiert sein können, wobei L vorzugsweise ausgewählt ist aus der Gruppe Fluor, Chlor, Brom, lod, C₁₋₃-Alkyl, Difluormethyl, Trifluormethyl und Cyan, besonders bevorzugt bedeutet X Brommethyl, lodmethyl, C₁₋₄-Alkylsulfonyloxy-methyl, Phenylsulfonyloxymethyl oder Phenylmethylsulfonyloxymethyl, wobei Alkyl-Reste teilweise oder vollständig fluoriert sein können und wobei Phenyl-Gruppen ein- oder zweifach mit gleichen oder verschiedenen Resten L substituiert sein können, wobei L vorzugsweise ausgewählt ist aus der Gruppe Fluor, Chlor, Brom und Methyl; und
- R²: Wasserstoff, Fluor, Chlor, Brom, Methyl, Hydroxy, Methoxy, Ethoxy, Trifluormethoxy, Cyan, Nitro oder durch 1 bis 3 Fluoratome substituiertes Methyl bedeutet, besonders bevorzugt Wasserstoff, Fluor, Hydroxy, Methoxy, Ethoxy oder Methyl, Insbesondere Wasserstoff oder Methyl bedeutet, und
- R⁴: Wasserstoff oder Fluor, insbesondere Wasserstoff bedeutet, und
- R⁵: Wasserstoff oder Fluor, insbesondere Wasserstoff bedeutet, und
- Z: Sauerstoff oder Methylen, insbesondere Methylen bedeutet, und
- R^{7a}, R^{7b},: R^{7c} unabhängig voneinander Wasserstoff, (C₁₋₈-Alkyl)oxycarbonyl-, (C₁₋₁₈-Alkyl)carbonyl oder Benzoyl, insbesondere Wasserstoff oder (C₁₋₆-Alkyl)oxycarbonyl-, (C₁₋₈-Alkyl)carbonyl bedeuten, besonders bevorzugt Wasserstoff, Methoxycarbonyl, Ethoxycarbonyl, Methylcarbonyl oder Ethylcarbonyl, ganz besonders bevorzugt Wasserstoff bedeuten, und
- L: unabhängig voneinander Fluor, Chlor, Brom, C₁₋₃-Alkyl, Difluormethyl, Trifluormethyl, C₁₋₃-Alkoxy, Difluormethoxy, Trifluormethoxy und Cyan bedeuten,
einschließlich deren Tautomere, deren Stereoisomere, deren Gemische und deren Salze, insbesondere deren physiologisch verträglichen Salze.

Gemäß einer Variante der zuvor angeführten Ausführungsformen sind diejenigen Verbindungen auch bevorzugt, in denen die Phenylgruppe, die den Substituenten R³ trägt, mindestens einen weiteren, von Wasserstoff verschiedenen Substituenten R⁴ und/oder R⁵ aufweist. Nach dieser Variante sind diejenigen Verbindungen auch bevorzugt, die einen Substituenten R⁴ in der Bedeutung Fluor aufweisen.

Der Phenylrest, der den Substituenten R³ trägt, ist vorzugsweise maximal zweifach fluoriert.

Besonders bevorzugte Verbindungen der allgemeinen Formel 1 sind ausgewählt aus der Gruppe:
- (a): 1-Chlor-2-(4-methoxy-benzyl)-4-(6-desoxy-6-fluor-β-D-glucopyranos-1-yl)-benzol,
- (b): 1-Chlor-2-(4-methoxy-benzyl)-4-(6-desoxy-6-chlor-β-D-glucopyranos-1-yl)-benzol,
- (c): 1-Chlor-2-(4-methoxy-benzyl)-4-(6-desoxy-6-acetylamino-β-D-glucopyranos-1-yl)-benzol,
- (d): 1-Chlor-2-(4-methoxy-benzyl)-4-(6-O-phenyl-β-D-glucopyranos-1-yl)-benzol,
- (e): 1-Chlor-2-(4-methoxy-benzyl)-4-(6-desoxy-β-D-glucopyranos-1-yl)-benzol,
- (f): 1-Chlor-2-(4-methoxy-benzyl)-4-(6-cyan-6-desoxy-β-D-glucopyranos-1-yl)-benzol,
- (g): 1-Chlor-2-(4-methoxy-benzyl)-4-(6-desoxy-6-mercapto-β-D-glucopyranos-1-yl)-benzol,

- (h): 1-Chlor-2-(4-methoxy-benzyl)-4-(6-desoxy-6-methylsulfanyl-β-D-glucopyranos-1-yl)-benzol,
- (i): 1-Chlor-2-(4-ethinyl-benzyl)-4-(6-cyan-6-desoxy-β-D-glucopyranos-l-yl)-benzol,
- (j): 1-Brom-2-(4-ethinyl-benzyl)-4-(6-cyan-6-desoxy-β-D-glucopyranos-1-yl)-benzol,
- (k): 1-Methyl-2-(4-ethinyl-benzyl)-4-(6-cyan-6-desoxy-β-D-glucopyranos-1-yl)-benzol,
- (l): 1-Methoxy-2-(4-ethinyl-benzyl)-4-(6-cyan-6-desoxy-β-D-glucopyranos-1-yl)-benzol,
- (m): 1-Chlor-2-(4-ethinyl-benzyl)-4-(6-O-methyl-β-D-glucopyranos-1-yl)-benzol,
- (n): 1-Chlor-2-(4-methoxy-benzyl)-4-(6-O-methyl-β-D-glucopyranos-1-yl)-benzol,
einschließlich deren Tautomere, deren Stereoisomere und deren Gemische.

Im folgenden werden Begriffe, die zuvor und nachfolgend zur Beschreibung der erfindungsgemäßen Verbindungen verwendet werden, näher definiert.

Die Bezeichnung Halogen bezeichnet ein Atom ausgewählt aus der Gruppe bestehend aus F, Cl, Br und I, insbesondere F, Cl und Br.

Die Bezeichnung C₁₋ₙ-Alkyl, wobei n einen Wert von 1 bis 18 besitzen kann, bedeutet eine gesättigte, verzweigte oder unverzweigte Kohlenwasserstoffgruppe mit 1 bis n C-Atomen. Beispiele solcher Gruppen umfassen Methyl, Ethyl, n-Propyl, iso-Propyl, Butyl, iso-Butyl, sec-Butyl, tert-Butyl, n-Pentyl, iso-Pentyl, neo-Pentyl, tert-Pentyl, n-Hexyl, iso-Hexyl, etc..

Der Begriff C₂₋ₙ-Alkinyl, wobei n einen Wert von 3 bis 6 besitzt, bezeichnet eine verzweigte oder unverzweigte Kohlenwasserstoffgruppe mit 2 bis n C-Atomen und einer C≡C-Dreifachbindung. Beispiele solcher Gruppen umfassen Ethinyl, 1-Propinyl, 2-Propinyl, 1-Butinyl, 2-Butinyl, 3-Butinyl, 1-Pentinyl, 2-Pentinyl, 3-Pentinyl, 4-Pentinyl, 1-Hexinyl, 2-Hexinyl, 3-Hexinyl, 4-Hexinyl-, 5-Hexinyl, 4-Methyl-2-pentinyl etc.. Sofern nicht anders angegeben sind Alkinyl-Gruppen über das C-Atom in Position 1 mit dem Rest des Moleküls verbunden. Daher sind Bezeichnungen wie 1-Propinyl, 2-Propinyl, 1-Butinyl, etc. gleichbedeutend mit den Bezeichnungen 1-Propin-1-yl, 2-Propin-1-yl, 1-Butin-1-yl, etc.. Dies gilt in anologer Anwendung auch für C₂₋ₙ-Alkenyl-Gruppen.

Der Begriff C₁₋ₙ-Alkoxy oder C₁₋ₙ-Alkyloxy bezeichnet eine C₁₋ₙ-Alkyl-O-Gruppe, worin C₁₋ₙ-Alkyl wie oben definiert ist. Beispiele solcher Gruppen umfassen Methoxy, Ethoxy, n-Propoxy, iso-Propoxy, n-Butoxy, iso-Butoxy, sec-Butoxy, tert-Butoxy, n-Pentoxy, iso-Pentoxy, neo-Pentoxy, tert-Pentoxy, n-Hexoxy, iso-Hexoxy etc..

Der Begriff C₁₋ₙ-Alkylcarbonyl bezeichnet eine C₁₋ₙ-Alkyl-C(=O)-Gruppe, worin C₁₋ₙ-Alkyl wie oben definiert ist. Beispiele solcher Gruppen umfassen Methylcarbonyl, Ethylcarbonyl, n-Propylcarbonyl, iso-Propylcarbonyl, n-Butylcarbonyl, iso-Butylcarbonyl, sec-Butylcarbonyl, tert-Butylcarbonyl, n-Pentylcarbonyl, iso-Pentylcarbonyl, neo-Pentylcarbonyl, tert-Pentylcarbonyl, n-Hexylcarbonyl, iso-Hexylcarbonyl, etc..

Der Begriff C₃₋ₙ-Cycloalkyl bezeichnet eine gesättigte mono-, bi-, tri- oder spirocarbocyclische Gruppe mit 3 bis n C-Atomen. Beispiele solcher Gruppen umfassen Cyclopropyl, Cyclobutyl, Cyclopentyl, Cyclohexyl, Cycloheptyl, Cyclooctyl, Cyclononyl, Cyclododecyl, Decalin, Bicyclo[3.2.1.]octyl, Spiro[4.5]decyl, Norpinyl, Norbonyl, Norcaryl, Adamantyl, etc.. Vorzugsweise umfasst der Begriff C₃₋₇-Cycloalkyl gesättigte monocyclische Gruppen.

Der Begriff C₃₋ₙ-Cycloalkyloxy bezeichnet eine C₃₋ₙ-Cycloalkyl-O-Gruppe, worin C₃₋ₙ-Cycloalkyl wie oben definiert ist. Beispiele solcher Gruppen umfassen Cyclopropyloxy, Cyclobutyloxy, Cyclopentyloxy, Cyclohexyloxy, Cycloheptyloxy, etc..

Der Begriff C₅₋ₙ-Cycloalkenyl bezeichnet eine C₅₋ₙ-Cycloalkyl-Gruppe, die wie oben definiert ist und zusätzlich mindestens eine ungesättigte C=C-Doppelbindung aufweist.

Der Begriff C₃₋ₙ-Cycloalkylcarbonyl bezeichnet eine C₃₋ₙ-Cycloalkyl-C(=O)-Gruppe, worin C₃₋ₙ-Cycloalkyl wie oben definiert ist.

Der Begriff Tri-(C₁₋₄-alkyl)silyl umfasst Silyl-Gruppen, die gleiche oder zwei oder drei verschiedene Alkylgruppen aufweisen.

Der Begriff Di-(C₁₋₃-alkyl)amino umfasst Amino-Gruppen, die gleiche oder zwei verschiedene Alkylgruppen aufweisen.

Der Begriff N-Heterocycloalkyl bezeichnet einen gesättigten carbocyclischen Ring, der eine Imino-Gruppe im Ring aufweist, und der zusätzlich eine weitere gegebenenfalls substituierte Imino-Gruppe oder ein O- oder S-Atom im Ring aufweisen kann. Unter einer Imino-Gruppe wird die Gruppe -NH- verstanden. Beispiele solcher N-Heterocycloalkyl-Gruppen sind Pyrrolidin, Piperidin, Piperazin, N-Alkyl-piperazin und Morpholin.

Falls in Gruppen, beispielsweise in X, R¹ oder R³, vorkommende Alkyl-Reste substituiert, beispielsweise fluoriert, sein können, so umfasst dies nicht nur Alkyl-Reste in den Gruppen die unmittelbar Alkyl bedeuten, sondern auch in anderen, Alkyl-Reste aufweisenden Bedeutungen, wie beispielsweise Alkoxy, Alkylcarbonyl, Alkoxyalkyl, etc.. So umfasst beispielsweise X, R¹ und R³ in der Bedeutung Alkoxy, wobei Alkylreste teilweise oder vollständig fluoriert sein können, auch Difluormethoxy und Trifluormethoxy.

Die vorstehend und nachfolgend verwendete Schreibweise, bei der in einer Phenyl-gruppe eine Bindung eines Substituenten zur Mitte des Phenylrings hin dargestellt ist, bedeutet, sofern nicht anders angegeben, dass dieser Substituent an jede freie, ein H-Atom tragende Position des Phenylrings gebunden sein kann.

Die erfindungsgemäßen Verbindungen sind unter Anwendung im Prinzip bekannter Syntheseverfahren erhältlich. Bevorzugt werden die Verbindungen nach den nachfolgend näher erläuterten erfindungsgemäßen Herstellungsverfahren erhalten.

Die nachfolgend beschriebenen D-Xylose-Derivate können aus D-Glucose durch Ersatz der 6-Hydroxygruppe oder geeignete Derivatisierung der 6-Hydroxygruppe und anschließender Substitution mit dem gewünschten Rest zugänglich gemacht werden. Solche Transformationen gehören zum allgemeinen Fachwissen oder sind zumindest aus der Fachliteratur als Methoden in der organischen Synthese bekannt und für den Fachmann im Hinblick auf die erfindungsgemäßen Verbindungen ohne weiteren anwendbar.

Zur Herstellung von Verbindungen der allgemeinen Formel I wird gemäß dem erfindungsgemäßen Verfahren a) eine Verbindung der allgemeinen Formel II 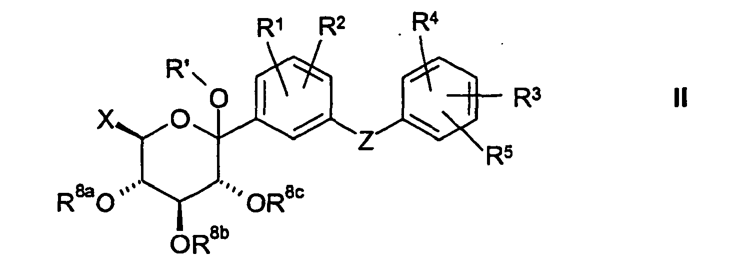 in der X, Z und R', R¹ bis R⁵ wie zuvor definiert sind und
R^{8a}, R^{8b} und R^{8c} wie zuvor definiert sind und beispielsweise unabhängig voneinander Acetyl, Pivaloyl, Benzoyl, tert-Butoxycarbonyl, Benzyloxycarbonyl, Trialkylsilyl, Benzyl oder substituiertes Benzyl bedeuten,
mit einem Reduktionsmittel in Gegenwart einer Säure umgesetzt.

Für die Umsetzung eignen sich als Reduktionsmittel beispielsweise Silane, wie Triethyl-, Tripropyl-, Triisopropyl- oder Diphenylsilan, Natriumborhydrid, Natriumcyanoborhydrid, Zinkborhydrid, Boran, Lithiumaluminiumhydrid, Diisobutylaluminiumhydrid oder Samariumiodid. Die Reduktionen finden vorzugsweise in Gegenwart einer geeigneten Säure, wie z.B. Salzsäure, Toluolsulfonsäure, Trifluoressigsäure, Essigsäure, Bortrifluoridetherat, Trimethylsilyltriflat, Titantetrachlorid, Zinntetrachlorid, Scandiumtriflat oder Zinkiodid statt. In Abhängigkeit vom Reduktionsmittel und der Säure kann die Reaktion in einem Lösungsmittel, wie beispielsweise Methylenchlorid, Chloroform, Acetonitril, Toluol, Hexan, Diethylether, Tetrahydrofuran, Dioxan, Ethanol, Wasser oder Gemischen daraus bei Temperaturen zwischen -60°C und 120°C durchgeführt werden. Ein besonders geeignete Reagenzienkombination besteht beispielsweise aus Triethylsilan und Bortrifluorid-Etherat, die zweckmäßigerweise in Acetonitril oder Dichlormethan bei Temperaturen von -60°C und 60°C zum Einsatz kommt. Des Weiteren kann Wasserstoff in Gegenwart eines Übergangsmetallkatalysators, wie z.B. Palldium auf Kohle oder Raney-Nickel, in Lösungsmitteln wie Tetrahydrofuran, Ethylacetat, Methanol, Ethanol, Wasser oder Essigsäure, für die dargestellte Transformation angewendet werden.

Alternativ werden zur Herstellung von Verbindungen der allgemeinen Formel I gemäß dem erfindungsgemäßen Verfahren b) in einer Verbindung der allgemeinen Formel III 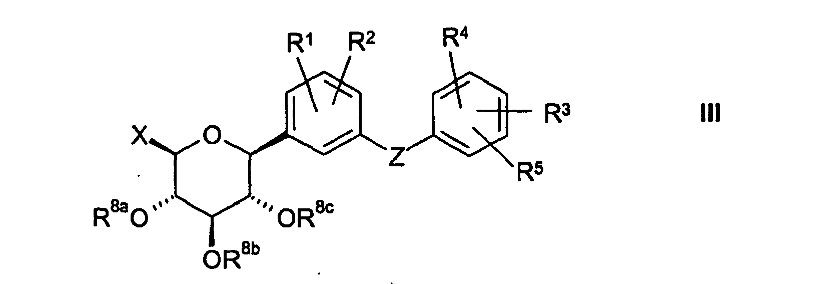 in der X, Z und R¹ bis R⁵ wie zuvor definiert sind und
R^{8a} bis R^{8c} eine der zuvor definierten Schutzgruppen, wie z.B. eine Acyl-, Arylmethyl-, Acetal-, Ketal- oder Silylgruppe bedeuten, die Schutzgruppen abgespalten.

Die Abspaltung eines verwendeten Acyl-, Acetal- oder Ketal-Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder aprotisch, z.B. in Gegenwart von Jodtrimethylsilan, bei Temperaturen zwischen 0 und 120°C, vorzugsweise bei Temperaturen zwischen 10 und 100°C. Die Abspaltung eines Acylrestes kann auch in Gegenwart einer Alkalibase wie Lithiumhydroxid, Natriumhydroxid oder Kaliumhydroxid durchgeführt werden. Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran oder Methanol bei Temperaturen zwischen 0 und 50°C.

Die Abspaltung eines Trimethylsilylrestes erfolgt beispielsweise in Wasser, einem wässrigen Lösemittelgemisch oder einem niederen Alkohol wie Methanol oder Ethanol in Gegenwart einer Base wie Lithiumhydroxid, Natriumhydroxid, Kaliumcarbonat oder Natriummethylat. In wässrigen oder alkoholischen Lösungsmitteln eignen sich ebenfalls Säuren, wie z.B. Salzsäure, Trifluoressigsäure oder Essigsäure. Zur Abspaltung in organischen Lösungsmitteln, wie beispielsweise Diethylether, Tetrahydrofuran oder Dichlormethan, eignen sich auch Fluoridreagenzien, wie z.B. Tetrabutylammoniumfluorid.

Die Abspaltung eines Benzyl-, Methoxybenzyl- oder Benzyloxycarbonylrestes erfolgt vorteilhaft hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig, gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Bei den vorstehend beschriebenen Umsetzungen können gegebenenfalls vorhandene reaktive Gruppen wie Ethinyl-, Hydroxy-, Amino-, Alkylamino- oder Iminogruppen während der Umsetzung durch übliche Schutzgruppen geschützt werden, welche nach der Umsetzung wieder wie u.a. oben beschrieben abgespalten werden.

Beispielsweise kommen als Schutzrest für eine Ethinylgruppe die Trimethylsilyl- oder Triisopropylgruppe in Betracht. Die 2-Hydroxisoprop-2-ylgruppe kann ebenfalls als Schutzgruppe Anwendung finden.

Beispielsweise kommen als Schutzrest für eine Hydroxygruppe die Trimethylsilyl-, Acetyl-, Trityl-, Benzyl- oder Tetrahydropyranylgruppe in Betracht.

Als Schutzreste für eine Amino-, Alkylamino- oder Iminogruppe kommen beispielsweise die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe in Betracht.

Des Weiteren können die so erhaltenen Verbindungen der allgemeinen Formel I selektiv an einer Hydroxygruppe derivatisiert oder die Hydroxygruppe selbst substituiert werden (siehe Beispiele VII, VIII, 1, 2, 4, 5, 6).

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Des Weiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Weiterhin können die erhaltenen Verbindungen in Gemische, beispielsweise in 1:1 oder 1:2 Gemische mit Aminosäuren, insbesondere mit alpha-Aminosäuren wie Prolin oder Phenylalanin, übergeführt werden, die besonders günstige Eigenschaften wie hohe Kristallinität aufweisen können.

Die als Ausgangsstoffe verwendeten Verbindungen der allgemeinen Formeln II und III sind teilweise literaturbekannt oder können nach an sich literaturbekannten Verfahren sowie in Analogie zu den in den Beispielen beschriebenen Verfahren, gegebenenfalls unter zusätzlicher Einführung von Schutzresten, erhalten werden.

Die erfindungsgemäßen Verbindungen sind vorteilhaft auch nach den in den nachfolgenden Beispielen beschriebenen Verfahren zugänglich, wobei diese hierzu auch mit dem Fachmann beispielsweise aus der Literatur bekannten Verfahren, insbesondere den in den WO 98/31697, WO 01/27128, WO 02/083066, WO 03/099836, WO 04/063209 und WO 04/76470 beschriebenen Verfahren, kombiniert werden können.

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf natriumabhängige Glucose-Cotransporter SGLT, vorzugsweise SGLT2.

Die biologischen Eigenschaften der neuen Verbindungen können wie folgt geprüft werden:

Die Fähigkeit der Substanzen die SGLT-2 Aktivität zu hemmen, kann in einem Versuchsaufbau gezeigt werden, in dem eine CHO-K1 Zelllinie (ATCC No. CCL 61) oder alternativ eine HEK293 Zelllinie (ATCC No. CRL-1573), die stabil mit einem Expressionsvektor pZeoSV (Invitrogen, EMBL accession number L36849) transfiziert ist, der die cDNA für die kodierende Sequenz des humanen Natrium Glucose Cotransporters 2 (Genbank Acc. No.NM_003041) enthält (CHO-hSGLT2 bzw. HEK-hSGLT2). Diese Zeillinien transportieren Natrium-abhängig ¹⁴C-markiertes alpha-Methyl-Glucopyranosid (¹⁴C-AMG, Amersham) in das Zellinnere.

Der SGLT-2 Assay wird wie folgt durchgeführt:
CHO-hSGLT2 Zellen werden in Ham's F12 Medium (BioWhittaker) mit 10% fötalem Kälberserum und 250 µg/ml Zeocin (Invitrogen), HEK293-hSGLT2 Zellen in DMEM Medium mit 10% fötalem Kälberserum und 250 µg/ml Zeocin (Invitrogen) kultiviert.
Die Zellen werden von den Kulturflaschen durch zweimaliges Waschen mit PBS und anschließende Behandlung mit Trypsin/EDTA abgelöst. Nach Zugabe von Zellkulturmedium werden die Zellen abzentrifugiert, in Kulturmedium resuspendiert und in einem Casy-cellcounter gezählt. Anschließend werden 40.000 Zellen pro Loch in eine weiße, Poly-D-Lysin beschichtete 96-Loch Platte ausgesät und über Nacht bei 37°C, 5% CO₂ inkubiert. Die Zellen werden zweimal mit 250µl Assaypuffer (Hanks Balanced Salt Solution, 137 mM NaCl, 5,4 mM KCI, 2,8 mM CaCl₂, 1,2 mM MgSO₄ und 10 mM HEPES (pH7,4), 50µg/ml Gentamycin) gewaschen. In jedes Loch werden dann 300 µl Assaypuffer und 5 µl Testverbindung hinzugegeben und für weitere 15 Minuten im Brutschrank inkubiert. Als Negativkontrolle werden 5 µl 10% DMSO eingesetzt. Durch Zugabe von 5 µl ¹⁴C-AMG (0.05 µCi) in jedes Loch wird die Reaktion gestartet. Nach einer 2 stündigen Inkubation bei 37°C, 5% CO₂ werden die Zellen wiederum mit 300 µl PBS (20°C) gewaschen und anschließend durch Zugabe von 25 µl 0.1 N NaOH lysiert (5 min. bei 37°C). Pro Loch werden 200 µl MicroScint20 (Packard) hinzugefügt und für weitere 20 min bei 37°C inkubiert. Nach dieser Inkubation wird die Radioaktivität des aufgenommenen ¹⁴C-AMG in einem Topcount (Packard) mittels eines ¹⁴C-Szintillationsprogramms gemessen.

Zur Bestimmung der Selektivität gegenüber dem humanen SGLT1 wird ein analoger Test aufgebaut, in dem die cDNA für hSGLT1 (Genbank Acc. No. NM000343) statt der hSGLT2 cDNA in CHO-K1 bzw. HEK293 Zellen exprimiert wird.

Alternativ kann für hSGLT1 und hSGLT2 auch die Messung des zellulären Membranpotentials zur biologischen Testung von Substanzen herangezogen werden. Hierzu können die weiter oben beschriebenen Zellmodelle angewendet werden. Für den Test werden 10.000 Zellen pro Loch einer poly-D-Lysin beschichteten schwarzen 384-LochPlatte mit durchsichtigem Boden in Kulturmedium ausgesät und 16 Stunden bei 37°C, 5% CO₂ inkubiert. Anschließend werden die Zellen zweimal mit glucosefreiem HBSS Puffer (12,67 mol/l CaCl₂, 4,93 mmol/l MgCl₂, 4,07 mmol/l MgSO₄, 4,41 mmol/l KH₂PO₄; pH 7,4) gewaschen und mit 20µl HBSS überschichtet. Nach Zugabe von 20 µl Ladepuffer (Membrane Potential Assay-Kit Explorer R8126, Molecular Devices GmbH, Ismaning) und 20 µl der zu testenden Substanz in geeigneter Konzentration wird für weitere 30 min. bei 37°C, 5% CO₂ inkubiert. Die Messung erfolgt im Fluorescent Imaging Plate Reader (Molecular Devices GmbH, Ismaning) bei 485 nm Anregungswellenlänge und wird durch Zugabe von 20µl Stimulationspuffer (140 mM NaCl und 120 mM Glucose) gestartet. Die durch den Glucose-induzierten Na⁺-Einstrom verursachte Depolarisation der Zelle kann als Fluoreszenzänderung gemessen und quantifiziert werden.

Die erfindungsgemäßen Verbindungen der allgemeinen Formel I können beispielsweise EC50-Werte unter 1000 nM, insbesondere unter 200 nM, besonders bevorzugt unter 50 nM aufweisen.

Im Hinblick auf die Fähigkeit, die SGLT Aktivität zu hemmen, sind die erfindungsgemäßen Verbindungen der allgemeinen Formel I und ihre entsprechenden pharmazeutisch akzeptablen Salze prinzipiell geeignet, alle diejenigen Zustände oder Krankheiten zu behandeln und/oder vorbeugend zu behandeln, die durch eine Hemmung der SGLT Aktivität, insbesondere der SGLT-2 Aktivität beeinflusst werden können. Daher sind erfindungsgemäße Verbindungen insbesondere zur Prophylaxe oder Behandlung von Krankheiten, insbesondere Stoffwechselerkrankungen, oder Zuständen wie Diabetes mellitus Typ 1 und Typ 2, diabetische Komplikationen (wie z.B. Retinopathie, Nephropathie oder Neuropathien, diabetischer Fuß, Ulcus, Makroangiopathien), metabolische Azidose oder Ketose, reaktiver Hypoglykämie, Hyperinsulinämie, Glukosestoffwechselstörung, Insulinresistenz, Metabolischem Syndrom, Dyslipidämien unterschiedlichster Genese, Atherosklerose und verwandte Erkrankungen, Adipositas, Bluthochdruck, chronisches Herzversagen, Ödeme, Hyperurikämie geeignet. Darüber hinaus sind diese Substanzen geeignet, die beta-Zelldegeneration wie z.B. Apoptose oder Nekrose von pankreatischen beta-Zellen zu verhindern. Die Substanzen sind weiter geeignet, die Funktionalität von pankreatischen Zellen zu verbessern oder wiederherzustellen, daneben die Anzahl und Größe von pankreatischen beta-Zellen zu erhöhen. Die erfindungsgemäßen Verbindungen sind ebenfalls als Diuretika oder Antihypertensiva einsetzbar und zur Prophylaxe und Behandlung des akuten Nierenversagens geeignet.

Ganz besonders sind die erfindungsgemäßen Verbindungen, einschließlich deren physiologisch verträglichen Salze, zur Prophylaxe oder Behandlung von Diabetes, insbesondere Diabetes mellitus Typ 1 und Typ 2, und/oder diabetischen Komplikationen geeignet.

Die zur Erzielung einer entsprechenden Wirkung bei der Behandlung oder Prophylaxe erforderliche Dosierung hängt üblicherweise von der zu verabreichenden Verbindung, vom Patienten, von der Art und Schwere der Krankheit oder des Zustandes und der Art und Häufigkeit der Verabreichung ab und liegt im Ermessen des zu behandelnden Arztes. Zweckmäßigerweise kann die Dosierung bei intravenöser Gabe im Bereich von 1 bis 100 mg, vorzugsweise 1 bis 30 mg, und bei oraler Gabe im Bereich von 1 bis 1000 mg, vorzugsweise 1 bis 100 mg, jeweils 1 bis 4 x täglich, liegen. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Lösungen, Suspensionen oder Zäpfchen einarbeiten.

Die erfindungsgemäßen Verbindungen können auch in Kombination mit anderen Wirkstoffen, insbesondere zur Behandlung und/oder Prophylaxe der zuvor angegebenen Krankheiten und Zustände verwendet werden. Für solche Kombinationen kommen als weitere Wirksubstanzen insbesondere solche in Betracht, die beispielsweise die therapeutische Wirksamkeit eines erfindungsgemäßen SGLT-Hemmer im Hinblick auf eine der genannten Indikationen verstärken und/oder die eine Reduzierung der Dosierung eines erfindungsgemäßen SGLT-Hemmer erlauben. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. Gl 262570) und -Antagonisten, PPAR-gamma/alpha Modulatoren (z.B. KRP 297), alpha-Glucosidasehemmer (z.B. Acarbose, Voglibose), DPPIV Inhibitoren (z.B. LAF237, MK-431), alpha2-Antagonisten, Insulin und Insulinanaloga, GLP-1 und GLP-1 Analoga (z.B. Exendin-4) oder Amylin. Daneben sind weitere als Kombinationspartner geeignete Wirkstoffe Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha Agonisten, PPAR-delta Agonisten, ACAT Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin, Dexfenfluramin, Axokine, Antagonisten des Cannabinoid1 Rezeptors, MCH-1 Rezeptorantagonisten, MC4 Rezeptor Agonisten, NPY5 oder NPY2 Antagonisten oder β3-Agonisten wie SB-418790 oder AD-9677 ebenso wie Agonisten des 5HT2c Rezeptors.

Daneben ist eine Kombination mit Medikamenten zur Beeinflussung des Bluthochdrucks, des chronischen Herzversagens oder der Atherosklerose wie z.B. A-II Antagonisten oder ACE Inhibitoren, ECE-Inhibitoren, Diuretika, β-Blocker, Ca-Antagonisten, zentral wirksamen Antihypertensiva, Antagonisten des alpha-2-adrenergen Rezeptors, Inhibitoren der neutralen Endopeptidase, Thrombozytenaggregationshemmer und anderen oder Kombinationen daraus geeignet. Beispiele von Angiotensin II Rezeptor Antagonisten sind Candesartan Cilexetil, Kalium Losartan, Eprosartan Mesylat, Valsartan, Telmisartan, Irbesartan, EXP-3174, L-158809, EXP-3312, Olmesartan, Medoxomil, Tasosartan, KT-3-671, GA-0113, RU-64276, EMD-90423, BR-9701, etc.. Angiotensin II Rezeptor Antagonisten werden vorzugsweise zur Behandlung oder Prophylaxe von Bluthochdruck und diabetischen Komplikationen verwendet, oft in Kombination mit einem Diuretikum wie Hydrochlorothiazide.

Zur Behandlung oder Prophylaxe der Gicht ist eine Kombination mit Hamsäuresynthese Inhibitoren oder Urikosurika geeignet.

Zur Behandlung oder Prophylaxe diabetischer Komplikationen kann eine Kombination mit GABA-Rezeptor-Antagonisten, Na-Kanal-Blockem, Topiramat, Protein-Kinase C Inhibitoren, advanced glycation endproduct Inhibitoren oder Aldose Reduktase Inhibitoren erfolgen.

Die Dosis für die zuvor angeführten Kombinationspartner beträgt hierbei zweckmäßigerweise 1/5 der üblicherweise empfohlenen niedrigsten Dosierung bis zu 1/1 der normalerweise empfohlenen Dosierung.

Daher betrifft ein weiterer Gegenstand dieser Erfindung die Verwendung einer erfindungsgemäßen Verbindung oder eines physiologisch verträglichen Salzes solch einer Verbindung in Kombination mit mindestens einem der zuvor als Kombinationspartner beschriebenen Wirkstoffe zur Herstellung eines Arzneimittels, das zur Behandlung oder Prophylaxe von Erkrankungen oder Zuständen geeignet ist, die durch Inhibierung des natriumabhängigen Glucose-Cotransporters SGLT beeinflussbar sind. Hierbei handelt es sich vorzugsweise um eine Stoffwechselerkrankung, insbesondere eine der zuvor angeführten Erkrankungen oder Zustände, ganz besonders Diabetes oder diabetischer Komplikationen.

Die Verwendung der erfindungsgemäßen Verbindung, oder eines physiologisch verträglichen Salzes hiervon, in Kombination mit einem weiteren Wirkstoff kann zeitgleich oder zeitlich versetzt, insbesondere aber zeitnah erfolgen. Bei einer zeitgleichen Verwendung werden beide Wirkstoffe dem Patienten zusammen verabreicht; bei einer zeitlich versetzten Verwendung werden beide Wirkstoffe dem Patienten in einem Zeitraum von kleiner gleich 12, insbesondere kleiner gleich 6 Stunden nacheinander verabreicht.

Folglich betrifft ein weiterer Gegenstand dieser Erfindung ein Arzneimittel, das eine erfindungsgemäße Verbindung oder ein physiologisch verträgliches Salz solch einer Verbindung sowie mindestens einen der zuvor als Kombinationspartner beschriebenen Wirkstoffe neben gegebenenfalls einem oder mehreren inerten Trägerstoffen und/oder Verdünnungsmitteln aufweist.

So weist beispielsweise ein erfindungsgemäßes Arzneimittel eine Kombination aus einer erfindungsgemäßen Verbindung der Formel I oder eines physiologisch verträglichen Salzes solch einer Verbindung sowie mindestens einem Angiotensin II Rezeptor Antagonisten neben gegebenenfalls einem oder mehreren inerten Trägerstoffen und/oder Verdünnungsmitteln auf.

Die erfindungsgemäße Verbindung, oder eines physiologisch verträglichen Salzes, und der damit zu kombinierende weitere Wirkstoff können zusammen in einer Darreichungsform, beispielsweise einer Tablette oder Kapsel, oder getrennt in zwei gleichen oder verschiedenen Darreichungsformen, beispielsweise als sogenanntes kit-of-parts, vorliegen.

Vorstehend und nachfolgend werden in Strukturformeln H-Atome von Hydroxylgruppen nicht in jedem Fall explizit dargestellt. Die nachfolgenden Beispiele sollen die vorliegende Erfindung näher erläutern ohne diese zu beschränken:

### Herstellung der Ausgangsverbindungen:

### Beispiel I

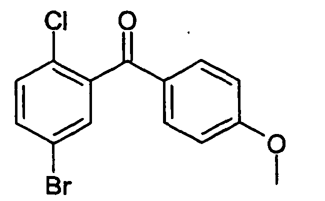

### (5-Brom-2-chlor-phenyl)-(4-methoxy-phenyl)-methanon

Zu einer Mischung von 100 g 5-Brom-2-chlor-benzoesäure in 500 ml Dichlormethan werden 38,3 ml Oxalylchlorid und 0,8 ml Dimethylformamid gegeben. Das Reaktionsgemisch wird 14 h gerührt, danach filtriert und von allen flüchtigen Bestandteilen im Rotationsverdampfer getrennt. Der Rückstand wird in 150 ml Dichlormethan gelöst, die Lösung auf -5°C abgekühlt, und es werden 46,5 g Anisol zugegeben. Danach werden 51,5 g Aluminiumtrichlorid portionsweise so zugegeben, dass die Temperatur nicht über 5°C steigt. Die Lösung wird noch 1 h bei 1-5°C gerührt und anschließend auf Eis gegossen. Die organische Phase wird abgetrennt und die wässrige noch drei Mal mit Dichlormethan extrahiert. Die vereinigten organischen Phasen werden mit wässriger 1 M Salzsäure, zwei Mal mit 1 M Natronlauge und mit gesättigter Natriumchlorid-Lösung gewaschen. Danach wird die organische Phase getrocknet, das Lösungsmittel entfernt und der Rückstand in Ethanol umkristallisiert.
Ausbeute: 86,3 g (64% der Theorie)
Massenspektrum (ESI⁺): m/z = 325/327/329 (Brom+Chlor) [M+H]⁺

### Beispiel II

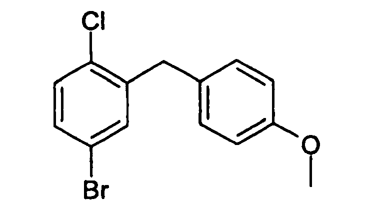

### 4-Brom-1-chlor-2-(4-methoxy-benzyl)-benzol

Eine Lösung von 86,2 g (5-Brom-2-chlor-phenyl)-(4-methoxy-phenyl)-methanon und 101,5 ml Triethylsilan in 75 ml Dichlormethan und 150 ml Acetonitril wird auf 10°C abgekühlt. Dann werden unter Rühren 50,8 ml Bortrifluoridetherat so zugegeben, dass die Temperatur nicht über 20°C steigt. Die Lösung wird 14 h bei Raumtemperatur gerührt, bevor noch einmal 9 ml Triethylsilan und 4,4 ml Bortrifluoridetherat zugegeben werden. Die Lösung wird weitere 3 h bei 45-50°C gerührt und dann auf Raumtemperatur abgekühlt. Es wird eine Lösung von 28 g Kaliumhydroxid in 70 ml Wasser zugesetzt und 2 h gerührt. Danach wird die organische Phase abgetrennt und die wässrige noch drei Mal mit Diisopropylether extrahiert. Die vereinten organischen Phasen werden zwei Mal mit 2 M Kalilauge und einmal mit wässriger Natriumchlorid-Lösung gewaschen und anschließend über Natriumsulfat getrocknet. Nach Entfernen des Lösungsmittels wird der Rückstand in Ethanol verrührt, wieder abgetrennt und bei 60°C getrocknet.
Ausbeute: 50,0 g (61% der Theorie)
Massenspektrum (ESI⁺): m/z = 310/312/314 (Brom+Chlor) [M+H]⁺

### Beispiel III

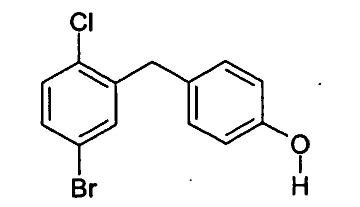

### 4-(5-Brom-2-chlor-benzyl)-phenol

Eine Lösung von 14,8 g 4-Brom-1-chlor-2-(4-methoxy-benzyl)-benzol in 150 ml Dichlormethan wird im Eisbad abgekühlt. Dann werden 50 ml einer 1 M Lösung von Bortribromid in Dichlormethan zugegeben und die Lösung 2 h bei Raumtemperatur gerührt. Die Lösung wird danach wieder im Eisbad gekühlt, und es wird gesättigte KaliumcarbonatLösung zugetropft. Bei Raumtemperatur wird mit wässriger 1 M Salzsäure auf einen pH-Wert von 1 eingestellt, die organische Phase abgetrennt und die wässrige noch drei Mal mit Ethylacetat extrahiert. Die vereinten organischen Phasen werden über Natriumsulfat getrocknet, und das Lösungsmittel wird vollständig entfernt.
Ausbeute: 13,9 g (98% der Theorie)
Massenspektrum (ESI⁻): m/z = 295/297/299 (Br+Cl) [M-H]⁻

### Beispiel IV

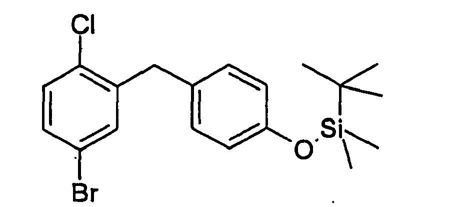

### [4-(5-Brom-2-chlor-benzyl)-Phenoxy]-tert-butyl-dimethyl-silan

Eine Lösung von 13,9 g 4-(5-Brom-2-chlor-benzyl)-phenol in 140 ml Dichlormethan wird im Eisbad abgekühlt. Dann werden 7,54 g tert-Butyldimethylsilylchlorid in 20 ml Dichlormethan gefolgt von 9,8 ml Triethylamin und 0,5 g Dimethylaminopyridin zugegeben. Die Lösung wird 16 h bei Raumtemperatur gerührt und dann mit 100 ml Dichlormethan verdünnt. Die organische Phase wird zwei Mal mit wässriger 1 M Salzsäure und einmal mit wässriger Natriumhydrogencarbonatlösung gewaschen und anschließend über Natriumsulfat getrocknet. Nach Entfernen des Lösungsmittels wird der Rückstand über Kieselgel filtriert (Cyclohexan/Ethylacetat 100:1).
Ausbeute: 16,8 g (87% der Theorie)
Massenspektrum (EI): m/z = 410/412/414 (Br+Cl) [M]⁺

### Beispiel V

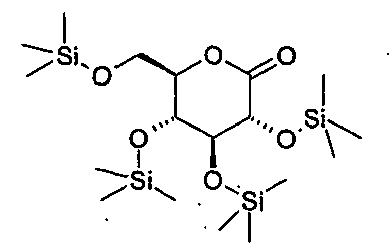

### 2,3,4,6-Tetrakis-O-(trimethylsilyl)-D-glucopyranon

Eine Lösung von 20 g D-Glucono-1,5-lacton und 98,5 ml N-Methylmorpholin in 200 ml Tetrahydrofuran wird auf -5°C abgekühlt. Dann werden 85 ml Trimethylsilylchlorid so zugetropft, dass die Temperatur nicht über 5°C steigt. Die Lösung wird danach 1 h bei Raumtemperatur, 5 h bei 35°C und noch einmal 14 h bei Raumtemperatur gerührt. Nach Zugabe von 300 ml Toluol wird die Lösung im Eisbad abgekühlt, und es werden 500 ml Wasser so zugegeben, dass die Temperatur nicht über 10°C steigt. Die organische Phase wird anschließend abgetrennt und jeweils einmal mit wässriger Natriumdihydrogenphosphatlösung, Wasser und gesättigter wässriger Natriumchloridlösung gewaschen. Das Lösungsmittel wird entfernt, der Rückstand in 250 ml Toluol aufgenommen und das Lösungsmittel erneut vollständig entfernt.
Ausbeute: 52,5 g (ca. 90% rein)
Massenspektrum (ESI⁺): m/z = 467 [M+H]⁺

### Beispiel VI

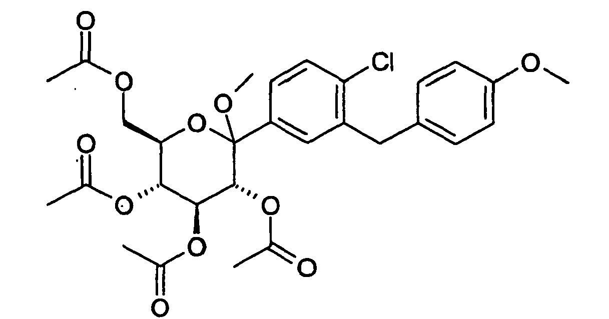

### 1-Chlor-4-(2,3,4,6-tetra-O-acetyl-1-methoxy-D-glucopyranos-1-yl)-2-(4-methoxybenzyl)-benzol

Eine Lösung von 1,0 g 4-Brom-1-chlor-2-(4-methoxy-benzyl)-benzol in 14 ml trockenem Diethylether wird unter Argon auf -80°C abgekühlt. Zu der gekühlten Lösung werden 4,0 ml einer 1,7 M Lösung von tert-Butyllithium in Pentan langsam getropft, und dann wird die Lösung 30 min bei -80°C gerührt. Diese Lösung wird nun über eine Umdrücknadel, die mit Trockeneis gekühlt wird, zu einer -80°C-kalten Lösung von 1,61 g 2,3,4,6-Tetrakis-O-(trimethylsilyl)-D-glucopyranon in 10 ml Diethylether getropft. Die resultierende Lösung wird 4 h bei -78°C gerührt. Danach wird eine Lösung von 0,4 ml Methansulfonsäure in 12 ml Methanol zugegeben und die Lösung 16 h bei Raumtemperatur gerührt. Die Lösung wird anschließend mit Ethyldiisopropylamin neutralisiert und eingeengt. Der Rückstand wird in Toluol aufgenommen und erneut eingeengt. Dann wird der Rückstand in 8 ml Toluol gelöst und 3,4 ml Ethyldiisopropylamin zur Lösung gegeben. Die Lösung wird im Eisbad abgekühlt und danach werden 1,4 ml Acetanhydrid und 0,04 g Dimethylaminopyridin zugegeben. Die Lösung wird 6 h bei Raumtemperatur gerührt und dann mit wässriger Natriumhydrogencarbonatlösung versetzt. Die organische Phase wird abgetrennt und die wässrige mit Ethylacetat extrahiert. Nach Trocknen der vereinten organischen Extrakte über Natriumsulfat und Entfernen des Lösungsmittels wird der Rückstand über Kieselgel chromatografiert (Cyclohexan/Ethylacetat 6:1->1:1).
Ausbeute: 1,55 g (85% der Theorie)
Massenspektrum (ESI⁺): m/z = 610/612 (Chlor) [M+NH₄]⁺

### Beispiel VII

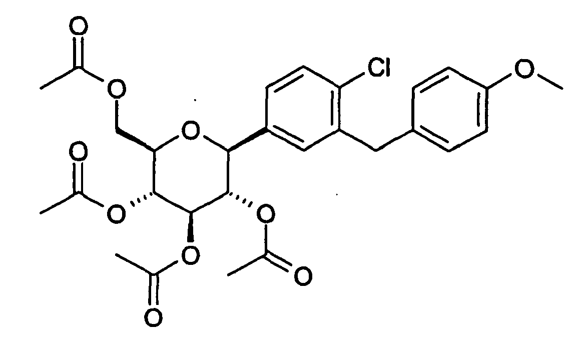

### 1-Chlor-4-(2,3,4,6-tetra-O-acetyl-β-D-glucopyranos-1-yl)-2-(4-methoxy-benzyl)-benzol

Eine Lösung von 1,44 g 1-Chlor-4-(2,3,4,6-tetra-O-acetyl-1-methoxy-D-glucopyranos-1-yl)-2-(4-methoxybenzyl)-benzol in 20 ml Acetonitril und 44 µl Wasser wird im Eisbad abgekühlt. Dann werden 1,2 ml Triethylsilan und 0,26 ml Bortrifluoridetherat zugegeben. Die Lösung wird 1 h im Eisbad und danach bei Raumtemperatur gerührt. Nach 3 und 5 h werden noch einmal jeweils 0,72 ml Triethylsilan und 0,15 ml Bortrifluoridetherat zugegeben. Nach weiteren 12 h Rühren bei Raumtemperatur wird wässrige Natriumhydrogencarbonatlösung zugesetzt, 0,5 h gerührt und dann mit Ethylacetat extrahiert. Die organische Phase wird über Natriumsulfat getrocknet, konzentriert und über Kieselgel chromatografiert (Cyciohexan/Ethylacetat 8:1->1:1).
Ausbeute: 1,12 g (82% der Theorie)
Massenspektrum (ESI⁺): m/z = 580/582 (Chlor) [M+NH₄]⁺

### Beispiel VIII

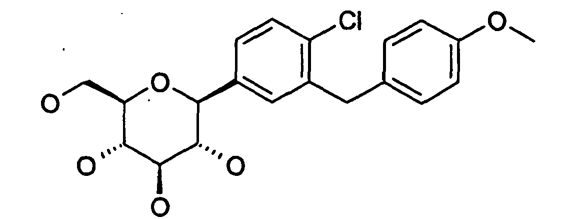

### 1-Chlor-2-(4-methoxy-benzyl)-4-(β-D-glucopyranos-1-yl)-benzol

Zu einer Lösung von 1,00 g 1-Chlor-4-(2,3,4,6-tetra-O-acetyl-β-D-glucopyranos-1-yl)-2-(4-methoxy-benzyl)-benzol in 20 ml Methanol werden 2 ml 4 M Kaliumhydroxidlösung gegeben. Die Lösung wird 8 h bei Raumtemperatur gerührt und dann mit 1 M Salzsäure neutralisiert. Die Lösung wird vom Methanol befreit, mit wässriger Natriumchloridlösung versetzt und mit Ethylacetat extrahiert. Die organische Phase wird über Natriumsulfat getrocknet und das Lösungsmittel entfernt. Der Rückstand wird über Kieselgel gereinigt (Dichlormethan/Methanol 1:0->3:1).
Ausbeute: 0,64 g (91 % der Theorie)
Massenspektrum (ESI⁺): m/z = 412/414 (Chlor) [M+ NH₄]⁺

### Beispiel IX

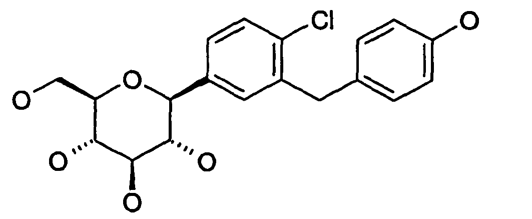

### 1-Chlor-4-(β-D-glucopyranos-1-yl)-2-(4-hydroxybenzyl)-benzol

Eine Lösung von 4,0 g [4-(5-Brom-2-chlor-benzyl)-phenoxy]-tert-butyl-dimethyl-silan in 42 ml trockenem Diethylether wird unter Argon auf -80°C abgekühlt. Zu der'gekühlten Lösung werden 11,6 ml einer 1,7 M Lösung von tert-Butyllithium in Pentan langsam getropft, und dann wird die Lösung 30 min bei -80°C gerührt. Diese Lösung wird nun über eine Umdrücknadel, die mit Trockeneis gekühlt wird, zu einer -80°C-kalten Lösung von 4,78 g 2,3,4,6-Tetrakis-O-(trimethylsilyl)-D-glucopyranon in 38 ml Diethylether getropft. Die resultierende Lösung wird 3 h bei -78°C gerührt. Danach wird eine Lösung von 1,1 ml Methansulfonsäure in 35 ml Methanol zugegeben und die Lösung 16 h bei Raumtemperatur gerührt. Die Lösung wird anschließend mit festem Natriumhydrogencarbonat neutralisiert, es wird Ethylacetat zugegeben und das Methanol zusammen mit dem Ether entfernt. Zur verbleibenden Lösung wird wässrige Natriumhydrogencarbonatlösung gegeben und vier Mal mit Ethylacetat extrahiert. Die organischen Phasen werden über Natriumsulfat getrocknet und eingeengt. Der Rückstand wird in 30 ml Acetonitril und 30 ml Dichlormethan gelöst und die Lösung auf -10°C abgekühlt. Nach Zugabe von 4,4 ml Triethylsilan werden 2,6 ml Bortrifluoridetherat so zugetropft, dass die Temperatur nicht über -5°C steigt. Nach vollständiger Zugabe wird die Lösung noch 5 h bei -5- -10°C gerührt und anschließend durch Zugabe von wässriger Natriumhydrogencarbonatlösung gequencht. Die organische Phase wird abgetrennt und die wässrige vier Mal mit Ethylacetat extrahiert. Die vereinigten organischen Phase werden über Natriumsulfat getrocknet, das Lösungsmittel wird entfernt und der Rückstand über Kieselgel gereinigt. Das danach erhaltene Produkt ist ein ca. 6:1-β/a-Gemisch, welches durch vollständige Acetylierung der Hydroxygruppen mit Acetanhydrid und Pyridin in Dichlormethan und Umkristallisieren des Produkts in Ethanol in das reine β-Anomer überführt werden kann. Das so erhaltene Produkt wird durch Umsetzung in Methanol mit 4 M Kalilauge in die Titelverbindung überführt.
Ausbeute: 1,6 g (46% der Theorie)
Massenspektrum (ESI⁺): m/z = 398/400 (Cl) [M+H]⁺

### Beispiel X

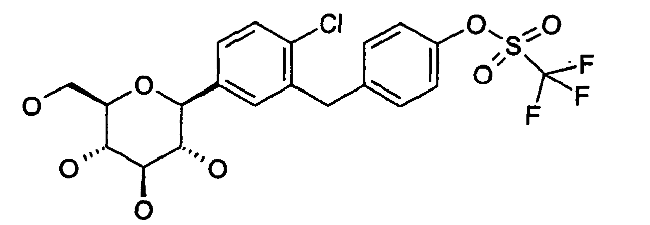

### 1-Chlor-4-(β-D-glucopyranos-1-yl)-2-[4-(trifluormethylsulfonyloxy)-benzyl]-benzol

Zu einer Lösung von 0,38 g 1-Chlor-4-(β-D-glucopyranos-1-yl)-2-(4-hydroxybenzyl)-benzol, 0,21 ml Triethylamin und 0,39 g N,N-Bis-(trifluormethansulfonyl)-anilin in 10 ml trockenem Dichlormethan werden 10 mg 4-Dimethylaminopyridin gegeben. Die Lösung wird 4 h bei Raumtemperatur gerührt und dann mit wässriger Natriumchloridlösung versetzt. Es wird mit Ethylacetat extrahiert, die organischen Extrakte werden über Natriumsulfat getrocknet, und das Lösungsmittel wird entfernt. Der Rückstand wird über Kieselgel chromatografiert (Dichlormethan/Methanol 1:0->4:1).
Ausbeute: 0,33 g (64% der Theorie)
Massenspektrum (ESI⁺): m/z = 530/532 (Cl) [M+NH₄]⁺

### Beispiel XI

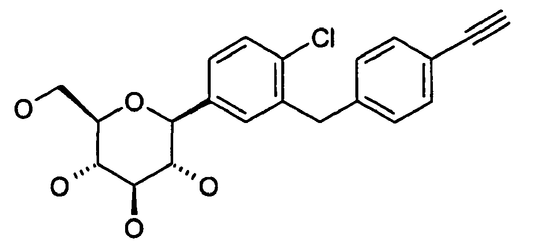

### 1-Chlor-4-(β-D-glucopyranos-1-yl)-2-(4-ethinyl-benzyl)-benzol

Unter Argon werden zu einer Lösung von 0,32 g 1-Chlor-4-(β-D-glucopyranos-1-yl)-2-[4-(trifluormethylsulfonyloxy)-benzyl]-benzol in 3 ml Dimethylformamid 25 mg Kupferiodid, 44 mg Bis-(triphenylphosphin)-palladiumdichlorid, 0,30 ml Triethylamin und zuletzt 0,14 ml Trimethylsilylacetylen gegeben. Der Kolben wird dicht verschlossen und 8 h bei 90°C gerührt. Danach werden noch einmal 25 mg Bis-(triphenylphosphin)-palladiumdichlorid und 0,1 ml Trimethylsilylacetylen zugegeben, und die Lösung wird weitere 10 h bei 90°C gerührt. Anschließend wird wässrige Natriumhydrogencarbonatlösung zugesetzt, drei Mal mit Ethylacetat extrahiert, und die gesammelten organischen Phasen werden über Natriumsulfat getrocknet. Nach Entfernen des Lösungsmittels wird der Rückstand in 5 ml Methanol gelöst und mit 0,12 g Kaliumcarbonat versetzt. Das Gemisch wird 1 h bei Raumtemperatur gerührt und dann mit 1 M Salzsäure neutralisiert. Danach wird das Methanol abgedampft, der Rückstand mit wässriger Natriumchloridlösung versetzt und mit Ethylacetat extrahiert. Die gesammelten organischen Extrakte werden über Natriumsulfat getrocknet, und das Lösungsmittel wird entfernt. Der Rückstand wird über Kieselgel chromatografiert (Dichiormethan/Methanol 1:0->5:1).
Ausbeute: 0,095 g (40% der Theorie)
Massenspektrum (ESI⁺): m/z = 406/408 (Cl) [M+NH₄]⁺

### Beispiel XII

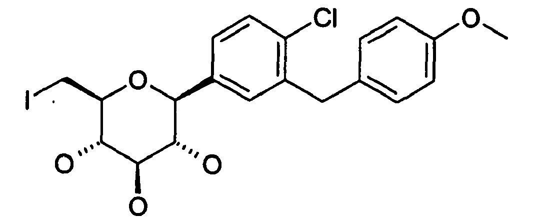

### 1-Chlor-2-(4-methoxy-benzyl)-4-(6-desoxy-6-iod-β-D-glucopyranos-1-yl)-benzol

Zu einer Lösung von 0,60 g 1-Chlor-2-(4-methoxy-benzyl)-4-(β-D-glucopyranos-1-yl)-benzol in 5 ml Dichlormethan werden 0,53 g Triphenylphoshin, 0,13 g Imidazol und 0,48 g Iod gegeben. Die Lösung wird 18 h bei 40-45°C gerührt und danach mit 30 ml Dichlormethan verdünnt. Die Lösung wird mit 1 M Salzsäure gewaschen, über Natriumsulfat getrocknet und vom Lösungsmittel befreit. Der Rückstand wird über Kieselgel gereinigt (Dichlormethan/Methanol 1:0->20:1).
Ausbeute: 0,66 g (87% der Theorie)
Massenspektrum (ESI⁺): m/z = 522/524 (Chlor) [M+NH₄]⁺

### Beispiel XIII

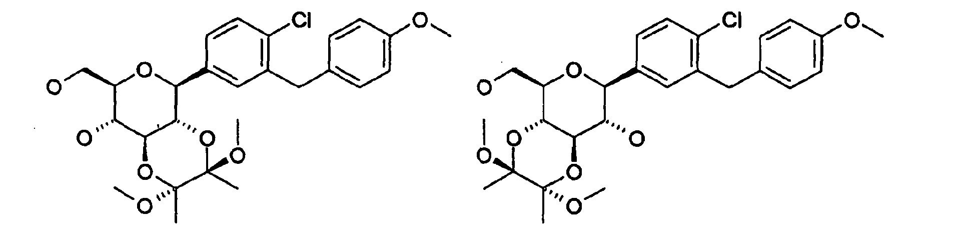

### 1-Chlor-2-(4-methoxy-benzyl)-4-[2,3-O-(2,3-dimethoxy-but-2,3-diyl)-β-D-glucopyranos-1-yl]-benzol und 1-Chlor-2-(4-methoxy-benzyl)-4-[3,4-O-(2,3-dimethoxy-but-2,3-diyl)-β-D-glucopyranos-1-yl]-benzol

Zu einer 60°C-warmen Lösung von 1,0 g 1-Chlor-2-(4-methoxy-benzyl)-4-(β-D-glucopyranos-1-yl)-benzol in 14 ml Methanol werden nacheinander 0,49 ml Diacetyl, 1,2 ml Orthoameisensäuremethylester und 0,64 ml Bortrifluoridetherat gegeben. Die Lösung wird 4 h bei 60°C gerührt und danach auf Raumtemperatur abgekühlt. Bei Raumtemperatur werden 3 ml Triethylamin zugegeben und die Lösung 0,5 h gerührt. Danach wird die Lösung eingeengt und der Rückstand über Kieselgel gereinigt (Cyclohexan/Ethylacetat 4: 1 -> 1:).
Ausbeute:
1-Chlor-2-(4-methoxy-benzyl)-4-[2,3-O-(2,3-dimethoxy-but-2,3-diyl)-β-D-glucopyranos-1-yl]-benzol: 0,70 g (54% der Theorie)
Massenspektrum (ESI⁺): m/z = 526/528 (Chlor) [M+NH₄]⁺
1-Chlor-2-(4-methoxy-benzyl)-4-[3,4-O-(2,3-dimethoxy-but-2,3-diyl)-β-D-glucopyranos-1-yl]-benzol: 0,54 g (42% der Theorie)
Massenspektrum (ESI⁺): m/z = 526/528 (Chlor) [M+NH₄]⁺

### Beispiel XIV

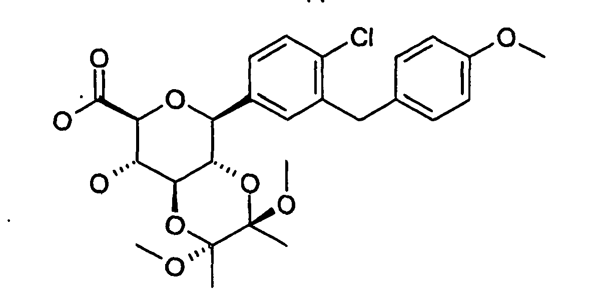

### 5-[4-Chlor-3-(4-methoxy-benzyl)-phenyl]-8-hydroxy-2,3-dimethoxy-2,3-dimethyl-hexahydropyran[3,4-b][1,4]dioxin-7-carbonsäure

Zu einer eiskalten Lösung von 1,40 g 1-Chlor-2-(4-methoxy-benzyl)-4-[2,3-O-(2,3-dimethoxy-but-2,3-diyl)-β-D-glucopyranos-1-yl]-benzol in 28 ml Dichlormethan werden 8 mg 2,2,6,6-Tetramethylpiperidin-1-yloxy gefolgt von einer Lösung aus 0,32 g Kaliumbromid und 0,42 g Tetrabutylammoniumbromid in 57 ml gesättigter Natriumhydrogencarbonatlösung gegeben. Unter heftigem Rühren wird eine Lösung von 5,7 ml gesättigter wässriger Natriumchloridlösung, 2,8 ml gesättigter wässriger Natriumhydrogencarbonatlösung und 7,7 ml Natriumhypchloritlösung (12% aktives Chlor) zugetropft. Nach 1 und 2 h Rühren werden jeweils noch einmal eine Lösung von 1,2 ml gesättigter wässriger Natriumchloridlösung, 0,6 ml gesättigter wässriger Natriumhydrogencarbonatlösung und 1,6 ml Natriumhypchloritlösung (12% aktives Chlor) zugetropft. Nach einer weiteren Stunde im Eisbad wird die Lösung mit 4 M Salzsäure auf pH=1 eingestellt und mit Dichlormethan (1x) und Ethylacetat (3x) ausgeschüttelt. Nach Trocknen über Natriumsulfat wird das Lösungsmittel entfernt und der Rückstand über Kieselgel gereinigt (Dichlormethan/Methanol 1:0-> 10:1).
Ausbeute: 0,92 g (64% der Theorie)
Massenspektrum (ESI⁺): m/z = 540/542 (Chlor) [M+NH₄]⁺

### Analog Beispiel XIV wird folgende Verbindung erhalten:

(1) 7-[4-Chlor-3-(4-methoxy-benzyl)-phenyl]-8-hydroxy-2,3-dimethoxy-2,3-dimethyl-hexahydro-pyran[3,4-b][1,4]dioxin-5-carbonsäure

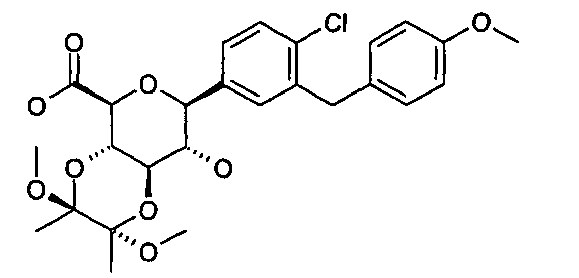

### Massenspektrum (ESI⁺): m/z = 540/542 (Chlor) [M+NH₄]⁺

### Herstellung der Endverbindungen:

### Beispiel 1

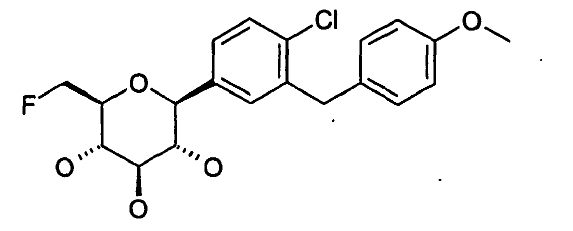

### 1-Chlor-2-(4-methoxy-benzyl)-4-(6-desoxy-6-fluor-β-D-glucopyranos-1-yl)-benzol

Zu einer auf -40°C gekühlten Lösung von 0,10 g 1-Chlor-2-(4-methoxy-benzyl)-4-(β-D-glucopyranos-1-yl)-benzol in 2,5 ml Dichlormethan werden 0,20 ml Diethylaminoschwefeltrifluorid in 0,5 ml Dichlormethan getropft. Die Lösung wird im Kühlbad auf Raumtemperatur erwärmen gelassen und dann 2 h bei Raumtemperatur gerührt. Danach wird die Lösung auf -40°C abgekühlt und mit 2 ml Methanol versetzt. Nach Erwärmen auf Raumtemperatur wird die Lösung eingeengt und der Rückstand über Kieselgel chromatografiert (Dichlormethan/Methanol 1:0->8:1).
Ausbeute: 0,038 g (38% der Theorie)
Massenspektrum (ESI⁺): m/z = 414/416 (Chlor) [M+NH₄]⁺

### Analog Beispiel 1 wird folgende Verbindung erhalten:

### (1) 1-Chlor-2-(4-ethinyl-benzyl)-4-(6-desoxy-6-fluor-β-D-glucopyranos-1-yl)-benzol

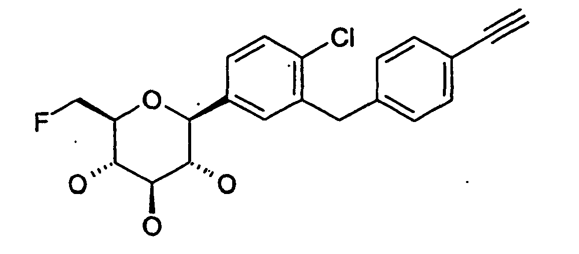

### Beispiel 2

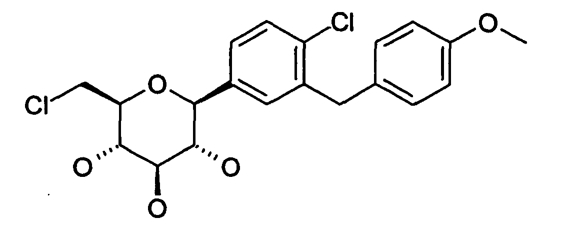

### 1-Chlor-2-(4-methoxy-benzyl)-4-(6-desoxy-6-chlor-β-D-glucopyranos-1-yl)-benzol

Zu einer Lösung von 0,15 g 1-Chlor-2-(4-methoxy-benzyl)-4-(β-D-glucopyranos-1-yl)-benzol und 0,11 g Triphenylphoshin in 2 ml Dichlormethan werden 50 µl Tetrachlorkohlenstoff gegeben. Die Lösung wird 24 h bei 45°C gerührt, bevor noch einmal 0,11 g Triphenylphosphin und 100 µl Tetrachlorkohlenstoff zugegeben werden. Nach weiteren 12 h Rühren bei 45°C wird das Lösungsmittel entfernt und der Rückstand über Kieselgel gereinigt (Dichlormethan/Methanol 1:0->15:1).
Ausbeute: 0,11 g (70% der Theorie)
Massenspektrum (ESI⁺): m/z = 430/432/434 (2 Chlor) [M+NH₄]⁺

### Beispiel 3

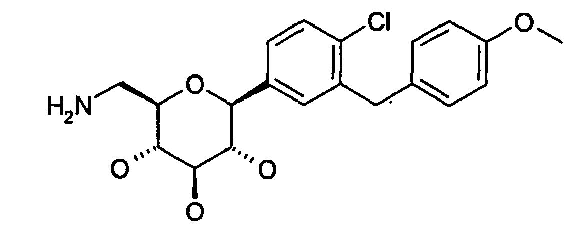

### 1-Chlor-2-(4-methoxy-benzyl)-4-(6-desoxy-6-amino-β-D-glucopyranos-1-yl)-benzol

Zu einer Lösung von 0,20 g 1-Chlor-2-(4-methoxy-benzyl)-4-(β-D-glucopyranos-1-yl)-benzol in 3,5 ml Tetrahydrofuran werden 0,19 g Triphenylphoshin, 0,09 g Phthalimid und zuletzt 0,14 ml Diisopropylazodicarboxylat gegeben. Die Lösung wird 2 h bei Raumtemperatur gerührt und danach mit Methanol verdünnt und eingeengt. Der Rückstand wird über Kieselgel gereinigt (Dichlormethan/Methanol 1:0->8:1). Das gereinigte Phthalsäure geschützte Zwischenprodukt wird in 2 ml Ethanol und 2 ml Toluol gelöst und mit 0,25 g Ethanolamin versetzt. Die Lösung wird 5 h bei 80°C gerührt und dann mit wässriger Kaliumcarbonatlösung versetzt. Die Lösung wird mit Ethylacetat extrahiert, die organischen Extrakte werden über Natriumsulfat getrocknet, und danach wird das Lösungsmittel abdestilliert. Der Rückstand wird über Kieselgel chromatografiert (Dichlormethan/Methanol 1:0->1:1).
Ausbeute: 0,086 g (43% der Theorie)
Massenspektrum (ESI⁺): m/z = 394/396 (Chlor) [M+H]⁺

### Beispiel 4

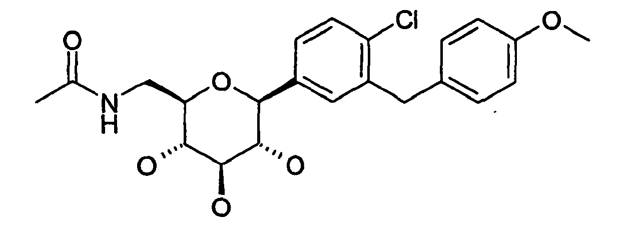

### 1-Chlor-2-(4-methoxy-benzyl)-4-(6-desoxy-6-acetylamino-β-D-glucopyranos-1-yl)-benzol

Zu einer eisgekühlten Lösung von 0,078 g 1-Chlor-2-(4-methoxy-benzyl)-4-(6-desoxy-6-amino-β-D-glucopyranos-1-yl)-benzol und 0,1 ml Pyridin in 2 ml Dichlormethan werden 0,1 ml Acetanhydrid und 10 mg 4-Dimethylaminopyridin gegeben. Die Lösung wird 1 h bei Raumtemperatur gerührt und dann mit 20 ml Dichlormethan verdünnt. Die verdünnte Lösung wird mit 1 M Salzsäure gewaschen, und das Lösungsmittel wird entfernt. Der Rückstand wird in 4 ml Methanol gelöst und die Lösung im Eisbad abgekühlt. Nach Zugabe von 1 ml 4 M Kaliumhydroxidlösung wird 1 h bei Raumtemperatur gerührt. Die Lösung wird dann mit 1 M Salzsäure neutralisiert und das Methanol abdestilliert. Wässrige Natriumhydrogencarbonatlösung wird zugegeben, die Lösung mit Ethylacetat extrahiert, und die organischen Extrakte werden über Natriumsulfat getrocknet. Nach Entfernen des Lösungsmittels wird der Rückstand über Kieselgel chromatografiert (Dichlormethan/Methanol 1:0->8:1).
Ausbeute: 0,078 g (90% der Theorie)
Massenspektrum (ESI⁺): m/z = 436/438 (Chlor) [M+H]⁺

### Beispiel 5

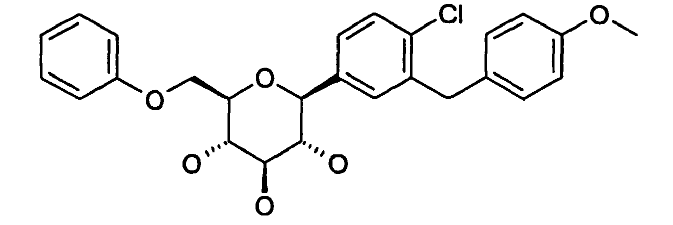

### 1-Chlor-2-(4-methoxy-benzyl)-4-(6-O-phenyl-β-D-glucopyranos-1-yl)-benzol

Zu einer Lösung von 0,20 g 1-Chlor-2-(4-methoxy-benzyl)-4-(β-D-glucopyranos-1-yl)-benzol in 3,5 ml Tetrahydrofuran werden 0,19 g Triphenylphoshin, 56 mg Phenol und zuletzt 0,14 ml Diisopropylazodicarboxylat gegeben. Die Lösung wird bei Raumtemperatur gerührt. Nach 1 h werden noch einmal 0,18 g Triphenylphosphin und 0,14 ml Diisopropylazodicarboxylat, und nach 2 und 4 h werden noch einmal jeweils 50 mg Phenol zugegeben. Nach insgesamt 18 h Rühren bei Raumtemperatur wird Methanol zugegeben und die Lösung vollständig eingeengt. Der Rückstand wird über Kieselgel gereinigt (Dichlormethan/Methanol 1:0->20:1).
Ausbeute: 0,13 g (55% der Theorie)
Massenspektrum (ESI⁺): m/z = 488/490 (Chlor) [M+NH₄]⁺

### Analog Beispiel 5 werden folgende Verbindung erhalten:

### (1) 1-Chlor-2-(4-methoxy-benzyl)-4-(6-cyan-6-desoxy-β-D-glucopyranos-1-yl)-benzol

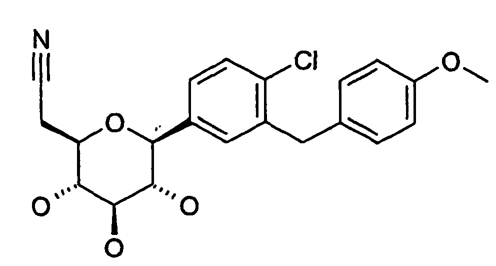

Die Reaktion wird mit 2-Cyan-2-hydroxy-propan statt mit Phenol und bei 45-50°C durchgeführt.
Massenspektrum (ESI⁺): m/z = 421/423 (Chlor) [M+NH₄]⁺

### (2) 1-Chlor-2-(4-ethinyl-benzyl)-4-(6-cyan-6-desoxy-β-D-glucopyranos-1-yl)-benzol

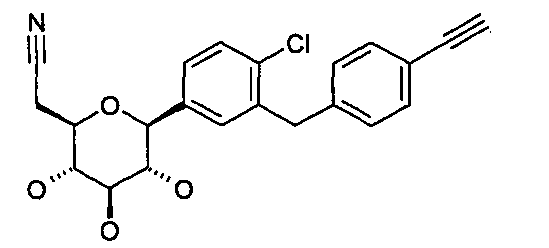

Die Reaktion wird mit 2-Cyan-2-hydroxy-propan statt mit Phenol und bei 45-50°C durchgeführt.

### Beispiel 6

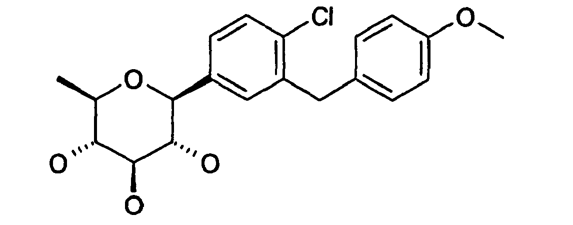

### 1-Chlor-2-(4-methoxy-benzyl)-4-(6-desoxy-β-D-glucopyranos-1-yl)-benzol

Zu einer Lösung von 0,10 g 1-Chlor-2-(4-methoxy-benzyl)-4-(6-desoxy-6-iod-β-D-glucopyranos-1-yl)-benzol in 1,5 ml Toluol werden 0,95 ml Tris-(trimethylsilyl)silan und 12 mg Azobisisobutyronitril gegeben. Die Lösung wird 22 h bei 120°C im dicht verschlossenen Kolben gerührt und dann mit Methanol verdünnt. Die Lösung wird eingeengt, der Rückstand mit 1 M Salzsäure versetzt und mit Ethylacetat extrahiert. Die organischen Extrakte werden über Natriumsulfat getrocknet, und das Lösungsmittel wird anschließend entfernt. Der Rückstand wird über Kieselgel gereinigt (Dichlormethan/Methanol 1:0→10:1).
Ausbeute: 0,062 g (83% der Theorie)
Massenspektrum (ESI⁺): m/z = 396/398 (Chlor) [M+NH₄]⁺

### Beispiel 7

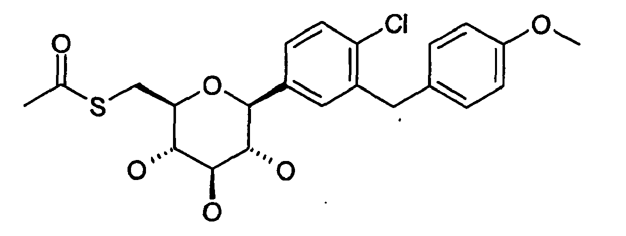

### 1-Chlor-2-(4-methoxy-benzyl)-4-(6-desoxy-6-acetylsulfanyl-β-D-glucopyranos-1-yl)-benzol

Zu einer eisgekühlten Lösung von 0,23 g 1-Chlor-2-(4-methoxy-benzyl)-4-(6-desoxy-6-iod-β-D-glucopyranos-1-yl)-benzol und 0,15 ml Thioessigsäure in 3 ml Dimethylformamid werden 0,58 g Cesiumcarbonat gegeben. Das Gemisch wird 14 h bei Raumtemperatur gerührt und dann mit wässriger Natriumhydrogencarbonatlösung versetzt. Danach wird mit Ethylacetat extrahiert, die organische Phase über Natriumsulfat getrocknet und das Lösungsmittel entfernt. Der Rückstand wird über Kieselgel chromatografiert (Dichlormethan/Methanol 1:0->10:1).
Ausbeute: 0,185 g (90% der Theorie)
Massenspektrum (ESI⁺): m/z = 470/472 (Chlor) [M+NH₄]⁺

### Beispiel 8

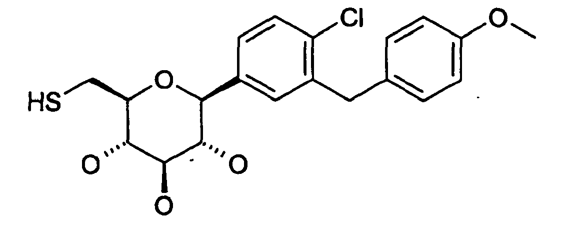

### 1-Chlor-2-(4-methoxy-benzyl)-4-(6-desoxy-6-mercapto-β-D-glucopyranos-1-yl)-benzol

Zu einer eisgekühlten Lösung von 0,145 g 1-Chlor-2-(4-methoxy-benzyl)-4-(6-desoxy-6-acetylsulfanyl-β-D-glucopyranos-1-yl)-benzol in 2,5 ml Methanol werden 0,12 ml einer 4 M Kaliumhydroxidlösung gegeben. Die Lösung wird 1 h bei Raumtemperatur gerührt und dann mit 1 M Salzsäure neutralisiert. Nach Entfernen des Methanols wird wässrige Natriumchloridlösung zugegeben, mit Ethylacetat extrahiert und die organische Phase über Natriumsulfat getrocknet. Die organische Phase wird eingeengt und der Rückstand über Kieselgel gereinigt (Dichlormethan/Methanol 1:0->10:1).
Ausbeute: 0,105 g (80% der Theorie)
Massenspektrum (ESI⁺): m/z = 428/430 (Chlor) [M+NH₄]⁺

### Beispiel 9

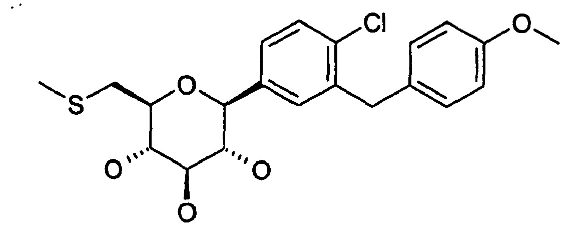

### 1-Chlor-2-(4-methoxy-benzyl)-4-(6-desoxy-6-methylsulfanyl-β-D-glucopyranos-1-yl)-benzol

Zu einer eisgekühlten Lösung von 0,082 g 1-Chlor-2-(4-methoxy-benzyl)-4-(6-desoxy-6-mercapto-β-D-glucopyranos-1-yl)-benzol in 2 ml Dimethylformamid werden 0,095g Cesiumcarbonat gegeben. Die Lösung wird 5 min im Eisbad gerührt und dann mit 16 µl Methyliodid versetzt. Die Lösung wird 1 h bei Raumtemperatur gerührt und dann mit Wasser verdünnt. Die wässrige Phase wird mit Ethylacetat extrahiert, die organische Phase über Natriumsulfat getrocknet und das Lösungsmittel entfernt. Der Rückstand wird über Kieselgel gereinigt (Dichlormethan/Methanol 1:0->10:1).
Ausbeute: 0,014 g (17% der Theorie)
Massenspektrum (ESI⁺): m/z = 442/444 (Chlor) [M+NH₄]⁺

### Beispiel 10

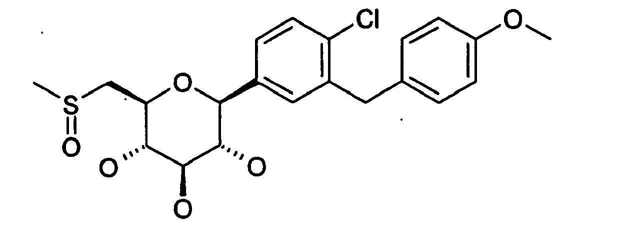

### 1-Chlor-2-(4-methoxy-benzyl)-4-(6-desoxy-6-methylsulfinyl-β-D-glucopyranos-1-yl)-benzol

Zu einer eisgekühlten Lösung von 0,16 g 1-Chlor-2-(4-methoxy-benzyl)-4-(6-desoxy-6-methylsulfanyl-β-D-glucopyranos-1-yl)-benzol in 7 ml 1,1,1,3,3,3-Hexafluorisopropanol werden 0,1 ml Wasserstoffperoxidlösung (35% in Wasser) gegeben. Die Lösung wird 1 h im Eisbad und dann 2 h bei Raumtemperatur gerührt. Danach wird wässrige Natriumthiosulfatlösung und Natriumhydrogencarbonatlösung zugegeben und mit Ethylacetat extrahiert. Die organischen Extrakte werden über Natriumsulfat getrocknet, und das Lösungsmittel wird entfernt. Der Rückstand wird über Kieselgel gereinigt (Dichlormethan/Methanol 1:0->5:1).
Ausbeute: 0,12 g (72% der Theorie)
Massenspektrum (ESI⁺): m/z = 441/443 (Chlor) [M+H]⁺

### Beispiel 11

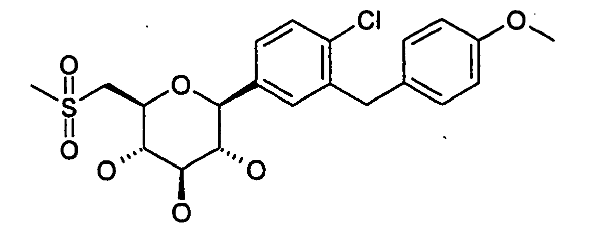

### 1-Chlor-2-(4-methoxy-benzyl)-4-(6-desoxy-6-methylsulfonyl-β-D-glucopyranos-1-yl)-benzol

Zu einer eisgekühlten Lösung von 0,26 g 1-Chlor-2-(4-methoxy-benzyl)-4-(6-desoxy-6-methylsulfanyl-β-D-glucopyranos-1-yl)-benzol in 8 ml Dichlormethan werden 0,31 g meta-Chlorperbenzoesäure (77% in Wasser) gegeben. Die Lösung wird 1 h im Eisbad und dann 2 h bei Raumtemperatur gerührt. Danach wird wässrige Natriumthiosulfatlösung und Natriumhydrogencarbonatlösung zugegeben und mit Ethylacetat extrahiert. Die organischen Extrakte werden über Natriumsulfat getrocknet, und das Lösungsmittel wird entfernt. Der Rückstand wird über Kieselgel gereinigt (Dichlormethan/Methanol 1:0->10:1).
Ausbeute: 0,19 g (68% der Theorie)
Massenspektrum (ESI⁺): m/z = 474/476 (Chlor) [M+NH₄]⁺

### Beispiel 12

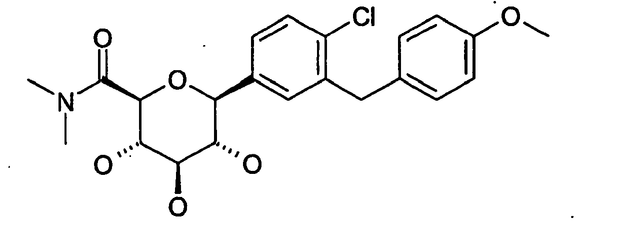

### 6-[4-Chlor-3-(4-methoxy-benzyl)-phenyl]-3,4,5-trihydroxy-tetrahydro-pyran-2-carbonsäuredimethylamid

Zu einer eisgekühlten Lösung von 0,15 g 5-[4-Chlor-3-(4-methoxy-benzyl)-phenyl]-8-hydroxy-2,3-dimethoxy-2,3-dimethyl-hexahydro-pyran[3,4-b][1,4]dioxin-7-carbonsäure in 2 ml Dimethylformamid werden 0,13 g O-(Benzotriazol-1-yl)-N,N,N',N'-tetramethyluroniumtetrafluoroborat gegeben. Nach 15 min Rühren im Eisbad werden 0,2 ml einer Lösung von Dimethylamin in Tetrahydrofuran (2 M) und 0,09 ml Diisopropylethylamin zugegeben. Nach 2 h Rühren im Eisbad wird wässrige Kaliumcarbonatlösung zugegeben und mit Ethylacetat ausgeschüttelt. Nach Trocknen über Natriumsulfat wird das Lösungsmittel entfernt und der Rückstand in 2,5 ml Trifluoressigsäure (80% in Wasser) aufgenommen. Die Lösung wird 2 h bei Raumtemperatur gerührt und dann mit 4 M Kalilauge neutralisiert. Die Lösung wird mit Ethylacetat extrahiert, die organische Phase über Natriumsulfat getrocknet und das Lösungsmittel entfernt. Der Rückstand wird über Kieselgel gereinigt (Dichlormethan/Methanol 1:0->15:1).
Ausbeute: 0,11 g (86% der Theorie)
Massenspektrum (ESI⁺): m/z = 436/438 (Chlor) [M+H]⁺

### Analog Beispiel 12 werden folgende Verbindungen erhalten:

### (1) 6-[4-Chlor-3-(4-methoxy-benzyl)-phenyl]-3,4,5-trihydroxy-tetrahydro-pyran-2-carbonsäureamid

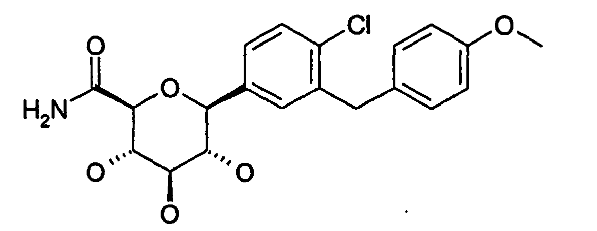

Massenspektrum (ESI⁺): m/z = 408/410 (Chlor) [M+H]⁺

### (2) 6-[4-Chlor-3-(4-methoxy-benzyl)-phenyl]-3,4,5-trihydroxy-tetrahydro-pyran-2-carbonsäuremethylamid

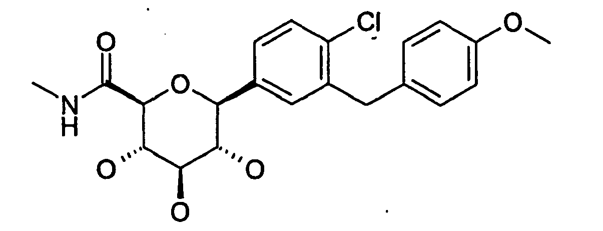

Massenspektrum (ESI⁺): m/z = 422/424 (Chlor) [M+H]⁺

### (3) 6-[4-Chlor-3-(4-methoxy-benzyl)-phenyl]-3,4,5-trihydroxy-tetrahydro-pyran-2-carbonsäurebenzylamid

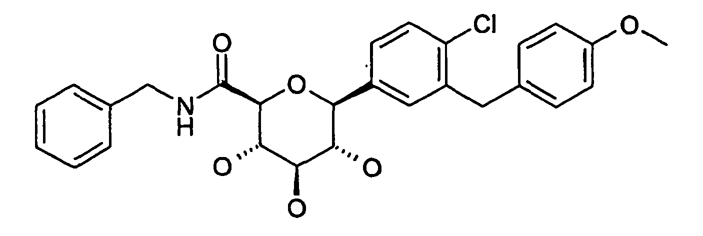

Massenspektrum (ESI⁺): m/z = 408/410 (Chlor) [M+H]⁺

### Beispiel 13

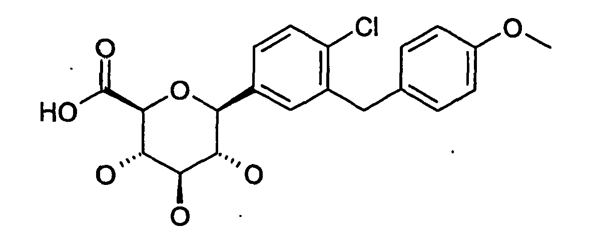

### 6-[4-Chlor-3-(4-methoxy-benzyl)-phenyl]-3,4,5-trihydroxy-tetrahydro-pyran-2-carbonsäure

Zu einer eiskalten Lösung von 0,33 g 1-Chlor-2-(4-methoxy-benzyl)-4-[2,3-O-(2,3-dimethoxy-but-2,3-diyl)-β-D-glucopyranos-1-yl]-benzol in 6,6 ml Dichlormethan werden 2 mg 2,2,6,6-Tetramethylpiperidin-1-yloxy gefolgt von einer Lösung aus 76 mg Kaliumbromid und 0,10 g Tetrabutylammoniumbromid in 13,5 ml gesättigter Natriumhydrogencarbonatlösung gegeben. Unter heftigem Rühren wird eine Lösung von 1,35 ml gesättigter wässriger Natriumchloridlösung, 0,65 ml gesättigter wässriger Natriumhydrogencarboriatlösung und 1,8 ml Nätriurnhypchloritlösung (12% aktives Chlor) zugetropft. Nach 1 und 2 h Rühren werden jeweils noch einmal eine Lösung von 0,25 ml gesättigter wässriger Natriumchloridlösung, 0,13 ml gesättigter wässriger Natriumhydrogencarbonatlösung und 0,32 ml Natriumhypchloritlösung (12% aktives Chlor) zugetropft. Nach einer weiteren Stunde im Eisbad wird die Lösung mit 4 M Salzsäure auf pH=1 eingestellt und mit Dichlormethan (1x) und Ethylacetat (3x) ausgeschüttelt. Nach Trocknen über Natriumsulfat wird das Lösungsmittel entfernt und der Rückstand in 4 ml Trifluoressigsäure (80% in Wasser) aufgenommen. Die Lösung wird 2 h bei Raumtemperatur gerührt und dann mit Wasser verdünnt. Die Lösung wird bis zur Trockene eingeengt, der Rückstand mit 1 M Salzsäure versetzt und mit Ethylacetat extrahiert. Die organische Phase wird über Natriumsulfat getrocknet und das Lösungsmittel vollständig entfernt.
Ausbeute: 0,16 g (60% der Theorie)
Massenspektrum (ESI⁺): m/z = 426/428 (Chlor) [M+NH₄]⁺

### Beispiel 14

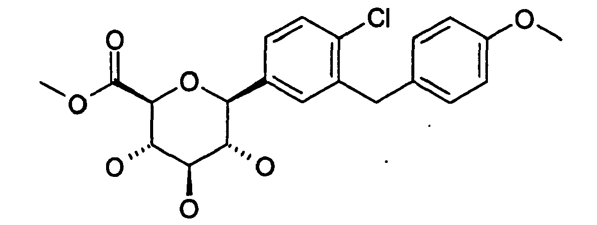

### 6-[4-Chlor-3-(4-methoxy-benzyl)-phenyl]-3,4,5-trihydroxy-tetrahydro-pyran-2-carbonsäuremethylester

Zu einer Lösung von 0,15 g 6-[4-Chlor-3-(4-methoxy-benzyl)-phenyl]-3,4,5-trihydroxy-tetrahydro-pyran-2-carbonsäure in 2 ml Dimethylformamid werden 90 mg Kaliumcarbonat gegeben, und das Gemisch wird 15 min bei Raumtemperatur gerührt. Danach werden 15 µl Methyliodid zugegeben, und das Gemisch wird über Nacht gerührt. Dann wird Wasser zugesetzt und mit Ethylacetat ausgeschüttelt. Nach Trocknen über Natriumsulfat wird das Lösungsmittel entfernt und der Rückstand chromatografiert (Dichlormethan/Methanol 1:0->10:1).
Ausbeute: 0,67 g (47% der Theorie)
Massenspektrum (ESI⁺): m/z = 440/442 (Chlor) [M+NH₄]⁺

### Beispiel 15

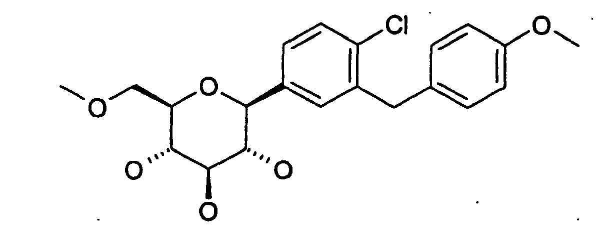

### 1-Chlor-2-(4-methoxy-benzyl)-4-(6-O-methyl-β-D-glucopyranos-1-yl)-benzol

Zu einer eiskalten Lösung von 0,25 g 1-Chlor-2-(4-methoxy-benzyl)-4-[3,4-O-(2,3-dimethoxy-but-2,3-diyl)-β-D-glucopyranos-1-yl]-benzol in 2 ml Dichlormethan werden 73 µl Tetrafluorborsäure (42% in Wasser) gegeben. Danach werden 0,25 ml Trimethylsilyldiazomethan in Hexan (2 M) langsam zugetropft. Nach 20 min Rühren im Eisbad werden noch einmal 0,13 ml, nach weiteren 20 min noch einmal 0,06 ml und nach weiteren 20 min noch einmal 0,06 ml Trimethylsilyldiazomethan (2 M in Hexan) zugetropft. Nach weiteren 30 min im Eisbad wird mit Wasser verdünnt und die Lösung mit Dichlormethan extrahiert. Nach Trocknen über Natriumsulfat wird das Lösungsmittel entfernt und der Rückstand in 2,5 ml Trifluoressigsäure (80% in Wasser) aufgenommen. Die Lösung wird 2 h bei Raumtemperatur gerührt und dann mit 4 M Kalilauge neutralisiert. Die Lösung wird mit Ethylacetat extrahiert, die organische Phase über Natriumsulfat getrocknet und das Lösungsmittel entfernt. Der Rückstand wird über Kieselgel gereinigt (Dichlormethan/Methanol 1:0->20:1).
Ausbeute: 0,10 g (50% der Theorie)
Massenspektrum (ESI⁺): m/z = 426/428 (Chlor) [M+NH₄]⁺

Analog den vorstehend genannten Beispielen und anderen literaturbekannten Verfahren werden auch folgende Verbindungen hergestellt:

| No. | Struktur | No. | Struktur |
|---|---|---|---|
| (9) | 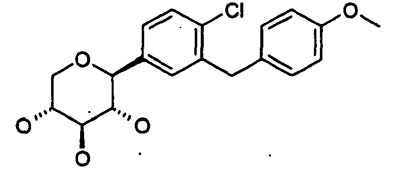 | (10) | 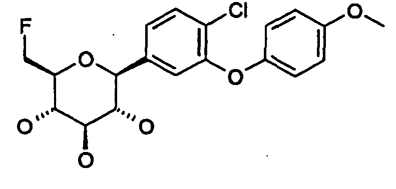 |
| (11) | 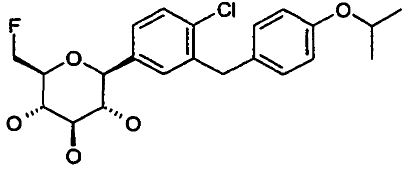 | (12) | 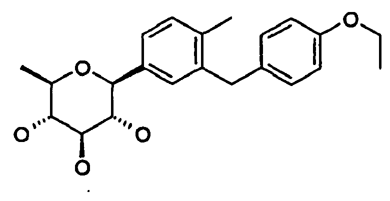 |
| (13) | 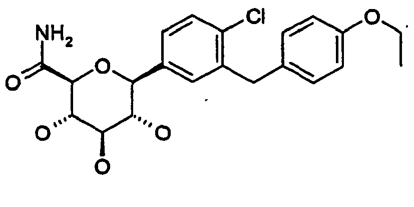 | (14) | 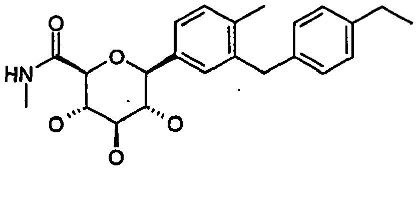 |
| (15) | 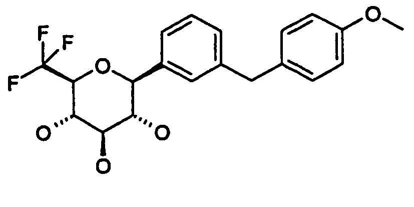 | (16) | 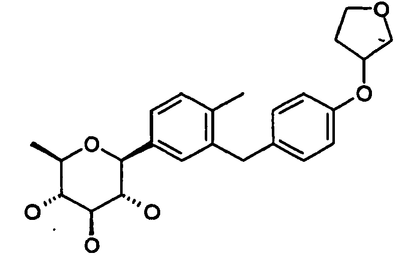 |
| (17) | 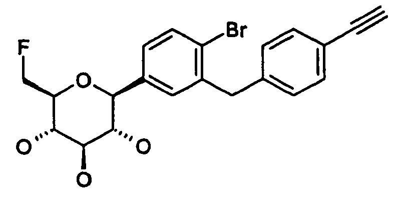 | (18) | 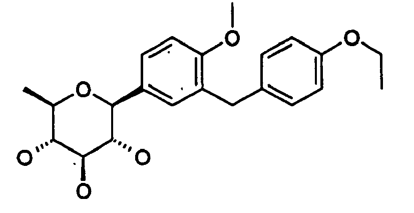 |
| (19) | 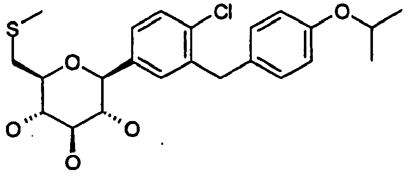 | (20) | 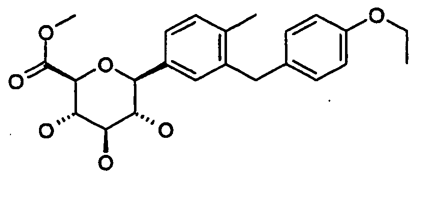 |
| (21) | 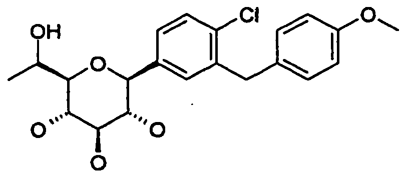 | (22) | 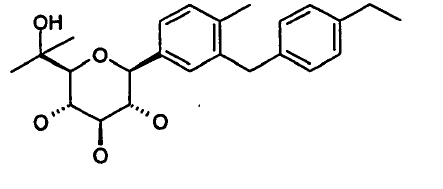 |
| (23) | 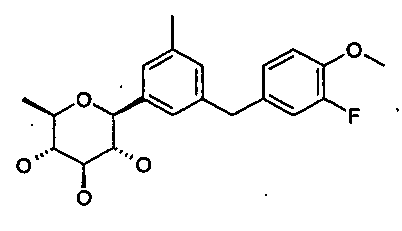 | (24) | 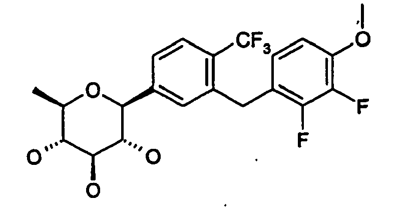 |
| (25) | 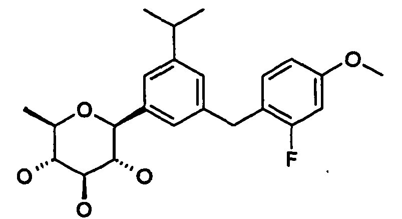 | (26) | 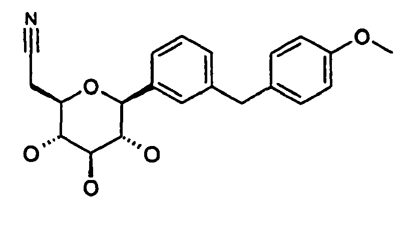 |
| (27) | 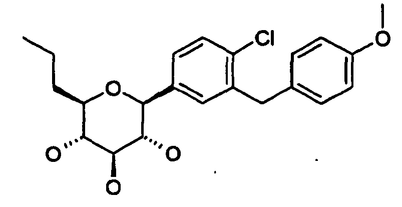 | (28) | 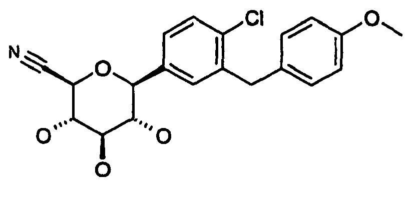 |
| (29) | 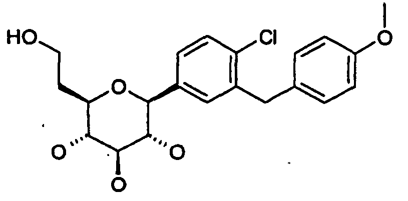 | (30) | 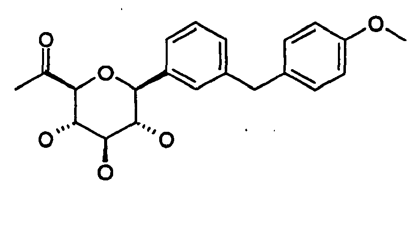 |
| (31) | 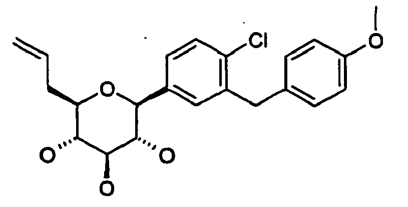 | (32) | 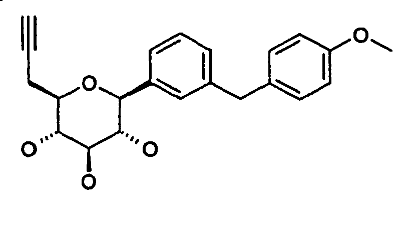 |
| (33) | 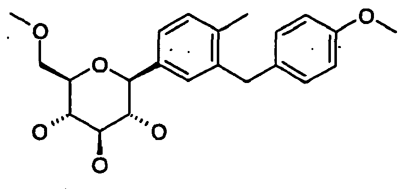 | (34) | 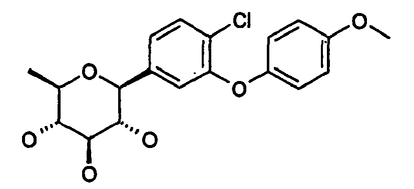 |
| (35) | 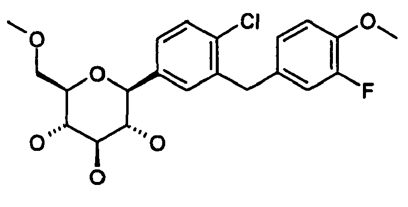 | (36) | 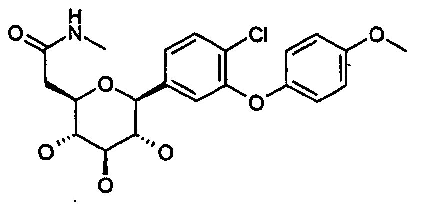 |
| (37) | 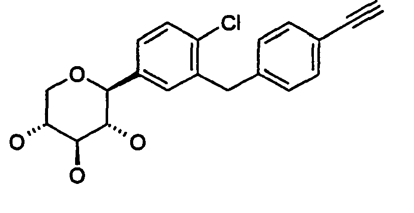 | (38) | 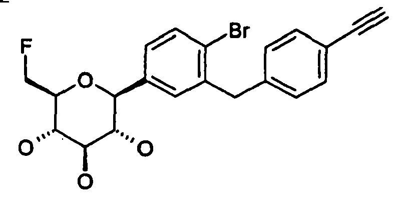 |
| (39) | 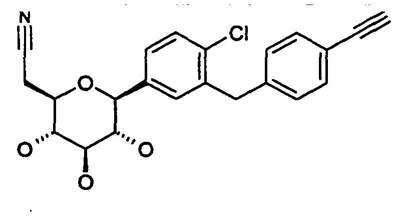 | (40) | 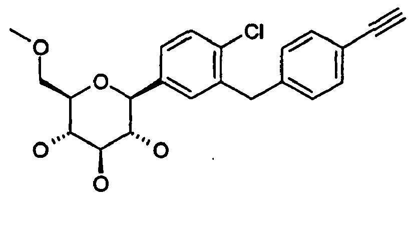 |
| (41) | 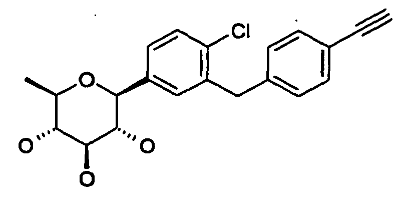 | (42) | 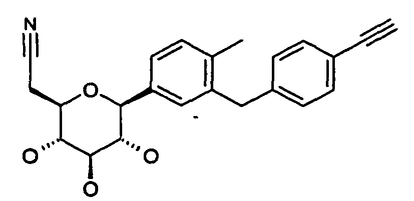 |
| (43) | 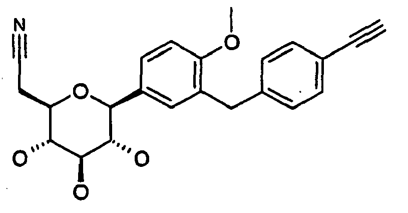 | (44) | 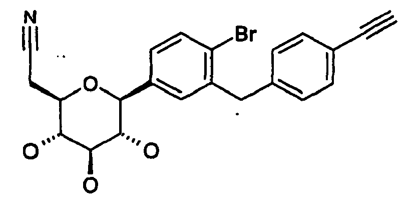 |
| (45) | 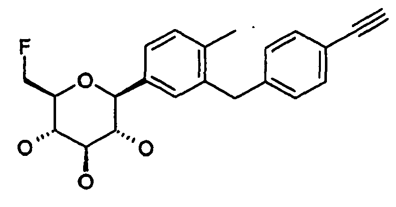 | (46) | 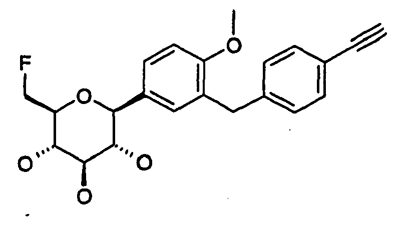 |

Nachfolgend werden Beispiele zu Darreichungsformen beschrieben, worin die Angabe "Wirkstoff" eine oder mehrere erfindungsgemäße Verbindungen, einschließlich deren Salze bedeutet. Im Falle einer der beschriebenen Kombinationen mit einem oder mehreren weiteren Wirksubstanzen umfasst der Begriff "Wirkstoff" auch die weiteren Wirksubstanzen.

### Beispiel A

Tabletten mit 100 mg Wirksubstanz

### Zusammensetzung:

| 1 | Tablette enthält: | |
|---|---|---|
| | Wirksubstanz | 100.0 mg |
| | Milchzucker | 80.0 mg |
| | Maisstärke | 34.0 mg |
| | Polyvinylpyrrolidon | 4.0 mg |
| | Magnesiumstearat | 2.0 mg |
| | | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die pressfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel B

### Tabletten mit 150 mg Wirksubstanz

### Zusammensetzung:

| 1 | Tablette enthält: | |
|---|---|---|
| | Wirksubstanz | 150.0 mg |
| | Milchzucker pulv. | 89.0 mg |
| | Maisstärke | 40.0 mg |
| | Kolloide Kieselgelsäure | 10.0 mg |
| | Polyvinylpyrrolidon | 10.0 mg |
| | Magnesiumstearat | 1.0 mg |
| | | 300.0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel C

### Hartgelatine-Kapseln mit 150 mg Wirksubstanz

### Zusammensetzung:

| 1 | Kapsel enthält: | | |
|---|---|---|---|
| | Wirkstoff | | 150.0 mg |
| | Maisstärke getr. | ca. | 180.0 mg |
| | Milchzucker pulv. | ca. | 87.0 mg |
| | Magnesiumstearat | | 3.0 mg |
| | ca. | | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt.
Die Endmischung wird in Hartgelatine-Kapseln der Größe 1 abgefüllt.
Kapselfüllung : ca. 320 mg
Kapselhülle: Hartgelatine-Kapsel Größe 1.

### Beispiel D

### Suppositorien mit 150 mg Wirksubstanz

### Zusammensetzung:

| 1 | Zäpfchen enthält: | |
|---|---|---|
| | Wirkstoff | 150.0 mg |
| | Polyäthylenglykol 1500 | 550.0 mg |
| | Polyäthylenglykol 6000 | 460.0 mg |
| | Polyoxyäthylensorbitanmonostearat | 840.0 mg |
| | | 2000.0 mg |

### Herstellung:

Nach dem Aufschmelzen der Suppositorienmasse wird der Wirkstoff darin homogen verteilt und die Schmelze in vorgekühlte Formen gegossen.

### Beispiel E

### Ampullen mit 10 mg Wirksubstanz

### Zusammensetzung:

| | |
|---|---|
| Wirkstoff | 10.0 mg |
| 0.01 n Salzsäure s.q. | |
| Aqua bidest ad | 2.0 ml |

### Herstellung:

Die Wirksubstanz wird in der erforderlichen Menge 0.01 n HCl gelöst, mit Kochsalz isotonisch gestellt, sterilfiltriert und in 2 ml Ampullen abgefüllt.

### Beispiel F

### Ampullen mit 50 mg Wirksubstanz

### Zusammensetzung:

| | |
|---|---|
| Wirkstoff | 50.0 mg |
| 0.01 n Salzsäure s.q. | |
| Aqua bidest | ad 10.0 ml |

### Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

## Patentansprüche

1. D-Xylopyranosyl-substituierte Phenyle der allgemeinen Formel I 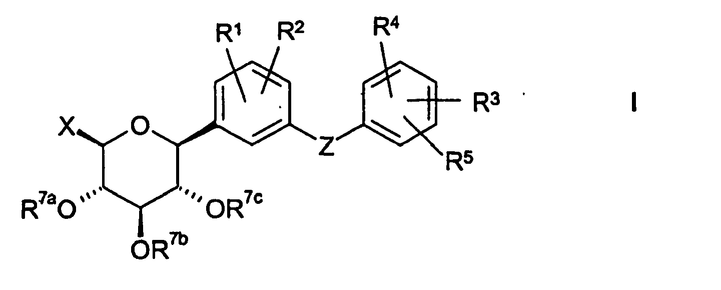 in der
R¹ Wasserstoff, Fluor, Chlor, Brom, C₁₋₆-Alkyl, C₂₋₆-Alkinyl, C₂₋₆-Alkenyl, C₃₋₇-Cycloalkyl, C₃₋₇Cycloalkyl-C₁₋₃-alkyl, C₅₋₇-Cycloalkenyl, C₅₋₇-Cycloalkenyl-C₁-₃-alkyl, C₁₋₄-Alkylcarbonyl, Arylcarbonyl, Heteroarylcarbonyl, Aminocarbonyl, C₁₋₄-Alkylaminocarbonyl, Di-(C₁₋₃-Alkyl)aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)piperazin-1-ylcarbonyl, C₁₋₄-Alkoxycarbonyl, Amino, C₁₋₄-Alkylamino, Di-(C₁₋₃-alkyl)amino, Pyrrolidin-1-yl, Piperidin-1-yl, Morpholin-4-yl, Piperazin-1-yl, 4-(C₁₋₄-Alkyl)piperazin-1-yl, C₁₋₄-Alkylcarbonylamino, C₁₋₆-Alkyloxy, C₃-₇-Cycloalkyloxy, C₅₋₇-Cycloalkenyloxy, Aryloxy, C₁₋₄-Alkylsulfanyl, C₁₋₄-Alkylsulfinyl, C₁₋₄-Alkylsulfonyl, C₃₋₇-Cycloalkylsulfanyl, C₃₋₇Cycloalkylsulfinyl, C₃₋₇-Cycloalkylsulfonyl, C₅₋₇-Cycloalkenylsulfanyl, C₅₋₇-Cycloalkenylsulfinyl, C₅₋₇-Cycloalkenylsulfonyl, Arylsulfanyl, Arylsulfinyl, Arylsulfonyl, Hydroxy, Cyan oder Nitro bedeutet,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und wobei in N-Heterocycloalkyl-Resten eine Methylengruppe durch CO oder SO₂ ersetzt sein kann, und
R² Wasserstoff, Fluor, Chlor, Brom, Hydroxy, C₁₋₄-Alkyl, C₁₋₄-Alkoxy, Cyan oder Nitro bedeutet, wobei Alkyl-Reste ein- oder mehrfach mit Fluor substituiert sein können, oder
für den Fall, dass R¹ und R² an zwei miteinander benachbarte C-Atome des Phenylrings gebunden sind, können R¹ und R² derart miteinander verbunden sein, dass R¹ und R² zusammen eine C₃₋₅-Alkylen- oder C₃₋₅-Alkenylen-Brücke bilden, die teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert kann und in der eine oder zwei Methylengruppen unabhängig voneinander durch O, S, CO, SO, SO₂ oder NR^{N} ersetzt sein können,
R³ Wasserstoff, Fluor, Chlor, Brom, C₁₋₆Alkyl, C₂₋₆-Alkinyl, C₂₋₆-Alkenyl, C₃₋₇-Cycloalkyl, C₃₋₇-Cycloalkyl-C₁₋₃-alkyl, C₅₋₇-Cycloalkenyl, C₅₋₇Cycloalkenyl-C₁₋₃-alkyl, Aryl, Heteroaryl, C₁₋₄-Alkylcarbonyl, Arylcarbonyl, Heteroarylcarbonyl, Aminocarbonyl, C₁₋₄-Alkylaminocarbonyl, Di-(C₁₋₃-Alkyl)aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)piperazin-1-ylcarbonyl, Hydroxycarbonyl, C₁₋₄-Alkoxycarbonyl, C₁₋₄-Alkylamino, Di-(C₁₋₃-alkyl)amino, Pyrrolidin-1-yl, Piperidin-1-yl, Morpholin-4-yl, Piperazin-1-yl, 4-(C₁₋₄-Alkyl)piperazin-1-yl, C₁₋₄-Alkylcarbonylamino, Arylcarbonylamino, Heteroarylcarbonylamino, C₁₋₄-Alkylsulfonylamino, Arylsulfonylamino, C₁₋₆-Alkoxy, C₃₋₇-Cycloalkyloxy, C₅-₇-Cycloalkenyloxy, Aryloxy, Heteroaryloxy, C₁₋₄-Alkylsulfanyl, C₁₋₄-Alkylsulfinyl, C₁₋₄-Alkylsulfonyl, C₃₋₇-Cycloalkylsulfanyl, C₃₋₇-Cycloalkylsulfinyl, C₃-₇-Cycloalkylsulfonyl, C₅₋₇-Cycloalkenylsulfanyl, C₅₋₇-Cycloalkenylsulfinyl, C₅₋₇-Cycloalkenylsulfonyl, Arylsulfanyl, Arylsulfinyl, Arylsulfonyl, Amino, Hydroxy, Cyan oder Nitro bedeutet,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und
wobei in N-Heterocycloalkyl-Resten eine Methylengruppe durch CO oder SO₂ ersetzt sein kann, und
R⁴ Wasserstoff, Fluor, Chlor, Brom, lod, Cyan, Nitro, C₁₋₃-Alkyl, C₁₋₃-Alkoxy oder durch 1 bis 3 Fluoratome substituiertes Methyl- oder Methoxy bedeutet, oder
für den Fall, dass R³ und R⁴ an zwei miteinander benachbarte C-Atome des Phenylrings gebunden sind, können R³ und R⁴ derart miteinander verbunden sein, dass R³ und R⁴ zusammen eine C₃₋₅-Alkylen- oder C₃₋₅-Alkenylen-Brücke bilden, die teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert kann urid in der eine oder zwei Methylengruppen unabhängig voneinander durch O, S, CO, SO, SO₂ oder NR^{N} ersetzt sein können,
R⁵ Wasserstoff, Fluor, Chlor, Brom, lod, Cyan, Nitro, C₁₋₃-Alkyl, C₁₋₃-Alkoxy oder durch 1 bis 3 Fluoratome substituiertes Methyl- oder Methoxy bedeutet, und
R^{N} unahbängig voneinander H oder C₁₋₄-Alkyl bedeuten,
L unabhängig voneinander ausgewählt aus der Gruppe bestehend aus Fluor, Chlor, Brom, lod, C₁₋₃-Alkyl, Difluormethyl, Trifluormethyl, C₁₋₃-Alkoxy, Difluormethoxy, Trifluormethoxy und Cyan,
R^{7a}, R^{7b}, R^{7c}, unabhängig voneinander eine Bedeutung ausgewählt aus der Gruppe Wasserstoff, (C₁₋₁₈-Alkyl)carbonyl, (C₁₋₁₈-Alkyl)oxycarbonyl, Arylcarbonyl und Aryl-(C₁₋₃-alkyl)-carbonyl besitzen,
X Wasserstoff, C₁₋₆-Alkyl, C₂₋₆-Alkinyl, C₂₋₆-Alkenyl, C₃₋₇-Cycloalkyl, C₃₋₇-Cycloalkyl-C₁₋₃-alkyl, C₅₋₇-Cycloalkenyl, C₅₋₇-Cycloalkenyl-C₁₋₃-alkyl, Aryl, Aryl-C₁₋₃-alkyl, Heteroaryl, Heteroaryl-C₁₋₃-alkyl, C₁₋₄-Alkylcarbonyl, Arylcarbonyl, Aminocarbonyl, C₁₋₄-Alkylaminocarbonyl, Di-(C₁₋₃-Alkyl)aminocarbonyl, (Aryl-C₁₋₃-alkyl)aminocarbonyl, Pyrrolidin-1-ylcarbonyl, Piperidin-1-ylcarbonyl, Morpholin-4-ylcarbonyl, Hydroxycarbonyl, C₁₋₄-Alkoxycarbonyl, C₁₋₄-Alkylcarbonylamino-C₁₋₃-alkyl, N-(C₁₋₄-Alkylcarbonyl)-N-(C₁₋₃-alkyl)-amino-C₁₋₃-alkyl, Arylcarbonylamino-C₁₋₃-alkyl, C₁₋₄-Alkylsulfonylamino-C₁₋₃-alkyl, Arylsulfonylamino-C₁₋₃-alkyl, C₁₋₆-Alkoxy-C₁-₃-alkyl, C₃₋₇-Cycloalkyloxy-C₁₋₃-alkyl, C₅₋₇-Cycloalkenyloxy-C₁₋₃-alkyl, Aryloxy-C₁₋₃-alkyl, Heteroaryloxy-C₁₋₃-alkyl, C₁₋₄-Alkylsulfanyl-C₁₋₃-alkyl, C₁₋₄-Alkylsulfinyl-C₁₋₃-alkyl, C₁₋₄-Alkylsulfinyl, C₁₋₄-Alkylsulfonyl, C₁₋₄-AlkylsulfonylC₁₋₃-alkyl, C₁₋₄-Arylsulfanyl-C₁₋₃-alkyl, Arylsulfonyl-C₁₋₃-alkyl, Aryl-C₁₋₃-alkylsulfonyl-C₁₋₃-alkyl, C₁₋₄-Alkylsulfonyloxy-C₁₋₃-alkyl, Arylsulfonyloxy-C₁₋₃-alkyl, Aryl-C₁₋₃-alkyl-sulfonyloxy-C₁₋₃-alkyl, C₃₋₇-Cycloalkylsulfanyl-C₁₋₃-alkyl, C₃₋₇-Cycloalkylsulfinyl, C₃₋₇-Cycloalkylsulfonyl, C₅₋₇-Cycloalkenylsulfanyl-C₁₋₃-alkyl, C₅₋₇-Cycloalkenylsulfinyl, C₅₋₇-Cycloalkenylsulfonyl, C₁₋₄-Alkylcarbonylsulfanyl-C₁₋₃-alkyl oder Cyan bedeuten,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Cyan, Hydroxy, Mercapto, C₁₋₃-Alkoxy und C₁₋₃-Alkyl substituiert sein können, und
wobei in Cycloalkyl- und Cycloalkenyl-Resten eine oder zwei Methylengruppen unabhängig voneinander durch O, S, CO, SO oder S0₂ ersetzt sein können, und
wobei in N-Heterocycloalkyl-Resten eine Methylengruppe durch CO oder SO₂ ersetzt sein kann, und
wobei X in der Bedeutung Hydroxymethyl ausgeschlossen ist,
Z Sauerstoff, Methylen, Dimethylmethylen, Difluormethylen oder Carbonyl ;
wobei unter den bei der Definition der vorstehend genannten Reste erwähnten Arylgruppen Phenyl- oder Naphthylgruppen zu verstehen sind, welche unabhängig voneinander ein- oder zweifach mit gleichen oder verschiedenen Resten L substituiert sein können; und
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl-, Pyridyl- oder Imidazolylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
wobei die vorstehend erwähnten Heteroarylgruppen unabhängig voneinander ein- oder zweifach mit gleichen oder verschiedenen Resten L substituiert sein können;
wobei unter dem bei der Definition der vorstehend erwähnten Reste erwähnten N-Heterocycloalkyl-Rest ein gesättigter carbocyclischer Ring, der eine Imino-Gruppe im Ring aufweist, zu verstehen ist, der eine weitere gegebenenfalls substituierte Imino-Gruppe oder ein O- oder S-Atom im Ring aufweisen kann, und
wobei, soweit nichts anderes erwähnt wurde, die vorstehend erwähnten Alkylgruppen geradkettig oder verzweigt sein können,
deren Tautomere, deren Stereoisomere, deren Gemische und deren Salze, insbesondere deren physiologisch verträglichen Salze.

2. D-Xylopyranosyl-substituierte Phenyle gemäß Anspruch 1, **gekennzeichnet durch** die Formel I.2 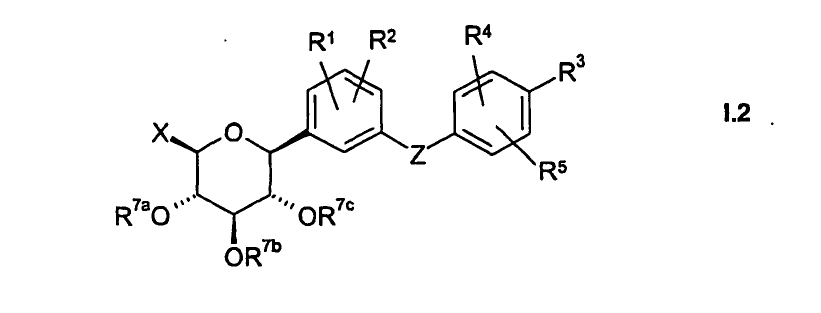 worin R¹ bis R⁵, X, Z, R^{7a}, R^{7b} , R^{7c} die Bedeutungen gemäß Anspruch 1 aufweisen.

3. D-Xylopyranosyl-substituierte Phenyle gemäß Anspruch 1, **gekennzeichnet durch** die Formel 1.2c 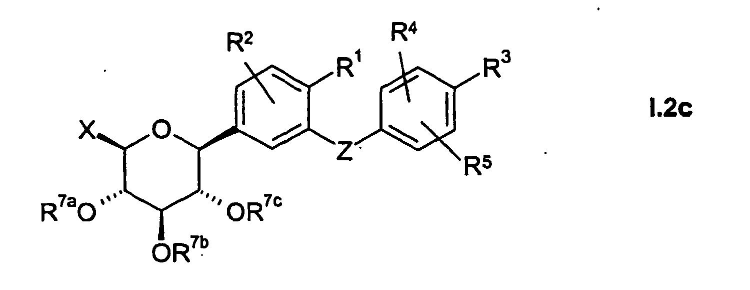 worin R¹ bis R⁶, X, Z, R^{7a}, R^{7b}, R^{7c} die Bedeutungen gemäß Anspruch 1 aufweisen.

4. D-Xylopyranosyl-substituierte Phenyle gemäß einem oder mehreren der Ansprüche 1 bis 3, **dadurch gekennzeichnet, dass**
R¹ Wasserstoff, Fluor, Chlor, Brom, C₁₋₆-Alkyl, C₂₋₆-Alkinyl, C₂₋₆-Alkenyl, C₃₋₇-Cycloalkyl, C₅₋₇-Cycloalkenyl, C₁₋₆-Alkyloxy, C₃₋₇-Cycloalkyloxy oder Cyan bedeutet, wobei in Cycloalkyl- und Cycloalkenylgruppen eine oder zwei Methyleneinheiten unabhängig voneinander durch O oder CO ersetzt und Alkyl-, Alkenyl- und Alkinyl-Reste teilweise oder vollständig fluoriert sein können.

5. D-Xylopyranosyl-substituierte Phenyle gemäß einem oder mehreren der Ansprüche 1 bis 4, **dadurch gekennzeichnet, dass**
R³ C₁₋₆-Alkyl, C₂₋₆-Alkinyl, C₁₋₄-Alkyloxy, C₃₋₇-Cycloalkyl, C₃₋₇-Cycloalkyloxy oder Hydroxy bedeutet, wobei in den Cycloalkylgruppen eine oder zwei Methyleneinheiten unabhängig voneinander durch Q oder CO ersetzt und Alkylreste teilweise oder vollständig fluoriert sein können.

6. D-Xylopyranosyl-substituierte Phenyle gemäß einem oder mehreren der Ansprüche 1 bis 5, **dadurch gekennzeichnet, dass**
X Wasserstoff, Cyan, C₁₋₆-Alkyl, C₂₋₆-Alkinyl, C₂₋₆-Alkenyl, C₁₋₄-Alkylcarbonyl, C₁₋₄-Alkoxycarbonyl, Aminocarbonyl, C₁₋₄-Alkylaminocarbonyl, Di-(C₁₋₄-alkyl)aminocarbonyl oder C₁₋₄-Alkylcarbonylamino-C₁₋₃-alkyl bedeutet, wobei Alkyl-Reste ein- oder mehrfach fluoriert oder einfach mit Chlor oder Cyan substituiert sein können und X in der Bedeutung Alkyl mit 2 oder mehr C-Atomen einen Hydroxy-Substituenten aufweisen kann.

7. D-Xylopyranosyl-substituierte Phenyle gemäß einem oder mehreren der Ansprüche 1 bis 5, **dadurch gekennzeichnet, dass**
X C₁₋₄-Alkyloxymethyl, C₃₋₇Cycloalkyloxymethyl oder Aryloxymethyl bedeutet,
wobei unter der Arylgruppe eine Phenyl- oder Naphthylgruppe, vorzugsweise eine Phenylgruppe zu verstehen ist, welche ein- oder zweifach mit gleichen oder verschiedenen Resten L substituiert sein kann und L gemäß Anspruch 1 definiert ist.

8. D-Xylopyranosyl-substituierte Phenyle gemäß einem oder mehreren der Ansprüche 1 bis 5, **dadurch gekennzeichnet, dass**
X Mercaptomethyl, C₁₋₄-Alkylsulfanylmethyl oder C₁₋₄-Alkylcarbonylsulfanylmethyl bedeutet.

9. D-Xylopyranosyl-substituierte Phenyle gemäß einem oder mehreren der Ansprüche 1 bis 5, **dadurch gekennzeichnet, dass**
X Chlormethyl, Brommethyl, Iodmethyl, C₁₋₆-Alkylsulfonyloxymethyl, Arylsulfonyloxymethyl oder Aryl-C₁₋₃-alkyl-sulfonyloxymethyl bedeutet,
wobei die vorstehend genannten Alkyl-Reste teilweise oder vollständig fluoriert oder ein- oder zweifach chloriert sein können und wobei die vorstehend genannten Aryl-Gruppen ein- oder zweifach mit gleichen oder verschiedenen Resten L substituiert sein können, wobei L vorzugsweise ausgewählt ist aus der Gruppe Fluor, Chlor, Brom, lod, C₁₋₃-Alkyl, Difluormethyl, Trifluormethyl und Cyan.

10. D-Xylopyranosyl-substituierte Phenyle gemäß einem oder mehreren der Ansprüche 1 bis 9, **dadurch gekennzeichnet, dass**
R² Wasserstoff, Fluor, Hydroxy, Methoxy, Ethoxy oder Methyl bedeutet.

11. D-Xylopyranosyl-substituierte Phenyle gemäß einem oder mehreren der Ansprüche 1 bis 10, **dadurch gekennzeichnet, dass**
R⁴ und R⁵ unabhängig voneinander Wasserstoff oder Fluor bedeuten.

12. D-Xylopyranosyl-substituierte Phenyle gemäß einem oder mehreren der Ansprüche 1 bis 11, **dadurch gekennzeichnet, dass**
Z Sauerstoff oder Methylen bedeutet.

13. D-Xylopyranosyl-substituierte Phenyle gemäß einem oder mehreren der Ansprüche 1 bis 12, **dadurch gekennzeichnet, dass**
R^{7a}, R^{7b}, R^{7c} unabhängig voneinander Wasserstoff, (C₁₋₆-Alkyl)oxycarbonyl-, (C₁₋₈-Alkyl)carbonyl oder Benzoyl, vorzugsweise Wasserstoff bedeuten.

14. Physiologisch verträgliche Salze der Verbindungen nach einem oder mehreren der Ansprüche 1 bis 13 mit anorganischen oder organischen Säuren.

15. Arzneimittel, enthaltend eine Verbindung nach einem oder mehreren der Ansprüche 1 bis 13 oder ein physiologisch verträgliches Salz gemäß Anspruch 14 neben gegebenenfalls einem oder mehreren inerten Trägerstoffen und/oder Verdünnungsmitteln.

16. Verwendung mindestens einer Verbindung nach einem oder mehreren der Ansprüche 1 bis 13 oder eines physiologisch verträglichen Salzes gemäß Anspruch 14 zur Herstellung eines Arzneimittels, das zur Behandlung oder Prophylaxe von Erkrankungen oder Zuständen geeignet ist, die durch Inhibierung des natriumabhängigen Glucose-Cotransporters SGLT beeinflussbar sind.

17. Verwendung mindestens einer Verbindung nach einem oder mehreren der Ansprüche 1 bis 13 oder eines physiologisch verträglichen Salzes gemäß Anspruch 14 zur Herstellung eines Arzneimittels, das zur Behandlung oder Prophylaxe von Stoffwechselerkrankungen geeignet ist.

18. Verwendung nach Anspruch 17 **dadurch gekennzeichnet, dass** die Stoffwechserkrankung ausgewählt ist aus der Gruppe bestehend aus Diabetes mellitus Typ 1 und/oder Typ 2, diabetische Komplikationen, metabolische Azidose oder Ketose, reaktiver Hypoglykämie, Hyperinsulinämie, Glukosestoffwechselstörung, Insulinresistenz, Metabolischem Syndrom, Dyslipidämien unterschiedlichster Genese, Atherosklerose und verwandte Erkrankungen, Adipositas, Bluthochdruck, chronisches Herzversagen, Ödeme, Hyperurikämie.

19. Verwendung mindestens einer Verbindung nach mindestens einem der Ansprüche 1 bis 13 oder eines physiologisch verträglichen Salzes gemäß Anspruch 14 zur Herstellung eines Arzneimittels zur Inhibition des natriumabhängigen Glucose-Cotransporters SGLT.

20. Verwendung mindestens einer Verbindung nach mindestens einem der Ansprüche 1 bis 13 oder eines physiologisch verträglichen Salzes gemäß Anspruch 14 zur Herstellung eines Arzneimittels zum Verhindern der Degeneration von pankreatischen beta-Zellen und/oder zum Verbessern und/oder Wiederherstellen der Funktionalität von pankreatischen beta-Zellen.

21. Verwendung mindestens einer Verbindung nach mindestens einem der Ansprüche 1 bis 13 oder eines physiologisch verträglichen Salzes gemäß Anspruch 14 zur Herstellung von Diuretika und/oder Antihypertensiva.

22. Verfahren zur Herstellung eines Arzneimittels gemäß Anspruch 15 **dadurch gekennzeichnet, dass** auf nicht-chemischem Wege eine Verbindung nach mindestens einem der Ansprüche 1 bis 13 oder ein physiologisch verträgliches Salz gemäß Anspruch 14 in einen oder mehrere inerte Trägerstoffe und/oder Verdünnungsmittel eingearbeitet wird.

23. Verfahren zur Herstellung der Verbindungen der allgemeinen Formel I gemäß den Ansprüchen 1 bis 13, **dadurch gekennzeichnet, dass** eine Verbindung der allgemeinen Formel II 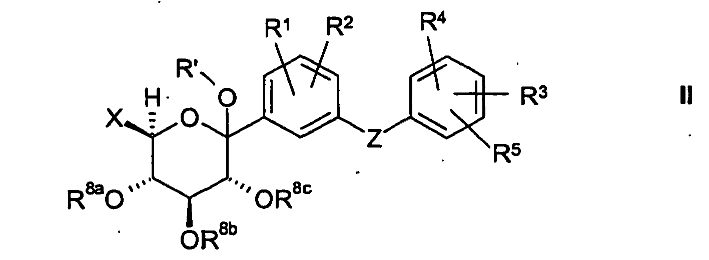 in der
R' H, C₁₋₄-Alkyl, (C₁₋₁₈-Alkyl)carbonyl, (C₁₋₁₈-Alkyl)oxycarbonyl, Arylcarbonyl oder Aryl-(C₁₋₃-alkyl)-carbonyl bedeutet, worin die Alkyl- oder Arylgruppen ein- oder mehrfach mit Halogen substituiert sein können;
R^{8a}, R^{8b}, R^{8c} unabhängig voneinander eine für die Reste R^{7a}, R^{7b}, R^{7c} angegebenen Bedeutungen aufweisen, eine Benzyl-Gruppe oder eine R^{a}R^{b}R^{c}Si-Gruppe oder eine Ketal- oder Acetalgruppe, insbesondere eine Alkyliden- oder Arylalkyliden- Ketal- oder Acetalgruppe bedeuten, wobei jeweils zwei benachbarte Reste R^{8a}, R^{8b}, R^{8c} eine cyclische Ketal- oder Acetalgruppe oder eine 1,2-Di(C₁₋₃-alkoxy)-1,2-di(C₁₋₃-alkyl)ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring, insbesondere einen 2,3-Dimethyl-2,3-di(C₁₋₃-alkoxy)-1,4-dioxan-Ring, bildet, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁₋₃-alkyl)amino-Gruppe substituiert sein können; und
R^{a}, R^{b}, R^{c} unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
wobei unter den bei der Definition der vorstehend genannten Reste erwähnten Aryl-gruppen Phenyl- oder Nathtylgruppen, vorzugsweise Phenylgruppen zu verstehen sind;
und X, Z, R¹ bis R⁵, R^{7a}, R^{7b}, R^{7c} die In den Ansprüchen 1 bis 13 angegebenen Bedeutungen besitzen,
mit einem Reduktionsmittel in Gegenwart einer Säure umgesetzt wird, wobei die eventuell vorhandenen Schutzgruppen gleichzeitig oder nachträglich abgespalten werden;
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I selektiv an einer Hydroxygruppe derivatisiert oder diese substituiert wird und/oder
gewünschtenfalls eine so erhaltene Verbindung der allgemeinen Formel I in ihre Stereoisomere aufgetrennt wird und/oder
gewünschtenfalls eine so erhaltene Verbindung der allgemeinen Formel I in ihre physiologisch verträglichen Salze überführt wird.

24. Verfahren zur Herstellung der Verbindungen der allgemeinen Formel I gemäß den Ansprüchen 1 bis 13, in der R^{7a}, R^{7b} und R^{7c} Wasserstoff bedeuten, **dadurch gekennzeichnet, dass** in einer Verbindung der allgemeinen Formel III 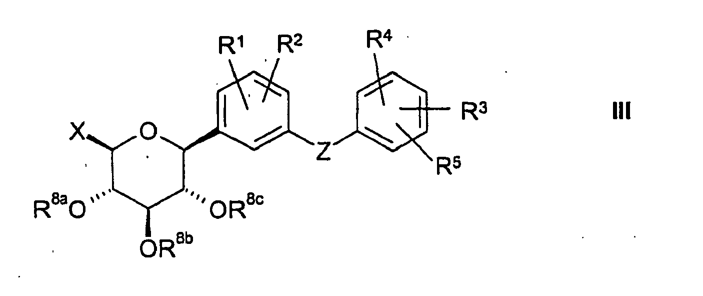 in der
R^{8a}, R^{8b}, R^{8c} unabhängig voneinander eine für die Reste R^{7a}, R^{7b}, R^{7c} angegebenen Bedeutungen aufweisen, eine Benzyl-Gruppe oder eine R^{a}R^{b}R^{c}Si-Gruppe oder eine Ketal- oder Acetalgruppe, insbesondere eine Alkyliden- oder Arylalkyliden- Ketal- oder Acetalgruppe bedeuten,
wobei jeweils zwei benachbarte Reste R^{8a}, R^{8b}, R^{8c} eine cyclische Ketal- oder Acetalgruppe oder eine 1,2-Di(C₁₋₃-alkoxy)-1,2-di(C₁₋₃-alkyl)ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring, insbesondere einen 2,3-Dimethyl-2,3-di(C₁₋₃-alkoxy)-1,4-dioxan-Ring, bildet, und wobei Alkyl-, Aryl- und/oder Benzylgruppen ein- öder mehrfach mit Halogen oder C₁₋₃-Alkoxy substituiert sein können und Benzyl-Gruppen auch mit einer Di-(C₁₋₃-alkyl)amino-Gruppe substituiert sein können; und wobei mindestens einer der Reste R^{8a}, R^{8b} und R^{8c} nicht Wasserstoff bedeutet; und
R^{a}, R^{b}, R^{c} unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
wobei unter den bei der Definition der vorstehend genannten Reste erwähnten Aryl-gruppen Phenyl- oder Naphthylgruppen, vorzugsweise Phenylgruppen zu verstehen sind;
und X, Z, R¹ bis R⁵, R^{7a}, R^{7b} , R^{7c} die in den Ansprüchen 1 bis 13 angegebenen Bedeutungen besitzen,
die nicht Wasserstoff bedeutenden Reste R^{8a}, R^{8b} bzw. R^{8c} entfernt werden, und
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I selektiv an einer Hydroxygruppe derivatisiert oder diese substituiert wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze, überführt wird.

## Claims

1. D-Xylopyranosyl-substituted phenyls of general formula I 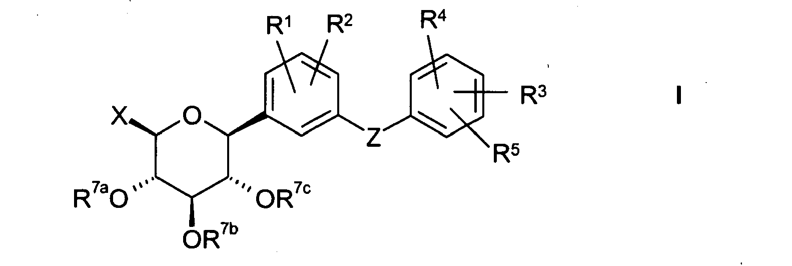 wherein
R¹ denotes hydrogen, fluorine, chlorine, bromine, C₁₋₆-alkyl, C₂₋₆-alkynyl, C₂₋₆-alkenyl, C₃₋₇-cycloalkyl, C₃₋₇-cycloalkyl-C₁₋₃-alkyl, C₅₋₇-cycloalkenyl, C₅₋₇-cycloalkenyl-C₁₋₃-alkyl, C₁₋₄-alkylcarbonyl, arylcarbonyl, heteroarylcarbonyl, aminocarbonyl, C₁₋₄-alkylaminocarbonyl, di-(C₁₋₃-alkyl)aminocarbonyl, pyrrolidin-1-ylcarbonyl, piperidin-1-ylcarbonyl, morpholin-4-ylcarbonyl, piperazin-1-ylcarbonyl, 4-(C₁₋₄-alkyl)piperazin-1-ylcarbonyl, C₁₋₄-alkoxycarbonyl, amino, C₁₋₄-alkylamino, di-(C₁₋₃-alkyl)amino, pyrrolidin-1-yl, piperidin-1-yl, morpholin-4-yl, piperazin-1-yl, 4-(C₁₋₄-alkyl)piperazin-1-yl, C₁₋₄-alkylcarbonylamino, C₁₋₆-alkyloxy, C₃₋₇-cycloalkyloxy, C₅₋₇-cycloalkenyloxy, aryloxy, C₁₋₄-alkylsulphanyl, C₁₋₄-alkylsulphinyl, C₁₋₄-alkylsulphonyl, C₃₋₇-cycloalkylsulphanyl, C₃₋₇-cycloalkylsulphinyl, C₃-₇-cycloalkylsulphonyl, C₅₋₇-cycloalkenylsulphanyl, C₅₋₇-cycloalkenylsulphinyl, C₅₋₇-cycloalkenylsulphonyl, arylsulphanyl, arylsulphinyl, arylsulphonyl, hydroxy, cyano or nitro,
wherein alkyl, alkenyl, alkynyl, cycloalkyl and cycloalkenyl groups may be partly or completely fluorinated or may be mono- or disubstituted by identical or different substituents selected from chlorine, hydroxy, C₁₋₃-alkoxy and C₁₋₃-alkyl, and
in cycloalkyl and cycloalkenyl groups one or two methylene groups may be replaced independently of one another by O, S, CO, SO or SO₂ and
in N-heterocycloalkyl groups a methylene group may be replaced by CO or SO₂, and
R² denotes hydrogen, fluorine, chlorine, bromine, hydroxy, C₁₋₄-alkyl, C₁₋₄-alkoxy, cyano or nitro, wherein alkyl groups may be mono- or polysubstituted by fluorine, or
in the event that R¹ and R² are bound to two C atoms of the phenyl ring which are adjacent to one another, R¹ and R² may be joined together in such a way that R¹ and R² together form a C₃₋₅-alkylene or C₃₋₅-alkenylene bridge, which may be partly or totally fluorinated or mono- or disubstituted by identical or different substituents selected from chlorine, hydroxy, C₁₋₃-alkoxy and C₁₋₃-alkyl and wherein one or two methylene groups may be replaced independently of one another by O, S, CO, SO, SO₂ or NR^{N},
R³ denotes hydrogen, fluorine, chlorine, bromine, C₁₋₆-alkyl, C₂₋₆-alkynyl, C₂₋₆-alkenyl, C₃₋₇-cycloalkyl, C₃₋₇-cycloalkyl-C₁₋₃-alkyl, C₅₋₇-cycloalkenyl, C₅₋₇-cycloalkenyl-C₁₋₃-alkyl, aryl, heteroaryl, C₁₋₄-alkylcarbonyl, arylcarbonyl, heteroarylcarbonyl, aminocarbonyl, C₁₋₄-alkylaminocarbonyl, di-(C₁₋₃-alkyl)aminocarbonyl, pyrrolidin-1-ylcarbonyl, piperidin-1-ylcarbonyl, morpholin-4-ylcarbonyl, piperazin-1-ylcarbonyl, 4-(C₁₋₄-alkyl)piperazin-1-ylcarbonyl, hydroxycarbonyl, C₁₋₄-alkoxycarbonyl, C₁₋₄-alkylamino, di-(C₁₋₃-alkyl)amino, pyrrolidin-1-yl, piperidin-1-yl, morpholin-4-yl, piperazin-1-yl, 4-(C₁₋₄-alkyl)piperazin-1-yl, C₁₋₄-alkylcarbonylamino, arylcarbonylamino, heteroarylcarbonylamino, C₁₋₄-alkylsulphonylamino, arylsulphonylamino, C₁₋₆-alkoxy, C₃₋₇-cycloalkyloxy, C₅₋₇-cycloalkenyloxy, aryloxy, heteroaryloxy, C₁₋₄-alkylsulphanyl, C₁₋₄-alkylsulphinyl, C₁₋₄-alkylsulphonyl, C₃₋₇-cycloalkylsulphanyl, C₃₋₇-cycloalkylsulphinyl, C₃₋₇-cycloalkylsulphonyl, C₅₋₇-cycloalkenylsulphanyl, C₅₋₇-cycloalkenylsulphinyl, C₅₋₇-cycloalkenylsulphonyl, arylsulphanyl, arylsulphinyl, arylsulphonyl, amino, hydroxy, cyano or nitro,
wherein alkyl, alkenyl, alkynyl, cycloalkyl and cycloalkenyl groups may be partly or totally fluorinated or mono- or disubstituted by identical or different substituents selected from chlorine, hydroxy, C₁₋₃-alkoxy and C₁₋₃-alkyl, and
in cycloalkyl and cycloalkenyl groups one or two methylene groups may be replaced independently of one another by O, S, CO, SO or SO₂, and
in N-heterocycloalkyl groups a methylene group may be replaced by CO or SO₂, and
R⁴ denotes hydrogen, fluorine, chlorine, bromine, iodine, cyano, nitro, C₁₋₃-alkyl, C₁₋₃-alkoxy or methyl or methoxy substituted by 1 to 3 fluorine atoms, or
in the event that R³ and R⁴ are bound to two C atoms of the phenyl ring which are adjacent to one another, R³ and R⁴ may be joined together in such a way that R³ and R⁴ together form a C₃₋₅-alkylene or C₃₋₅-alkenylene bridge, which may be partly or totally fluorinated or mono- or disubstituted by identical or different substituents selected from chlorine, hydroxy, C₁₋₃-alkoxy and C₁₋₃-alkyl and wherein one or two methylene groups may be replaced independently of one another by O, S, CO, SO, SO₂ or NR^{N},
R⁵ denotes hydrogen, fluorine, chlorine, bromine, iodine, cyano, nitro, C₁₋₃-alkyl, C₁₋₃-alkoxy, or methyl or methoxy substituted by 1 to 3 fluorine atoms, and
R^{N} independently of one another denote H or C₁₋₄-alkyl,
L are selected independently of one another from among fluorine, chlorine, bromine, iodine, C₁₋₃-alkyl, difluoromethyl, trifluoromethyl, C₁₋₃-alkoxy, difluoromethoxy, trifluoromethoxy and cyano,
R^{7a}, R^{7b}, R^{7c} independently of one another have a meaning selected from among hydrogen, (C₁₋₁₈-alkyl)carbonyl, (C₁₋₁₈-alkyl)oxycarbonyl, arylcarbonyl and aryl-(C₁₋₃-alkyl)-carbonyl,
X denotes hydrogen, C₁₋₆-alkyl, C₂₋₆-alkynyl, C₂₋₆-alkenyl, C₃₋₇-cycloalkyl, C₃₋₇-cycloalkyl-C₁₋₃-alkyl, C₅₋₇-cycloalkenyl, C₅₋₇-cycloalkenyl-C₁₋₃-alkyl, aryl, aryl-C₁₋₃-alkyl, heteroaryl, heteroaryl-C₁₋₃-alkyl, C₁₋₄-alkylcarbonyl, arylcarbonyl, aminocarbonyl, C₁₋₄-alkylaminocarbonyl, di-(C₁₋₃-alkyl)aminocarbonyl, (aryl-C₁₋₃-alkyl)aminocarbonyl, pyrrolidin-1-ylcarbonyl, piperidin-1-ylcarbonyl, morpholin-4-ylcarbonyl, hydroxycarbonyl, C₁₋₄-alkoxycarbonyl, C₁₋₄-alkylcarbonylamino-C₁₋₃-alkyl, N-(C₁₋₄-alkylcarbonyl)-N-(C₁₋₃-alkyl)-amino-C₁₋₃-alkyl, arylcarbonylamino-C₁₋₃-alkyl, C₁₋₄-alkylsulphonylamino-C₁₋₃-alkyl, arylsulphonylamino-C₁₋₃-alkyl, C₁₋₆-alkoxy-C₁₋₃-alkyl, C₃₋₇-cycloalkyloxy-C₁₋₃-alkyl, C₅₋₇-cycloalkenyloxy-C₁₋₃-alkyl, aryloxy-C₁₋₃-alkyl, heteroaryloxy-C₁₋₃-alkyl, C₁₋₄-alkylsulphanyl-C₁₋₃-alkyl, C₁₋₄-alkylsulphinyl-C₁₋₃-alkyl, C₁₋₄-alkylsulphinyl, C₁₋₄-alkylsulphonyl, C₁₋₄-alkylsulphonyl-C₁₋₃-alkyl, C₁₋₄-arylsulphanyl-C₁₋₃-alkyl, arylsulphonyl-C₁₋₃-alkyl, aryl-C₁₋₃-alkylsulphonyl-C₁₋₃-alkyl, C₁₋₄-alkylsulphonyloxy-C₁₋₃-alkyl, arylsulphonyloxy-C₁₋₃-alkyl, aryl-C₁₋₃-alkyl-sulphonyloxy-C₁₋₃-alkyl, C₃₋₇-cycloalkylsulphanyl-C₁₋₃-alkyl, C₃₋₇-cycloalkylsulphinyl, C₃₋₇-cycloalkylsulphonyl, C₅₋₇-cycloalkenylsulphanyl-C₁₋₃-alkyl, C₅₋₇-cycloalkenylsulphinyl, C₅₋₇-cycloalkenylsulphonyl, C₁₋₄-alkylcarbonylsulphanyl-C₁₋₃-alkyl or cyano,
wherein alkyl, alkenyl, alkynyl, cycloalkyl and cycloalkenyl groups may be partly or totally fluorinated or mono- or disubstituted by identical or different substituents selected from chlorine, cyano, hydroxy, mercapto, C₁₋₃-alkoxy and C₁₋₃-alkyl, and
in cycloalkyl and cycloalkenyl groups one or two methylene groups may be replaced independently of one another by O, S, CO, SO or SO₂, and
in N-heterocycloalkyl groups a methylene group may be replaced by CO or SO₂ and
X representing hydroxymethyl is excluded,
Z denotes oxygen, methylene, dimethylmethylene, difluoromethylene or carbonyl;
while the term aryl groups used in the definition of the above groups denotes phenyl or naphthyl groups, which may be mono- or disubstituted independently of one another by identical or different groups L; and
the term heteroaryl groups used in the definition of the above-mentioned groups denotes a pyrrolyl, furanyl, thienyl, pyridyl, indolyl, benzofuranyl, benzothiophenyl, quinolinyl or isoquinolinyl group,
or a pyrrolyl, furanyl, thienyl, pyridyl or imidazolyl group, wherein one or two methyne groups are replaced by nitrogen atoms,
or an indolyl, benzofuranyl, benzothiophenyl, quinolinyl or isoquinolinyl group, wherein one to three methyne groups are replaced by nitrogen atoms,
while the above-mentioned heteroaryl groups may be mono- or disubstituted independently of one another by identical or different groups L;
while by the N-heterocycloalkyl group mentioned in the definition of the above-mentioned groups is meant a saturated carbocyclic ring which comprises an imino group in the ring, which may comprise another optionally substituted imino group or an O or S atom in the ring, and
unless otherwise stated the above-mentioned alkyl groups may be straight-chain or branched,
the tautomers, the stereoisomers, the mixtures thereof and the salts thereof, particularly the physiologically acceptable salts thereof.

2. D-Xylopyranosyl-substituted phenyls according to claim 1, **characterised by** the formula 1.2 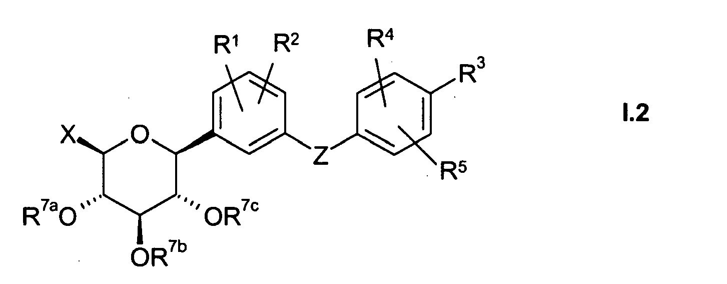 wherein R¹ to R⁵, X, Z, R^{7a}, R^{7b}, R^{7c} have the meanings according to claim 1.

3. D-Xylopyranosyl-substituted phenyls according to claim 1,
**characterised by** the formula I.2c 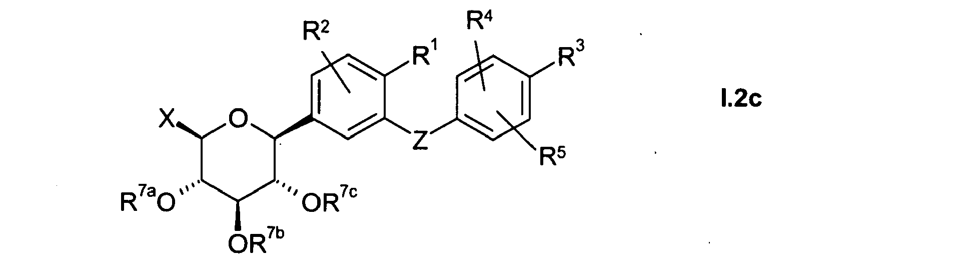 wherein R¹ to R⁵, X, Z, R^{7a}, R^{7b}, R^{7c} have the meanings according to claim 1.

4. D-Xylopyranosyl-substituted phenyls according to one or more of claims 1 to 3, **characterised in that**
R¹ denotes hydrogen, fluorine, chlorine, bromine, C₁₋₆-alkyl, C₂₋₆-alkynyl, C₂₋₆-alkenyl, C₃₋₇-cycloalkyl, C₅₋₇-cycloalkenyl, C₁₋₆-alkyloxy, C₃₋₇-cycloalkyloxy or cyano, while in cycloalkyl and cycloalkenyl groups one or two methylene units may be replaced independently of one another by O or CO and alkyl, alkenyl and alkynyl groups may be partly or totally fluorinated.

5. D-Xylopyranosyl-substituted phenyls according to one or more of claims 1 to 4, **characterised in that**
R³ denotes C₁₋₆-alkyl, C₂₋₆-alkynyl, C₁₋₄-alkyloxy, C₃₋₇-cycloalkyl, C₃₋₇-cycloalkyloxy or hydroxy, while in the cycloalkyl groups one or two methylene units may be replaced independently of one another by O or CO and alkyl groups may be partly or totally fluorinated.

6. D-Xylopyranosyl-substituted phenyls according to one or more of claims 1 to 5, **characterised in that**
X denotes hydrogen, cyano, C₁₋₆-alkyl, C₂₋₆-alkynyl, C₂₋₆-alkenyl, C₁₋₄-alkylcarbonyl, C₁₋₄-alkoxycarbonyl, aminocarbonyl, C₁₋₄-alkylaminocarbonyl, di-(C₁₋₄-alkyl)aminocarbonyl or C₁₋₄-alkylcarbonylamino-C₁₋₃-alkyl, wherein alkyl groups may be mono- or polyfluorinated or monosubstituted by chlorine or cyano and X representing alkyl with 2 or more C atoms may comprise a hydroxy substituent.

7. D-Xylopyranosyl-substituted phenyls according to one or more of claims 1 to 5, **characterised in that**
X denotes C₁₋₄-alkyloxymethyl, C₃₋₇-cycloalkyloxymethyl or aryloxymethyl,
while by the aryl group is meant a phenyl or naphthyl group, preferably a phenyl group, which may be mono- or disubstituted by identical or different groups L and L is defined according to claim 1.

8. D-Xylopyranosyl-substituted phenyls according to one or more of claims 1 to 5, **characterised in that**
X denotes mercaptomethyl, C₁₋₄-alkylsulphanylmethyl or C₁₋₄-alkylcarbonylsulphanylmethyl.

9. D-Xylopyranosyl-substituted phenyls according to one or more of claims 1 to 5, **characterised in that**
X denotes chloromethyl, bromomethyl, iodomethyl, C₁₋₆-alkylsulphonyloxymethyl, arylsulphonyloxymethyl or aryl-C₁₋₃-alkylsulphonyloxymethyl,
while the above-mentioned alkyl groups may be partly or totally fluorinated or mono- or dichlorinated and the above-mentioned aryl groups may be mono- or disubstituted by identical or different groups L, while L is preferably selected from among fluorine, chlorine, bromine, iodine, C₁₋₃-alkyl, difluoromethyl, trifluoromethyl and cyano.

10. D-Xylopyranosyl-substituted phenyls according to one or more of claims 1 to 9, **characterised in that**
R² denotes hydrogen, fluorine, hydroxy, methoxy, ethoxy or methyl.

11. D-Xylopyranosyl-substituted phenyls according to one or more of claims 1 to 10, **characterised in that**
R⁴ and R⁵ independently of one another represent hydrogen or fluorine.

12. D-Xylopyranosyl-substituted phenyls according to one or more of claims 1 to 11, **characterised in that**
Z denotes oxygen or methylene.

13. D-Xylopyranosyl-substituted phenyls according to one or more of claims 1 to 12, **characterised in that**
R^{7a}, R^{7b}, R^{7c} independently of one another represent hydrogen, (C₁₋₆-alkyl)oxycarbonyl, (C₁₋₈-alkyl)carbonyl or benzoyl, preferably hydrogen.

14. Physiologically acceptable salts of the compounds according to one or more of claims 1 to 13 with inorganic or organic acids.

15. Pharmaceutical compositions, containing a compound according to one or more of claims 1 to 13 or a physiologically acceptable salt according to claim 14, optionally together with one or more inert carriers and/or diluents.

16. Use of at least one compound according to one or more of claims 1 to 13 or a physiologically acceptable salt according to claim 14 for preparing a pharmaceutical composition which is suitable for the treatment or prevention of diseases or conditions which can be influenced by inhibiting the sodium-dependent glucose cotransporter SGLT.

17. Use of at least one compound according to one or more of claims 1 to 13 or a physiologically acceptable salt according to claim 14 for preparing a pharmaceutical composition which is suitable for the treatment or prevention of metabolic disorders.

18. Use according to claim 17, **characterised in that** the metabolic disorder is selected from the group consisting of type 1 and/or type 2 diabetes mellitus, complications of diabetes, metabolic acidosis or ketosis, reactive hypoglycaemia, hyperinsulinaemia, glucose metabolic disorder, insulin resistance, metabolic syndrome, dyslipidaemias of different origins, atherosclerosis and related diseases, obesity, high blood pressure, chronic heart failure, oedema and hyperuricaemia.

19. Use of at least one compound according to at least one of claims 1 to 13 or a physiologically acceptable salt according to claim 14 for preparing a pharmaceutical composition for inhibiting the sodium-dependent glucose cotransporter SGLT.

20. Use of at least one compound according to at least one of claims 1 to 13 or a physiologically acceptable salt according to claim 14 for preparing a pharmaceutical composition for preventing the degeneration of pancreatic beta cells and/or for improving and/or restoring the functionality of pancreatic beta cells.

21. Use of at least one compound according to at least one of claims 1 to 13 or a physiologically acceptable salt according to claim 14 for preparing diuretics and/or antihypertensives.

22. Process for preparing a pharmaceutical composition according to claim 15, **characterised in that** a compound according to at least one of claims 1 to 13 or a physiologically acceptable salt according to claim 14 is incorporated in one or more inert carriers and/or diluents by a non-chemical method.

23. Process for preparing the compounds of general formula I according to claims 1 to 13, **characterised in that** a compound of general formula II 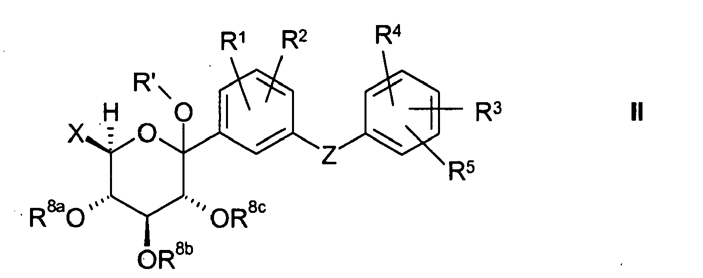 wherein
R' denotes H, C₁₋₄-alkyl, (C₁₋₁₈-alkyl)carbonyl, (C₁₋₁₈-alkyl)oxycarbonyl, arylcarbonyl or aryl-(C₁₋₃-alkyl)carbonyl, wherein the alkyl or aryl groups may be mono- or polysubstituted by halogen;
R^{8a}, R^{8b}, R⁸c independently of one another have one of the meanings given for the groups R^{7a}, R^{7b}, R^{7c}, denote a benzyl group or an R^{a}R^{b}R^{c}Si group or a ketal or acetal group, particularly an alkylidene or arylalkylidene ketal or acetal group, wherein in each case two adjacent groups R^{8a}, R^{8b}, R^{8c}, may form a cyclic ketal or acetal group or a 1,2-di(C₁₋₃-alkoxy)-1,2-di(C₁₋₃-alkyl)-ethylene bridge, wherein the above-mentioned ethylene bridge together with two oxygen atoms and the associated two carbon atoms of the pyranose ring form a substituted dioxane ring, particularly a 2,3-dimethyl-2,3-di(C₁₋₃-alkoxy)-1,4-dioxane ring, and
wherein alkyl, aryl and/or benzyl groups may be mono- or polysubstituted by halogen or C₁₋₃-alkoxy and benzyl groups may also be substituted by a di-(C₁₋₃-alkyl)amino group; and
R^{a}, R^{b}, R^{c} independently of one another represent C₁₋₄-alkyl, aryl or aryl-C₁₋₃-alkyl, wherein the aryl or alkyl groups may be mono- or polysubstituted by halogen;
while the term aryl groups used in the definition of the above groups denotes phenyl or naphthyl groups, preferably phenyl groups;
and X, Z, R¹ to R⁵, R^{7a}, R^{7b}, R^{7c} have the meanings given in claims 1 to 13,
is reacted with a reducing agent in the presence of an acid, and any protective groups present are cleaved at the same time or subsequently;
if necessary any protective group used in the reactions described above is cleaved and/or
if desired a compound of general formula I thus obtained is selectively derivatised at a hydroxy group or this group is substituted and/or
if desired a compound of general formula I thus obtained is resolved into its stereoisomers and/or
if desired a compound of general formula I thus obtained is converted into the physiologically acceptable salts thereof.

24. Process for preparing the compounds of general formula I according to claims 1 to 13, wherein R^{7a}, R^{7b} and R^{7c} represent hydrogen, **characterised in that** in a compound of general formula III 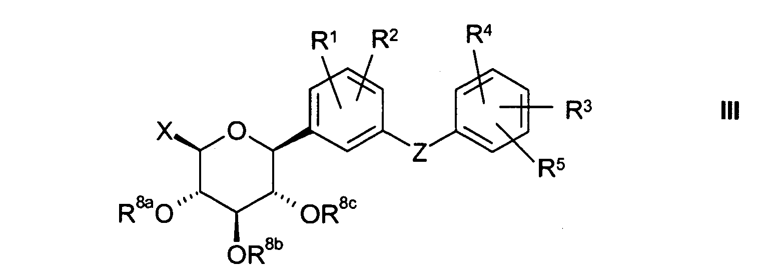 wherein
R^{8a}, R^{8b}, R^{8c} independently of one another have one of the meanings given for the groups R^{7a}, R^{7b}, R^{7c}, denote a benzyl group or an R^{a}R^{b}R^{c}Si group or a ketal or acetal group, particularly an alkylidene or arylalkylidene ketal or acetal group, while in each case two adjacent groups R^{8a}, R^{8b}, R^{8c} may form a cyclic ketal or acetal group or a 1,2-di (C₁₋₃-alkoxy)-1,2-di (C₁₋₃-alkyl) -ethylene bridge, wherein the above-mentioned ethylene bridge together with two oxygen atoms and the associated two carbon atoms of the pyranose ring form a substituted dioxane ring, particularly a 2,3-dimethyl-2,3-di(C₁₋₃-alkoxy)-1,4-dioxane ring, and
wherein alkyl, aryl and/or benzyl groups may be mono- or polysubstituted by halogen or C₁₋₃-alkoxy and benzyl groups may also be substituted by a di-(C₁₋₃-alkyl)amino group; and wherein at least one of the groups R^{8a}, R^{8b}, R^{8c} does not represent hydrogen; and
R^{a}, R^{b}, R^{c} independently of one another represent C₁₋₄-alkyl, aryl or aryl-C₁₋₃-alkyl, wherein the aryl or alkyl groups may be mono- or polysubstituted by halogen;
while the term aryl groups used in the definition of the above groups denotes phenyl or naphthyl groups, preferably phenyl groups;
and X, Z, R¹ to R⁵, R^{7a}, R^{7b}, R^{7c} have the meanings given in claims 1 to 13,
the groups R^{8a}, R^{8b} or R^{8c} which do not represent hydrogen are removed, and
if necessary any protective group used in the reactions described above is cleaved and/or
if desired a compound of general formula I thus obtained is selectively derivatised at a hydroxy group or this group is substituted and/or
if desired a compound of general formula I thus obtained is resolved into its stereoisomers and/or
if desired a compound of general formula I thus obtained is converted into the salts thereof, particularly for pharmaceutical use into the physiologically acceptable salts thereof.

## Revendications

1. Phényles D-xylopyranosyl-substitués de formule générale I 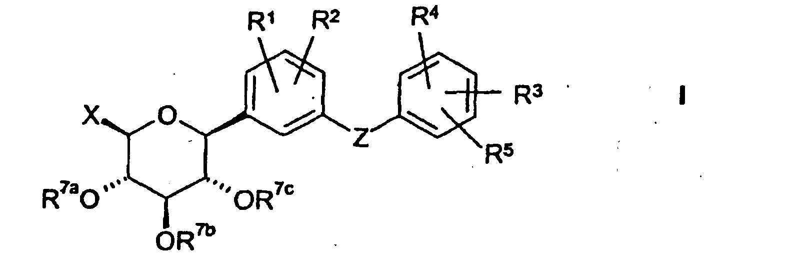 où
R¹ représente l'hydrogène, le fluor, le chlore, le brome, C₁₋₆-alkyle, C₂₋₆-alcynyle, C₂₋₆-alcényle, C₃₋₇-cycloalkyle, C₃₋₇-cycloalkyl-C₁₋₃-alkyle, C₅₋₇-cycloalcényle, C₅₋₇-cycloalcényl-C₁₋₃-alkyle, C₁₋₄-alkylcarbonyle, arylcarbonyle, hétéroarylcarbonyle, aminocarbonyle, C₁₋₄-alkylaminocarbonyle, di-(C₁₋₃-alkyl)aminocarbonyle, pyrrolidin-1-ylcarbonyle, pipéridin-1-ylcarbonyle, morpholin-4-ylcarbonyle, pipérazin-1-ylcarbonyle, 4-(C₁₋₄-alkyl)pipérazin-1-ylcarbonyle, C₁₋₄-alcoxycarbonyle, amino, C₁₋₄-alkylamino, di-(C₁₋₃-alkyl)amino, pyrrolidin-1-yle, pipéridin-1-yle, morpholin-4-yle, pipérazin-1-yle, 4-(C₁₋₄-alkyl)pipérazin-1-yle, C₁₋₄-alkylcarbonylamino, C₁₋₆-alkyloxy, C₃₋₇-cycloalkyloxy, C₅₋₇-cycloalcényloxy, aryloxy, C₁₋₄-alkylsulfanyle, C₁₋₄-alkylsulfinyle, C₁₋₄-alkylsulfonyle, C₃₋₇-cycloalkylsulfanyle, C₃₋₇-cycloalkylsulfinyle, C₃₋₇-cycloalkylsulfonyle, C₅₋₇-cycloalcénylsulfanyle, C₅₋₇-cycloalcénylsulfinyle, C₅₋₇-cycloalcénylsulfonyle, arylsulfanyle, arylsulfinyle, arylsulfonyle, hydroxy, cyano ou nitro,
où les groupements alkyle, alcényle, alcynyle, cycloalkyle et cycloalcényle peuvent être partiellement ou totalement fluorés ou substitués une ou deux fois avec des substituants identiques ou différents choisis parmi le chlore, hydroxy, C₁₋₃-alcoxy et C₁₋₃-alkyle, et
où dans les groupements cycloalkyle et cycloalcényle, un ou deux groupes méthylène peuvent être remplacés indépendamment l'un de l'autre par O, S, CO, SO ou SO₂, et
où dans les groupements N-hétérocycloalkyle un groupe méthylène peut être remplacé par CO ou SO₂, et
R² représente l'hydrogène, le fluor, le chlore, le brome, hydroxy, C₁₋₄-alkyle, C₁₋₄-alcoxy, cyano ou nitro, où les groupements alkyle peuvent être substitués une ou plusieurs fois par le fluor, ou
au cas où R¹ et R² sont liés à deux atomes C du cycle phényle voisins, R¹ et R² peuvent être liés l'un à l'autre de telle sorte que R¹ et R² forment ensemble un pont C₃₋₅-alkylène ou C₃₋₅-alcénylène qui peut être fluoré partiellement ou totalement ou substitué une ou deux fois avec des substituants identiques ou différents choisis parmi le chlore, hydroxy, C₁₋₃-alcoxy et C₁₋₃-alkyle et où un ou deux groupes méthylène peuvent être remplacés indépendamment l'un de l'autre par O, S, CO, SO, SO₂ ou NR^{N},
R³ représente l'hydrogène, le fluor, le chlore, le brome, C₁₋₆-alkyle, C₂₋₆-alcynyle, C₂₋₆-alcényle, C₃₋₇-cycloalkyle, C₃₋₇-cycloalkyl-C₁₋₃-alkyle, C₅₋₇-cycloalcényle, C₅₋₇-cycloalcényl-C₁₋₃-alkyle, aryle, hétéroaryle, C₁₋₄-alkylcarbonyle, arylcarbonyle, hétéroarylcarbonyle, aminocarbonyle, C₁₋₄-alkylaminocarbonyle, di-(C₁₋₃-alkyl)aminocarbonyle, pyrrolidin-1-ylcarbonyle, pipéridin-1-ylcarbonyle, morpholin-4-ylcarbonyle, pipérazin-1-ylcarbonyle, 4-(C₁₋₄-alkyl)pipérazin-1-ylcarbonyle, hydroxycarbonyle, C₁₋₄-alcoxycarbonyle, C₁₋₄-alkylamino, di-(C₁₋₃-alkyl)amino, pyrrolidin-1-yle, pipéridin-1-yle, morpholin-4-yle, pipérazin-1-yle, 4-(C₁₋₄-alkyl)pipérazin-1-yle, C₁₋₄-alkylcarbonylamino, arylcarbonylamino, hétéroarylcarbonylamino, C₁₋₄-alkylsulfonylamino, arylsulfonylamino, C₁₋₆-alcoxy, C₃₋₇-cycloalkyloxy, C₅₋₇-cycloalcényloxy, aryloxy, hétéroaryloxy, C₁₋₄-alkylsulfanyle, C₁₋₄-alkylsulfinyle, C₁₋₄-alkylsulfonyle, C₃₋₇-cycloalkylsulfanyle, C₃₋₇-cycloalkylsulfinyle, C₃₋₇-cycloalkylsulfonyle, C₅₋₇-cycloalcénylsulfanyle, C₅-₇-cycloalcénylsulfinyle, C₅₋₇-cycloalcénylsulfonyle, arylsulfanyle, arylsulfinyle, arylsulfonyle, amino, hydroxy, cyano ou nitro,
où les groupements alkyle, alcényle, alcynyle, cycloalkyle et cycloalcényle peuvent être partiellement ou totalement fluorés ou substitués une ou deux fois avec des substituants identiques ou différents choisis parmi le chlore, hydroxy, C₁-₃-alcoxy et C₁₋₃-alkyle, et
où dans les groupements cycloalkyle et cycloalcényle, un ou deux groupes méthylène peuvent être remplacés indépendamment l'un de l'autre par O, S, CO, SO ou SO₂, et
où dans les groupements N-hétérocycloalkyle un groupe méthylène peut être remplacé par CO ou SO₂, et
R⁴ représente l'hydrogène, le fluor, le chlore, le brome, l'iode, cyano, nitro, C₁₋₃-alkyle, C₁₋₃-alcoxy ou méthyle ou méthoxy substitué par 1 à 3 atomes de fluor, ou au cas où R³ et R⁴ sont liés à deux atomes C du cycle phényle voisins, R³ et R⁴ peuvent être liés l'un à l'autre de telle sorte que R³ et R⁴ forment ensemble un pont C₃₋₅-alkylène ou C₃₋₅-alcénylène qui peut être fluoré partiellement ou totalement ou substitué une ou deux fois avec des substituants identiques ou différents choisis parmi le chlore, hydroxy, C₁₋₃-alcoxy et C₁₋₃-alkyle et où un ou deux groupes méthylène peuvent être remplacés indépendamment l'un de l'autre par O, S, CO, SO, SO₂ ou NR^{N},
R⁵ représente l'hydrogène, le fluor, le chlore, le brome, l'iode, cyano, nitro, C₁₋₃-alkyle, C₁₋₃-alcoxy ou méthyle ou méthoxy substitué par 1 à 3 atomes de fluor, et R^{N} représentent indépendamment l'un de l'autre H ou C₁₋₄-alkyle,
L indépendamment l'un de l'autre sont choisis dans le groupe consistant en le fluor, le chlore, le brome, l'iode, C₁₋₃-alkyle, difluorométhyle, trifluorométhyle, C₁₋₃-alcoxy, difluorométhoxy, trifluorométhoxy et cyano,
R^{7a}, R^{7b}, R^{7c} possèdent indépendamment les uns des autres une signification choisie dans le groupe hydrogène, (C₁₋₁₈-alkyl)carbonyle, (C₁₋₁₈-alkyl)oxycarbonyle, arylcarbonyle et aryl-(C₁₋₃-alkyl)carbonyle,
X représente l'hydrogène, C₁₋₆-alkyle, C₂₋₆-alcynyle, C₂₋₆-alcényle, C₃₋₇-cycloalkyle, C₃₋₇-cycloalkyl-C₁₋₃-alkyle, C₅₋₇-cycloalcényle, C₅₋₇-cycloalcényl-C₁-₃-alkyle, aryle, aryl-C₁₋₃-alkyle, hétéroaryle, hétéroaryl-C₁₋₃-alkyle, C₁₋₄-alkylcarbonyle, arylcarbonyle, aminocarbonyle, C₁₋₄-alkylaminocarbonyle, di-(C₁-₃-alkyl)aminocarbonyle, (aryl-C₁₋₃-alkyl)aminocarbonyle, pyrrolidin-1-ylcarbonyle, pipéridin-1-ylcarbonyle, morpholin-4-ylcarbonyle, hydroxycarbonyle, C₁₋₄-alcoxycarbonyle, C₁₋₄-alkylcarbonylamino-C₁₋₃-alkyle, N-(C₁₋₄-alkylcarbonyl)-N-(C₁₋₃-alkyl)-amino-C₁₋₃-alkyle, arylcarbonylamino-C₁₋₃-alkyle, C₁₋₄-alkylsulfonylamino-C₁₋₃-alkyle, arylsulfonylamino-C₁₋₃-alkyle, C₁₋₆-alcoxy-C₁₋₃-alkyle, C₃₋₇-cycloalkyloxy-C₁₋₃-alkyle, C₅₋₇-cycloalcényloxy-C₁₋₃-alkyle, aryloxy-C₁₋₃-alkyle, hétéroaryloxy-C₁₋₃-alkyle, C₁₋₄-alkylsulfanyl-C₁₋₃-alkyle, C₁₋₄-alkylsulfinyl-C₁₋₃-alkyle, C₁₋₄-alkylsulfinyle, C₁₋₄-alkylsulfonyle, C₁₋₄-alkylsulfonyl-C₁₋₃-alkyle, C₁₋₄-arylsulfanyl-C₁₋₃-alkyle, arylsulfonyl-C₁₋₃-alkyle, aryl-C₁₋₃-alkylsulfonyl-C₁₋₃-alkyle, C₁₋₄-alkylsulfonyloxy-C₁₋₃-alkyle, arylsulfonyloxy-C₁₋₃-alkyle, aryl-C₁₋₃-alkylsulfonyloxy-C₁₋₃-alkyle, C₃₋₇-cycloalkylsulfanyl-C₁₋₃-alkyle, C₃₋₇-cycloalkylsulfinyle, C₃₋₇-cycloalkylsulfonyle, C₅₋₇-cycloalcénylsulfanyl-C₁₋₃-alkyle, C₅₋₇-cycloalcénylsulfinyle, C₅₋₇-cycloalcénylsulfonyle, C₁₋₄-alkylcarbonylsulfanyl-C₁₋₃-alkyle ou cyano,
où les groupements alkyle, alcényle, alcynyle, cycloalkyle et cycloalcényle peuvent être partiellement ou totalement fluorés ou substitués une ou deux fois avec des substituants identiques ou différents choisis parmi le chlore, cyano, hydroxy, mercapto, C₁₋₃-alcoxy et C₁₋₃-alkyle, et
où dans les groupements cycloalkyle et cycloalcényle, un ou deux groupes méthylène peuvent être remplacés indépendamment l'un de l'autre par O, S, CO, SO ou SO₂, et
où dans les groupements N-hétérocycloalkyle un groupe méthylène peut être remplacé par CO ou SO₂, et
où X dans la signification d'hydroxyméthyle est exclus,
Z représente l'oxygène, méthylène, diméthylméthylène, difluorométhylène ou carbonyle ;
où, concernant les groupes aryle cités dans la définition des groupements cités précédemment, il convient de comprendre des groupes phényle ou naphtyle qui peuvent être substitués indépendamment les uns des autres une ou deux fois avec des groupements L identiques ou différents ; et
concernant les groupes hétéroaryle cités dans la définition des groupements cités précédemment, il convient de comprendre un groupe pyrrolyle, furanyle, thiényle, pyridyle, indolyle, benzofuranyle, benzothiophényle, quinolinyle ou isoquinolinyle,
ou il convient de comprendre un groupe pyrrolyle, furanyle, thiényle, pyridyle ou imidazolyle dans lequel un ou deux groupes méthyne sont remplacés par des atomes d'azote,
ou il convient de comprendre un groupe indolyle, benzofuranyle, benzothiophényle, quinolinyle ou isoquinolinyle dans lequel un à trois groupes méthyne sont remplacés par des atomes d'azote,
où les groupes hétéroaryle mentionnés précédemment peuvent être substitués indépendamment les uns des autres une ou deux fois par des groupements L identiques ou différents ;
où concernant le groupement N-hétérocycloalkyle cité dans la définition des groupements cités précédemment, il convient de comprendre un cycle carbocyclique saturé, qui comporte un groupe imino dans le cycle, qui peut comporter un autre groupe imino éventuellement substitué ou un atome O ou S dans le cycle, et
où, dans la mesure où rien d'autre n'a été cité, les groupes alkyle cités précédemment peuvent être linéaires ou ramifiés,
leurs tautomères, leurs stéréoisomères, leurs mélanges et leurs sels, en particulier leurs sels physiologiquement acceptables.

2. Phényles D-xylopyranosyl-substitués selon la revendication 1 **caractérisés par** la formule 1.2 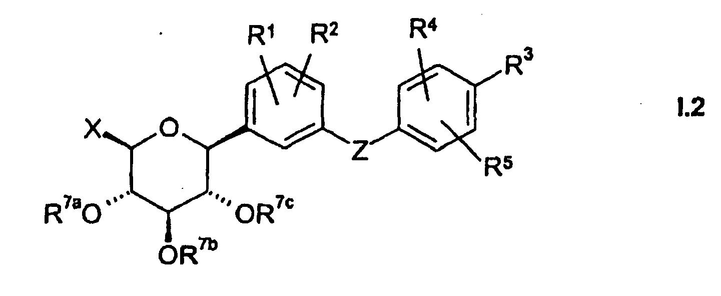 où R¹ à R⁵, X, Z, R^{7a}, R^{7b}, R^{7c} présentent les significations selon la revendication 1.

3. Phényles D-xylopyranosyl-substitués selon la revendication 1 **caractérisés par** la formule I.2c 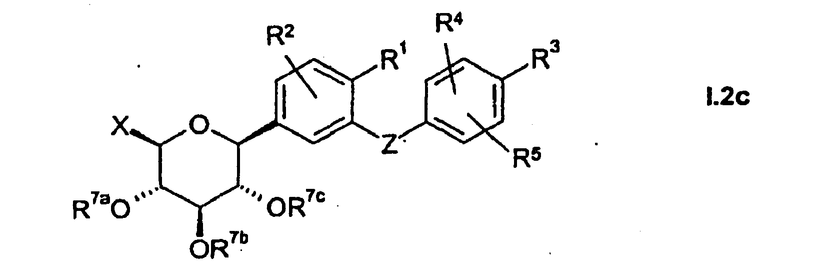 où
R¹ à R⁵, X, Z, R^{7a}, R^{7b}, R^{7c} présentent les significations selon la revendication 1.

4. Phényles D-xylopyranosyl-substitués selon une ou plusieurs des revendications 1 à 3 **caractérisés en ce que**
R¹ représente l'hydrogène, le fluor, le chlore, le brome, C₁₋₆-alkyle, C₂₋₆-alcynyle, C₂₋₆-alcényle, C₃₋₇-cycloalkyle, C₅₋₇-cycloalcényle, C₁₋₆-alkyloxy, C₃₋₇-cycloalkyloxy ou cyano, où dans les groupes cycloalkyle et cycloalcényle, une ou deux unités méthylène peuvent être remplacées indépendamment l'une de l'autre par O ou CO et les groupements alkyle, alcényle et alcynyle peuvent être partiellement ou totalement fluorés.

5. Phényles D-xylopyranosyl-substitués selon une ou plusieurs des revendications 1 à 4 **caractérisés en ce que**
R³ représente C₁₋₆-alkyle, C₂₋₆-alcynyle, C₁₋₄-alkyloxy, C₃₋₇-cycloalkyle, C₃₋₇-cycloalkyloxy ou hydroxy, où dans les groupes cycloalkyle, une ou deux unités méthylène peuvent être remplacées indépendamment l'une de l'autre par O ou CO et les groupements alkyle peuvent être partiellement ou totalement fluorés.

6. Phényles D-xylopyranosyl-substitués selon une ou plusieurs des revendications 1 à 5 **caractérisés en ce que**
X représente l'hydrogène, cyano, C₁₋₆-alkyle, C₂₋₆-alcynyle, C₂₋₆-alcényle, C₁₋₄-alkylcarbonyle, C₁₋₄-alcoxycarbonyle, aminocarbonyle, C₁₋₄-alkylaminocarbonyle, di-(C₁₋₄-alkyl)aminocarbonyle ou C₁₋₄-alkylcarbonylamino-C₁₋₃-alkyle, où les groupements alkyle peuvent être fluorés une ou plusieurs fois ou substitués une fois avec le chlore ou cyano et X dans la signification d'alkyle à 2 ou plusieurs atomes C peut comporter un substituant hydroxy.

7. Phényles D-xylopyranosyl-substitués selon une ou plusieurs des revendications 1 à 5 **caractérisés en ce que**
X représente C₁₋₄-alkyloxyméthyle, C₃₋₇-cycloalkyloxyméthyle ou aryloxyméthyle, où par groupe aryle il convient de comprendre un groupe phényle ou naphtyle, de préférence un groupe phényle qui peut être substitué une ou deux fois avec des groupements L identiques ou différents et L est défini selon la revendication 1.

8. Phényles D-xylopyranosyl-substitués selon une ou plusieurs des revendications 1 à 5 **caractérisés en ce que**
X représente mercaptométhyle, C₁₋₄-alkylsulfanylméthyle ou C₁₋₄-alkylcarbonylsulfanylméthyle.

9. Phényles D-xylopyranosyl-substitués selon une ou plusieurs des revendications 1 à 5 **caractérisés en ce que**
X représente chlorométhyle, bromométhyle, iodométhyle, C₁₋₆-alkylsulfonyloxyméthyle, arylsulfonyloxyméthyle ou aryl-C₁₋₃-alkylsulfonyloxyméthyle,
où les groupements alkyle cités précédemment peuvent être partiellement ou totalement fluorés ou une ou deux fois chlorés et où les groupes aryle cités précédemment peuvent être substitués une ou deux fois avec des groupements L identiques ou différents, où L est choisi de préférence dans le groupe fluor, chlore, brome, iode, C₁₋₃-alkyle, difluorométhyle, trifluorométhyle et cyano.

10. Phényles D-xylopyranosyl-substitués selon une ou plusieurs des revendications 1 à 9 **caractérisés en ce que**
R² représente l'hydrogène, le fluor, hydroxy, méthoxy, éthoxy ou méthyle.

11. Phényles D-xylopyranosyl-substitués selon une ou plusieurs des revendications 1 à 10 **caractérisés en ce que**
R⁴ et R⁵ représentent indépendamment l'un de l'autre l'hydrogène ou le fluor.

12. Phényles D-xylopyranosyl-substitués selon une ou plusieurs des revendications 1 à 11 **caractérisés en ce que**
Z représente l'oxygène ou méthylène.

13. Phényles D-xylopyranosyl-substitués selon une ou plusieurs des revendications 1 à 12 **caractérisés en ce que**
R^{7a}, R^{7b}, R^{7c} représentent indépendamment les uns des autres l'hydrogène, (C₁₋₆-alkyl)oxycarbonyle, (C₁₋₈-alkyl)carbonyle ou benzoyle, de préférence l'hydrogène.

14. Sels physiologiquement acceptables des composés selon une ou plusieurs des revendications 1 à 13 avec des acides inorganiques ou organiques.

15. Médicament contenant un composé selon une ou plusieurs des revendications 1 à 13 ou un sel physiologiquement acceptable selon la revendication 14 outre éventuellement un ou plusieurs vecteurs et/ou diluants inertes.

16. Utilisation d'au moins un composé selon une ou plusieurs des revendications 1 à 13 ou d'un sel physiologiquement acceptable selon la revendication 14 pour la production d'un médicament qui convient pour le traitement ou la prophylaxie des maladies ou états qui sont influençables par l'inhibition du cotransporteur de glucose dépendant du sodium SGLT.

17. Utilisation d'au moins un composé selon une ou plusieurs des revendications 1 à 13 ou d'un sel physiologiquement acceptable selon la revendication 14 pour la production d'un médicament qui convient pour le traitement ou la prophylaxie des maladies métaboliques.

18. Utilisation selon la revendication 17 **caractérisée en ce que** la maladie métabolique est choisie dans le groupe consistant en le diabète sucré de type 1 et/ou de type 2, les complications diabétiques, l'acidose ou la cétose métabolique, l'hypoglycémie réactive, l'hyperinsulinémie, les troubles du métabolisme du glucose, la résistance à l'insuline, le syndrome métabolique, les dyslipidémies de genèse diverse, l'athérosclérose et les maladies apparentées, l'adipositas, l'hypertension artérielle, l'insuffisance cardiaque chronique, les oedèmes, l'hyperuricémie.

19. Utilisation d'au moins un composé selon au moins l'une des revendications 1 à 13 ou d'un sel physiologiquement acceptable selon la revendication 14 pour la production d'un médicament pour l'inhibition du cotransporteur de glucose dépendant du sodium SGLT.

20. Utilisation d'au moins un composé selon au moins l'une des revendications 1 à 13 ou d'un sel physiologiquement acceptable selon la revendication 14 pour la production d'un médicament pour éviter la dégénérescence des cellules bêta pancréatiques et/ou pour améliorer et/ou pour rétablir la fonctionnalité des cellules bêta pancréatiques.

21. Utilisation d'au moins un composé selon au moins l'une des revendications 1 à 13 ou d'un sel physiologiquement acceptable selon la revendication 14 pour la production de diurétiques et/ou d'antihypertenseurs.

22. Procédé de production d'un médicament selon la revendication 15 **caractérisé en ce que**, par voie non chimique, un composé selon au moins l'une des revendications 1 à 13 ou un sel physiologiquement acceptable selon la revendication 14 est incorporé dans un ou plusieurs vecteurs et/ou diluants inertes.

23. Procédé de production des composés de formule générale I selon les revendications 1 à 13, **caractérisé en ce qu'**un composé de formule générale II 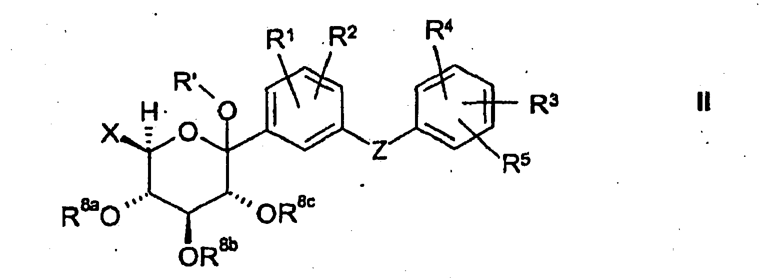 où
R' représente H, C₁₋₄-alkyle, (C₁₋₈-alkyl)carbonyle, (C₁₋₁₈-alkyl)oxycarbonyle, arylcarbonyle ou aryl-(C₁₋₃-alkyl)carbonyle, où les groupes alkyle ou aryle peuvent être substitués une ou plusieurs fois avec halogène ;
R^{8a}, R^{8b}, R^{8c} présentent indépendamment les uns des autres les significations indiquées pour les groupements R^{7a}, R^{7b}, R^{7c}, représentent un groupe benzyle ou un groupe R^{a}R^{b}R^{c}Si ou un groupe cétal ou acétal, en particulier un groupe alkylidène- ou arylalkylidène-cétal ou acétal, où à chaque fois deux groupements R^{8a}, R^{8b}, R^{8c} voisins peuvent former un groupe cétal ou acétal cyclique ou un pont 1,2-di(C₁₋₃-alcoxy)-1,2-di(C₁₋₃-alkyl)-éthylène, où le pont éthylène cité forme avec deux atomes d'oxygène et les deux atomes de carbone correspondants du cycle pyranose un cycle dioxane substitué, en particulier un cycle 2,3-diméthyl-2,3-di(C₁₋₃-alcoxy)-1,4-dioxane, et où les groupes alkyle, aryle et/ou benzyle peuvent être substitués une ou plusieurs fois avec halogène ou C₁₋₃-alcoxy et les groupes benzyle peuvent aussi être substitués avec un groupe di-(C₁₋₃-alkyl)amino ; et
R^{a}, R^{b}, R^{c} représentent indépendamment les uns des autres C₁₋₄-alkyle, aryle ou aryl-C₁₋₃-alkyle, où les groupes alkyle ou aryle peuvent être substitués une ou plusieurs fois avec halogène ;
où par les groupes aryle cités dans la définition des groupements cités précédemment il convient de comprendre des groupes phényle ou naphtyle, de préférence des groupes phényle ;
et X, Z, R¹ à R⁵, R^{7a}, R^{7b}, R^{7c} possèdent les significations indiquées dans les revendications 1 à 13,
est mis à réagir avec un réducteur en présence d'un acide, où les groupes protecteurs éventuellement présents sont clivés en même temps ou ultérieurement ;
si nécessaire, un groupement protecteur utilisé dans les réactions décrites précédemment est clivé et/ou
si on le souhaite un composé de formule générale I ainsi obtenu est dérivatisé sélectivement au niveau d'un groupe hydroxy ou celui-ci est substitué et/ou
si on le souhaite un composé de formule générale I ainsi obtenu est séparé en ses stéréoisomères et/ou
si on le souhaite un composé de formule générale I ainsi obtenu est converti en ses sels physiologiquement acceptables.

24. Procédé de production des composés de formule générale I selon les revendications 1 à 13, où R^{7a}, R^{7b} et R^{7c} représentent l'hydrogène, **caractérisé en ce que**, dans un composé de formule générale III 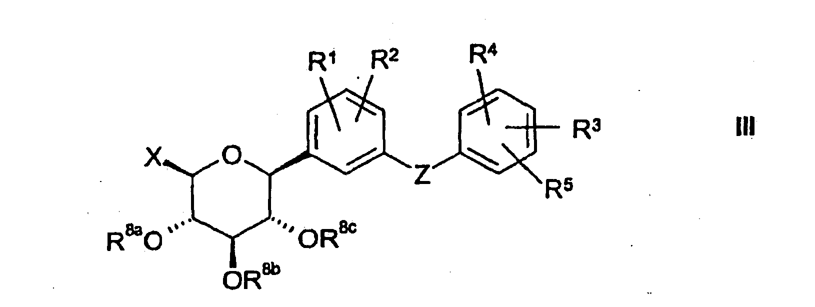 où
R^{8a}, R^{8b}, R^{8c} présentent indépendamment les uns des autres les significations indiquées pour les groupements R^{7a}, R^{7b}, R^{7c}, représentent un groupe benzyle ou un groupe R^{a}R^{b}R^{c}Si ou un groupe cétal ou acétal, en particulier un groupe alkylidène- ou arylalkylidène-cétal ou acétal, où à chaque fois deux groupements R^{8a}, R^{8b}, R^{8c} voisins peuvent former un groupe cétal ou acétal cyclique ou un pont 1,2-di(C₁₋₃-alcoxy)-1,2-di(C₁₋₃-alkyl)-éthylène, où le pont éthylène cité forme avec deux atomes d'oxygène et les deux atomes de carbone correspondants du cycle pyranose un cycle dioxane substitué, en particulier un cycle 2,3-diméthyl-2,3-di(C₁₋₃-alcoxy)-1,4-dioxane, et où les groupes alkyle, aryle et/ou benzyle peuvent être substitués une ou plusieurs fois avec halogène ou C₁₋₃-alcoxy et les groupes benzyle peuvent aussi être substitués avec un groupe di-(C₁₋₃-alkyl)amino ; et où au moins l'un des groupements R^{8a}, R^{8b} et R^{8c} ne représente pas l'hydrogène ; et
R^{a}, R^{b}, R^{c} représentent indépendamment les uns des autres C₁₋₄-alkyle, aryle ou aryl-C₁₋₃-alkyle, où les groupes alkyle ou aryle peuvent être substitués une ou plusieurs fois avec halogène ;
où par les groupes aryle cités dans la définition des groupements cités précédemment il convient de comprendre des groupes phényle ou naphtyle, de préférence des groupes phényle ;
et X, Z, R¹ à R⁵, R^{7a}, R^{7b}, R^{7c} possèdent les significations indiquées dans les revendications 1 à 13,
les groupements R^{8a}, R^{8b} ou R^{8c} qui ne représentent pas l'hydrogène sont éliminés, et
si nécessaire, un groupement protecteur utilisé dans les réactions décrites précédemment est clivé et/ou
si on le souhaite un composé de formule générale I ainsi obtenu est dérivatisé sélectivement au niveau d'un groupe hydroxy ou celui-ci est substitué et/ou
si on le souhaite un composé de formule générale I ainsi obtenu est séparé en ses stéréoisomères et/ou
un composé de formule générale I ainsi obtenu est converti en ses sels, en particulier pour l'utilisation pharmaceutique en ses sels physiologiquement acceptables.
